# EUROPEAN PATENT APPLICATION

(11) **EP 4 101 841 A1**
(43) Date of publication of application: **14.12.2022**
(21) Application number: 22176676.9
(22) Date of filing: 01.06.2022
(51) Int. Cl.: C07D 209/86, C07D 209/88, C07D 219/08, C07D 405/12, C07D 409/12, H01L 51/50

(54) **TRIARYLAMINE DERIVATIVE AND ORGANIC ELECTROLUMINESCENT DEVICE THEREOF**

(30) Priority: 11.06.2021 CN 202110654004
(71) Applicant: Changchun Hyperions Technology Co., Ltd., Bayhood Science and Technology Development Zone Changchun City Jilin 130000 (CN)
(72) Inventor: LIU, Hui, Changchun City, Jilin Province, 130000 (CN); MIAO, Yuhe, Changchun City, Jilin Province, 130000 (CN); LIU, Xiqing, Changchun City, Jilin Province, 130000 (CN); DONG, Xiuqin, Changchun City, Jilin Province, 130000 (CN)
(74) Representative: Ricker, Mathias

(57) **Abstract**

Provided are a triarylamine derivative and an organic electroluminescent device thereof, which relate to the technical field of organic electroluminescence. The triarylamine derivative has a relatively high triplet energy level, high hole mobility, a high refractive index and good film formability and thermal stability and can effectively reduce the driving voltage of the organic electroluminescent device, improve the luminescence efficiency, and extend the service life of the device when applied to the organic electroluminescent device. The triarylamine derivative has a good application effect and a good industrialization prospect in the organic electroluminescent device.

## Description

### TECHNICAL FIELD

The present disclosure relates to the technical field of organic electroluminescence and, in particular, to a triarylamine derivative and an organic electroluminescent device thereof.

### BACKGROUND

An organic electroluminescent device (such as an organic light-emitting diode (OLED)) refers to the phenomenon that an organic semiconductor material and a light-emitting material emit light through carrier injection and recombination when driven by an electric field and can directly convert electrical energy into optical energy. Due to the advantages of active luminescence, a large viewing angle, a fast response speed, a wide adaptable temperature range, a low driving voltage, low power consumption, high brightness, a simple manufacturing process, a light weight and flexible display, the OLED is increasingly applied to the fields of commerce, electronic products, transportation, industry, medical treatment and the military and considered to be a new type of flat-panel display device that can replace a liquid crystal display. Known as flat-panel display technology having a magic display feature, the OLED has become the hot spot of research and development in the fields of new materials and display technology.

The stability of the organic electroluminescent device is related to many factors such as a device structure, an organic light-emitting material, an electrode material and process conditions. With the continuous advancement of science and technology, the structure of the organic electroluminescent device has been continuously optimized and has developed from a simple single-layer device structure to a three-layer or multi-layer device structure. At present, organic function layers involved in the organic electroluminescent device include a hole injection layer, a hole transport layer, a hole blocking layer, a light-emitting layer, an electron blocking layer, an electron transport layer, an electron injection layer, a capping layer and the like. The process conditions for preparing the organic electroluminescent device are continuously innovated and improved. However, since the organic light-emitting material has not been developed to be perfect at this stage, the organic electroluminescent device still has many problems.

As for hole transport materials, traditionally used hole transport materials have low highest occupied molecular orbital (HOMO) values and low triplet energy levels, which results in a charge imbalance in the light-emitting layer so that such hole transport materials generally cannot provide satisfactory luminescence efficiency. Moreover, the material of the hole transport layer has a relatively low glass transition temperature and will reduce the service life of the device. Therefore, it is very necessary to design a class of hole transport materials with good film formability and thermal stability, high HOMO values and triplet energy levels, high hole transport rates and an electron blocking ability.

At present, the researches on organic electroluminescent materials have received wide attention from the academia and industry, and organic light-emitting devices have been developed towards practicality and commercialization. In general, the future direction of the OLED is to develop a display device with high efficiency, high brightness, a long lifetime and a low cost. However, how to ensure that the hole transport material has a high triplet energy level, a high glass transition temperature, a high hole mobility so as to improve the luminescence efficiency and service life of the device has become an urgent problem to be solved.

A class of compounds used as materials of the hole transport layer are described in Chinese Patent Application No. 201711089080.2. However, such structures have problems such as ease to crystallize, relatively poor film formability and relatively low hole mobility, which lead to relatively low luminescence efficiency and a relatively short service life of the organic electroluminescent device. Therefore, a hole transport material with good film formability and stability needs to be developed.

### SUMMARY

To solve the preceding problems, the present disclosure provides a triarylamine derivative and an organic electroluminescent device thereof. The organic electroluminescent device has a low driving voltage, high luminescence efficiency and a long lifetime.

Specifically, the present disclosure provides a triarylamine derivative which has a structure represented by Formula I: 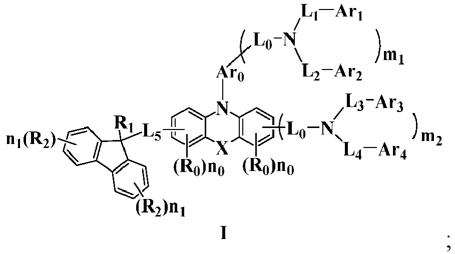 where in Formula I, X is selected from a single bond or C(R₃R₄); where R₃ and R₄ are independently selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl, or R₃ and R₄ are joined to form a substituted or unsubstituted ring;
Ar₁ and Ar₂ are the same as or different from each other and independently selected from any one of substituted or unsubstituted C6 to C30 aryl or substituted or unsubstituted C2 to C30 heteroaryl;
Ar₃ and Ar4 are the same as or different from each other and independently selected from any one of substituted or unsubstituted C6 to C30 aryl or the following groups: 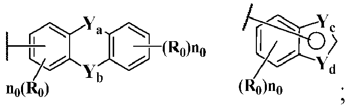
wherein, Ya is selected from O or S, Yb is selected from a single bond or C(RₓR_{y}),
Yc is selected from any one of O, S or N(R_{z}), and Yd is selected from O, S or N(R_{z});
Rₓ and R_{y} are independently selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl, or R₃ and R₄ are joined to form a substituted or unsubstituted ring;
R_{z} is selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
L₀ to L₅ are independently selected from any one of a single bond, substituted or unsubstituted C6 to C30 arylene or substituted or unsubstituted C2 to C30 heteroarylene;
m₁ is selected from 0 or 1, m₂ is selected from 0 or 1, and m₁ and m₂ are not simultaneously selected from 0;
when m₁ is 0, Ar₀ is selected from any one of substituted or unsubstituted C6 to C30 aryl; when m₁ is 1, Ar₀ is selected from a single bond;
R₁ is selected from any one of substituted or unsubstituted C1 to C12 alkyl, substituted or unsubstituted C3 to C12 cycloalkyl or substituted or unsubstituted C6 to C30 aryl;
R₀ and R₂ are independently selected from any one of hydrogen, deuterium, cyano, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
n₀ is independently selected from an integer from 0 to 4, and n₁ is independently selected from an integer from 0 to 4; when n₀ is greater than 1, two or more R₀ are the same as or different from each other;
when n₁ is greater than 1, two or more R₂ are the same as or different from each other, or two adjacent R₂ are joined to form a benzene ring or a naphthalene ring;
a substituent in the preceding "substituted or unsubstituted" group is selected from one or more of: deuterium, cyano, a halogen atom, amino, nitro, substituted or unsubstituted C1 to C12 alkyl, substituted or unsubstituted C3 to C12 cycloalkyl, substituted or unsubstituted C1 to C12 alkoxy, substituted or unsubstituted C6 to C30 aryl and substituted or unsubstituted C2 to C30 heteroaryl; in the presence of a plurality of substituents, the plurality of substituents are the same as or different from each other.

The present disclosure further provides an organic electroluminescent device. The device includes an anode, a cathode and an organic layer, where the organic layer is disposed between the anode and the cathode or disposed on an outer side of one or more of the anode and the cathode, where the organic layer includes at least one of the triarylamine derivatives of the present disclosure.

### Beneficial Effects

The triarylamine derivative provided by the present disclosure includes an unconjugated group so that the triarylamine derivative has a relatively high triplet energy level. Moreover, the present disclosure has suitable HOMO and LUMO energy levels. On one hand, the triarylamine derivative can reduce the injection barrier of holes, thereby reducing the driving voltage of the organic electroluminescent device. On the other hand, the triarylamine derivative can effectively prevent excitons from escaping to an anode side, confine the excitons within a light-emitting layer, and effectively improve the recombination rate of the excitons in the light-emitting layer, thereby improving the luminescence efficiency of the organic electroluminescent device. After the group R₁ is introduced into the structure, the material has a further improved triplet energy level, an improved HOMO energy level and improved hole mobility and can further reduce the driving voltage of the organic electroluminescent device to a certain extent and improve the luminescence efficiency of the device.

The triarylamine derivative provided by the present disclosure has a steric structure. The introduction of R₁ can improve the stericity of the compound while improving hole mobility so that the overall structure has better stability. Such triarylamine derivatives have relatively high glass transition temperatures, are not easy to crystallize in a thin-film state, have good film formability and thermal stability, and exhibit a long lifetime when applied to organic electroluminescent devices.

Meanwhile, the triarylamine derivative provided by the present disclosure has a high refractive index. When used as a capping layer in the organic electroluminescent device, the triarylamine derivative can avoid total reflection of the device, improve the light extraction efficiency of the device, and improve the luminescence efficiency of the device.

To sum up, the triarylamine derivative provided by the present disclosure has a relatively high triplet energy level, high hole mobility, a high refractive index and good film formability and thermal stability and can effectively reduce the driving voltage of the organic electroluminescent device, improve the luminescence efficiency, and extend the service life of the device when applied to the organic electroluminescent device. The triarylamine derivative of the present disclosure has a good application effect and a good industrialization prospect in the organic electroluminescent device.

### BRIEF DESCRIPTION OF DRAWINGS

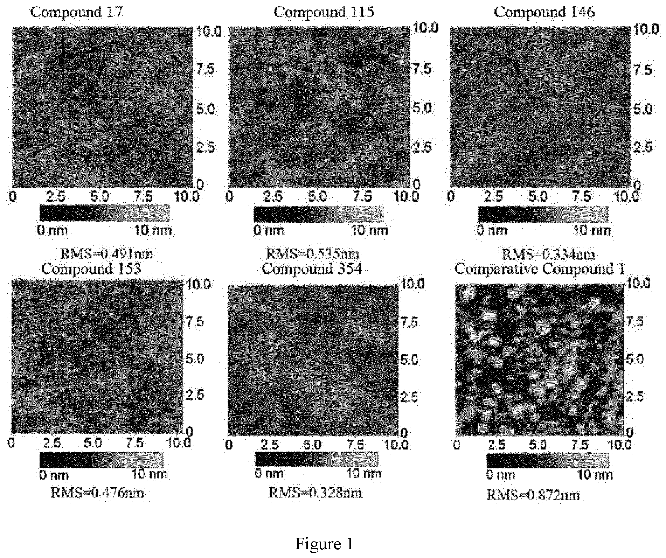

FIG. 1 shows the morphology of sample thin films prepared from Compounds 17, 115, 146, 153 and 354 of the present disclosure and Comparative Compound 1.

### DETAILED DESCRIPTION

The present disclosure is described below clearly and completely in conjunction with technical solutions in embodiments of the present disclosure. Apparently, the embodiments described herein are part, not all, of the embodiments of the present disclosure. After reading the present disclosure, those skilled in the art can make various equivalent modifications on the present disclosure, which fall within the scope of the present disclosure.

In the specification, 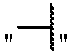 refers to a moiety joined to another substituent.

Examples of halogen atoms of the present disclosure may include fluorine, chlorine, bromine and iodine.

An alkyl group of the present disclosure refers to a hydrocarbyl group formed by removing one hydrogen atom from an alkane molecule. Alkyl may be linear alkyl or branched alkyl. An alkyl group has preferably 1 to 12 carbon atoms, more preferably 1 to 8 carbon atoms, and most preferably 1 to 6 carbon atoms. Examples may include, but are not limited to, methyl, ethyl, n-propyl, isopropyl, n-butyl, tert-butyl, isobutyl, pentyl, isopentyl, hexyl and the like.

A cycloalkyl group of the present disclosure refers to a hydrocarbyl group formed by removing two hydrogen atoms from an alkane molecule. A cycloalkyl group has preferably 3 to 12 carbon atoms, more preferably 3 to 10 carbon atoms, and most preferably 3 to 6 carbon atoms. Examples may include, but are not limited to, cyclopentyl, cyclohexyl, adamantyl, norbornyl and the like.

An aryl group of the present disclosure refers to a general term for the remaining monovalent group after one hydrogen atom is removed from an aromatic nucleus carbon of an arene molecule. An aryl group may be a monocyclic aryl group, a polycyclic aryl group or a fused-ring aryl group and has preferably 6 to 30 carbon atoms, more preferably 6 to 22 carbon atoms, still more preferably 6 to 18 carbon atoms, and most preferably 6 to 12 carbon atoms. For the preceding aryl group, a monocyclic aryl group may be, but is not limited to, phenyl or the like. The preceding polycyclic aryl group may be, but is not limited to, biphenyl, terphenyl, tetraphenyl or the like. The preceding fused-ring aryl group may be, but is not limited to, naphthyl, anthryl, phenanthryl, pyrenyl, fluorenyl, spirofluorenyl, triphenylene, perylenyl, fluoranthenyl, chrysenyl or the like.

A heteroaryl group of the present disclosure refers to a general term for the remaining monovalent group after one hydrogen atom is removed from a nucleus carbon of a heteroaromatic ring composed of carbon and a heteroatom, where the heteroatom may be one or more of N, O and S. A heteroaryl group may be a monocyclic heteroaryl group, a polycyclic heteroaryl group or a fused-ring heteroaryl group and has preferably 2 to 30 carbon atoms, more preferably 2 to 22 carbon atoms, still more preferably 2 to 20 carbon atoms, and most preferably 3 to 12 carbon atoms. Examples may include, but are not limited to, pyridyl, pyrimidinyl, pyrazinyl, pyridazinyl, triazinyl, thienyl, pyrrolyl, furyl, pyranyl, oxazolyl, thiazolyl, imidazolyl, benzoxazolyl, benzothiazolyl, benzimidazolyl, benzotriazolyl, carbazolyl, benzocarbazolyl, acridinyl, xanthyl, thioxanthenyl, phenazinyl, phenothiazinyl, phenoxazinyl, indolyl, quinolyl, isoquinolyl, benzothienyl, benzofuryl, dibenzofuryl, dibenzothienyl, quinoxalinyl, quinazolinyl, naphthyridinyl, purinyl, an o-phenanthrolinyl and the like.

An arylene group of the present disclosure refers to a general term for the remaining divalent group after two hydrogen atoms are removed from an aromatic nucleus carbon of an arene molecule. An arylene group may be a monocyclic arylene group, a polycyclic arylene group or a fused-ring arylene group and has preferably 6 to 30 carbon atoms, more preferably 6 to 22 carbon atoms, still more preferably 6 to 18 carbon atoms, and most preferably 6 to 12 carbon atoms. For the preceding arylene group, the monocyclic arylene group may be, but is not limited to, phenylene or the like. The preceding polycyclic arylene group may be, but is not limited to, biphenylene, terphenylene, tetraphenylene or the like. The preceding fused-ring arylene group may be, but is not limited to, naphthylene, anthrylene, phenanthrylene, pyrenylene, fluorenylene, spirofluorenylene, triphenylenylene, perylenylene, fluoranthenylene, chrysenylene or the like.

A heteroarylene group of the present disclosure refers to a general term for the remaining divalent group after two hydrogen atoms are removed from a nucleus carbon of a heteroaromatic ring composed of carbon and a heteroatom, where the heteroatom may be one or more of N, O and S. A heteroarylene group may be a monocyclic heteroarylene group, a polycyclic heteroarylene group or a fused-ring heteroarylene group and has preferably 2 to 30 carbon atoms, more preferably 2 to 22 carbon atoms, still more preferably 2 to 20 carbon atoms, and most preferably 3 to 12 carbon atoms. Examples may include, but are not limited to, pyridylene, pyrimidinylene, pyrazinylene, pyridazinylene, triazinylene, thienylene, pyrrolylene, furylene, pyranylene, oxazolylene, thiazolylene, imidazolylene, benzoxazolylene, benzothiazolylene, benzimidazolylene, carbazolylene, benzocarbazolylene, acridinylene, xanthylene, thioxanthenylene, phenazinylene, phenothiazinylene, phenoxazinylene, indolylene, quinolylene, isoquinolylene, benzothienylene, benzofurylene, dibenzofurylene, dibenzothienylene, quinoxalinylene, quinazolinylene, naphthyridinylene, purinylene, o-phenanthrolinylene and the like. A "substitution" in the present disclosure refers to that a hydrogen atom in a compound group is replaced with another atom or group, and the position of the substitution is not limited.

A substituent in the "substituted or unsubstituted" group of the present disclosure may be independently selected from any one of deuterium, cyano, nitro, amino, a halogen atom, substituted or unsubstituted C1 to C12 alkyl, substituted or unsubstituted C1 to C12 alkoxy, substituted or unsubstituted C1 to C12 alkylamino, substituted or unsubstituted C3 to C12 cycloalkyl, substituted or unsubstituted C6 to C30 aryl, substituted or unsubstituted C2 to C30 heteroaryl or substituted or unsubstituted C1 to C30 silyl, preferably deuterium, cyano, a halogen atom, amino, C1 to C12 alkyl, C3 to C12 cycloalkyl, C1 to C12 alkoxy, C6 to C30 aryl or C2 to C30 heteroaryl. Specific examples may include, but are not limited to, deuterium, fluorine, chlorine, bromine, iodine, cyano, methyl, ethyl, n-propyl, isopropyl, n-butyl, tert-butyl, cyclopropyl, cyclohexyl, adamantyl, norbornyl, phenyl, tolyl, mesityl, pentadeuterophenyl, biphenyl, naphthyl, anthryl, phenanthryl, pyrenyl, triphenylene, chrysenyl, perylenyl, fluoranthenyl, fluorenyl, 9,9-dimethylfluorenyl, 9,9-diphenylfluorenyl, 9-methyl-9-phenylfluorenyl, spirofluorenyl, carbazolyl, 9-phenylcarbazolyl, 9,9'-spirobifluorenyl, carbazoloindolyl, pyrrolyl, furyl, thienyl, benzofuryl, benzothienyl, dibenzofuryl, dibenzothienyl, pyridyl, pyrimidinyl, pyridazinyl, pyrazinyl, triazinyl, oxazolyl, thiazolyl, imidazolyl, benzoxazolyl, benzothiazolyl, benzotriazolyl, benzimidazolyl, quinolinyl, isoquinolinyl, quinoxalinyl, quinazolinyl, phenothiazinyl, phenoxazinyl, acridinyl and the like. Alternatively, when multiple substituents are present, the multiple substituents are the same as or different from each other or adjacent substituents may be joined to form a ring.

"Selected from an integer from 0 to M" in the present disclosure refers to that the value is selected from any one of integers from 0 to M, including 0, 1, 2, ..., M-2, M-1 and M. For example, in the present disclosure, the expression that "no is selected from an integer from 0 to 4" refers to that n₀ is selected from 0, 1, 2, 3 or 4. The expression that "n₁ is selected from an integer from 0 to 4" refers to that n₁ is selected from 0, 1, 2, 3 or 4. The expression that "a₁ is selected from an integer from 0 to 5" refers to that a₁ is selected from 0, 1, 2, 3, 4 or 5. The expression that "a₂ is selected from an integer from 0 to 7" refers to that a₂ is selected from 0, 1, 2, 3, 4, 5, 6 or 7. The expression that "a₃ is selected from an integer from 0 to 9" refers to that a₃ is selected from 0, 1, 2, 3, 4, 5, 6, 7, 8 or 9. The expression that "a₄ is selected from an integer from 0 to 11" refers to that a₄ is selected from 0, 1, 2, 3, 4, 5, 6, 7, 8, 9, 10 or 11. The expression that "a₅ is selected from an integer from 0 to 3" refers to that as is selected from 0, 1, 2 or 3. The expression that "a₆ is selected from an integer from 0 to 4" refers to that a₆ is selected from 0, 1, 2, 3 or 4. The rest can be done in the same manner.

"Joined to form a ring" in the present disclosure refers to that two groups are joined to each other by a chemical bond and are optionally aromatized, which can be exemplified by the following formulas:

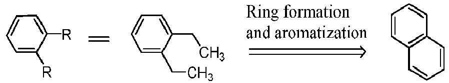

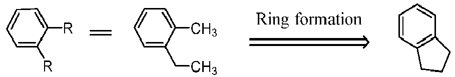

In the present disclosure, the formed ring may be a five-membered ring or a six-membered ring or a fused ring, such as, but not limited to, phenyl, naphthyl, fluorenyl, cyclopentyl, cyclohexanephenyl, phenanthryl or pyrenyl.

The present disclosure provides a triarylamine derivative which has a structure represented by Formula I: 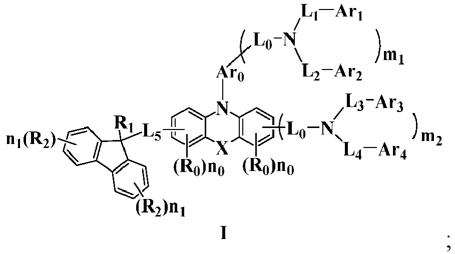 where in Formula I, X is selected from a single bond or C(R₃R₄); where R₃ and R₄ are independently selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl, or R₃ and R₄ are joined to form a substituted or unsubstituted ring;
Ar₁ and Ar₂ are the same as or different from each other and independently selected from any one of substituted or unsubstituted C6 to C30 aryl or substituted or unsubstituted C2 to C30 heteroaryl;
Ar₃ and Ar₄ are the same as or different from each other and independently selected from any one of substituted or unsubstituted C6 to C30 aryl or the following groups: 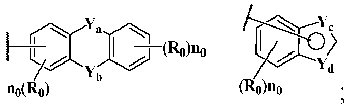
Ya is selected from O or S, Yb is selected from a single bond or C(RₓR_{y}),
Yc is selected from any one of O, S or N(R_{z}), and Yd is selected from O, S or N(R_{z});
Rₓ and R_{y} are independently selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl, or R₃ and R₄ are joined to form a substituted or unsubstituted ring;
R_{z} is selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
L₀ to L₅ are independently selected from any one of a single bond, substituted or unsubstituted C6 to C30 arylene or substituted or unsubstituted C2 to C30 heteroarylene;
m₁ is selected from 0 or 1, m₂ is selected from 0 or 1, and m₁ and m₂ are not simultaneously selected from 0;
when m₁ is 0, Ar₀ is selected from any one of substituted or unsubstituted C6 to C30 aryl; when m₁ is 1, Ar₀ is selected from a single bond;
R₁ is selected from any one of substituted or unsubstituted C1 to C12 alkyl, substituted or unsubstituted C3 to C12 cycloalkyl or substituted or unsubstituted C6 to C30 aryl;
R₀ and R₂ are independently selected from any one of hydrogen, deuterium, cyano, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
n₀ is independently selected from an integer from 0 to 4, and n₁ is independently selected from an integer from 0 to 4; when n₀ is greater than 1, two or more R₀ are the same as or different from each other;
when n₁ is greater than 1, two or more R₂ are the same as or different from each other, or two adjacent R₂ are joined to form a benzene ring or a naphthalene ring;
preferably, a substituent in the preceding "substituted or unsubstituted" group is selected from one or more of: deuterium, cyano, a halogen atom, amino, nitro, substituted or unsubstituted C1 to C12 alkyl, substituted or unsubstituted C3 to C12 cycloalkyl, substituted or unsubstituted C1 to C12 alkoxy, substituted or unsubstituted C6 to C30 aryl and substituted or unsubstituted C2 to C30 heteroaryl; in the presence of multiple substituents, the multiple substituents are the same as or different from each other.

Preferably, the compound of Formula I is selected from any one of structures represented by Formulas I-A to I-C:

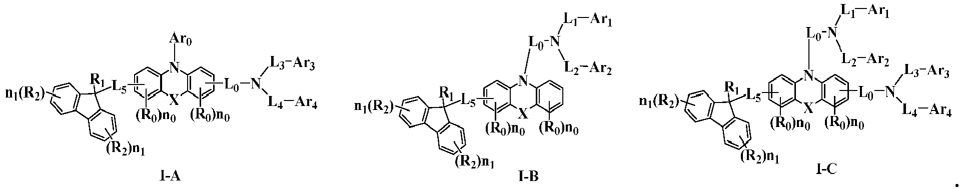

Preferably, the compound of Formula I is selected from any one of structures represented by Formulas I-1 to 1-6:

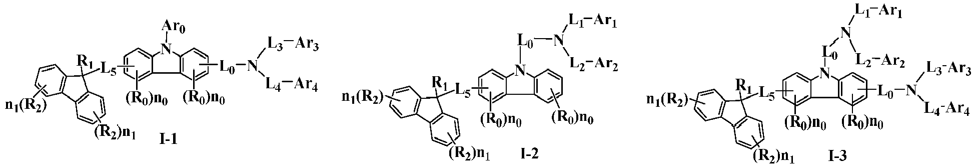

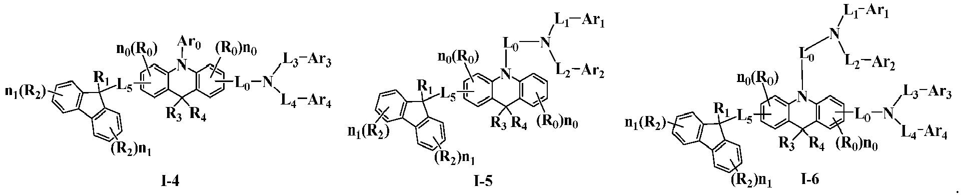

Preferably, Formula I-A is selected from any one of structures represented by Formulas I-1 and 1-4.

Preferably, Formula I-B is selected from any one of structures represented by Formulas 1-2 and 1-5.

Preferably, Formula I-C is selected from any one of structures represented by Formulas 1-3 and 1-6.

Preferably, Ar₁ and Ar₂ are the same as or different from each other and independently selected from any one of substituted or unsubstituted phenyl, substituted or unsubstituted naphthyl, substituted or unsubstituted anthryl, substituted or unsubstituted phenanthryl, substituted or unsubstituted triphenylene, substituted or unsubstituted pyrenyl, substituted or unsubstituted chrysenyl, substituted or unsubstituted fluoranthenyl, substituted or unsubstituted fluorenyl, substituted or unsubstituted furyl, substituted or unsubstituted thienyl, substituted or unsubstituted benzofuryl, substituted or unsubstituted benzothienyl, substituted or unsubstituted dibenzofuryl, substituted or unsubstituted dibenzothienyl, substituted or unsubstituted acridinyl, substituted or unsubstituted phenazinyl, substituted or unsubstituted xanthyl, substituted or unsubstituted thioxanthenyl, substituted or unsubstituted phenothiazinyl, substituted or unsubstituted phenoxazinyl, substituted or unsubstituted oxazolyl, substituted or unsubstituted thiazolyl, substituted or unsubstituted imidazolyl, substituted or unsubstituted benzoxazolyl, substituted or unsubstituted benzothiazolyl or substituted or unsubstituted benzimidazolyl.

More preferably, Ar₁ to Ar₄ are the same as or different from each other and independently selected from any one of the following groups: 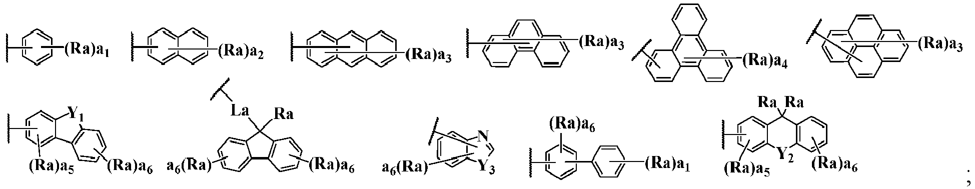 where Ra is selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
a₁ is independently selected from an integer from 0 to 5, a₂ is selected from an integer from 0 to 7, a₃ is independently selected from an integer from 0 to 9, a₄ is selected from an integer from 0 to 11, as is independently selected from an integer from 0 to 3, and a₆ is independently selected from an integer from 0 to 4;
when a₁, a₂, a₃, a₄, as or a₆ is greater than 1, two or more Ra are the same as or different from each other, or two adjacent Ra are joined to form a substituted or unsubstituted ring;
Y₁ is selected from any one of O, S or C(RbRc),
Y₂ is selected from any one of O or S, and
Y₃ is selected from any one of O, S or N(Re);
Rb and Rc are independently selected from any one of hydrogen, C1 to C12 alkyl, C3 to C12 cycloalkyl, C6 to C30 aryl or C2 to C30 heteroaryl, or Rb and Rc are joined to form a substituted or unsubstituted ring; Re is selected from any one of hydrogen, C1 to C12 alkyl, C3 to C12 cycloalkyl, C6 to C30 aryl or C2 to C30 heteroaryl;
La is selected from any one of a single bond or substituted or unsubstituted C6 to C18 arylene.

More preferably, Ar₁ to Ar₄ are independently selected from any one of the following groups:

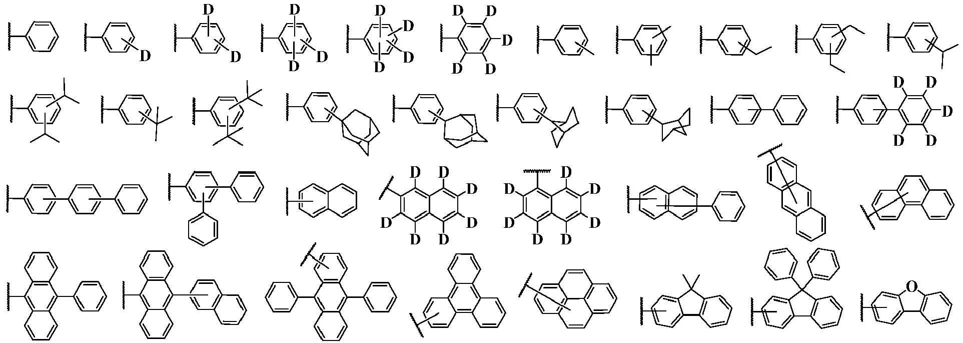

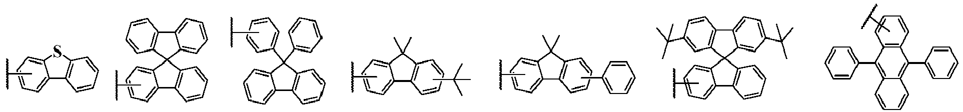

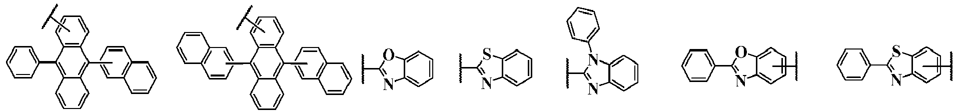

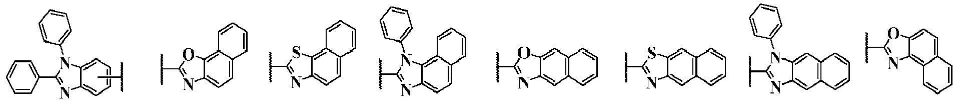

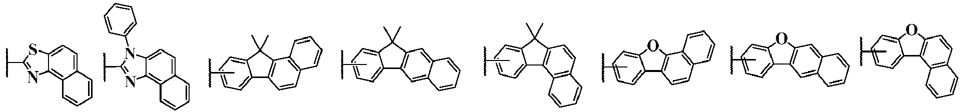

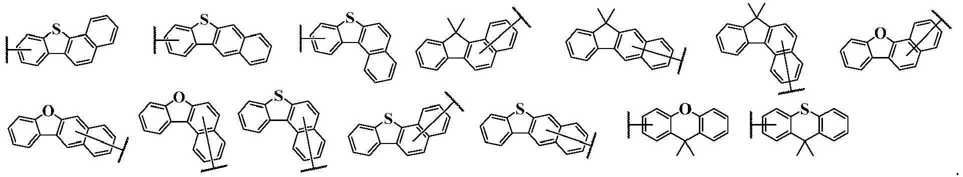

Preferably, L₀ to L₅ are independently selected from any one of a single bond or the following groups:

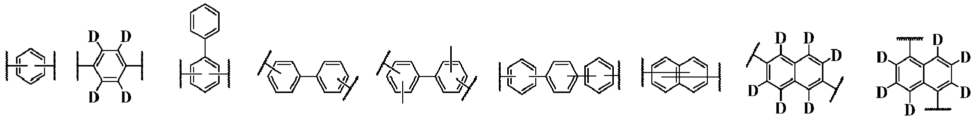

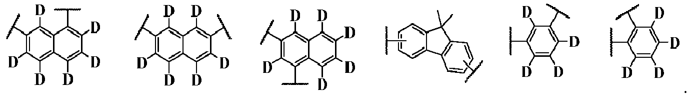

More preferably, L₅ is selected from a single bond.

Preferably, R₁ is selected from any one of methyl, ethyl, isopropyl, tert-butyl, phenyl or biphenyl.

Most preferably, the compound of Formula I is selected from any one of the following structures:

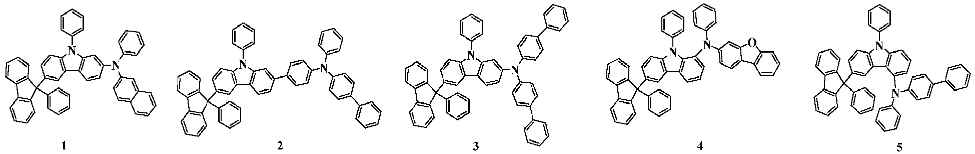

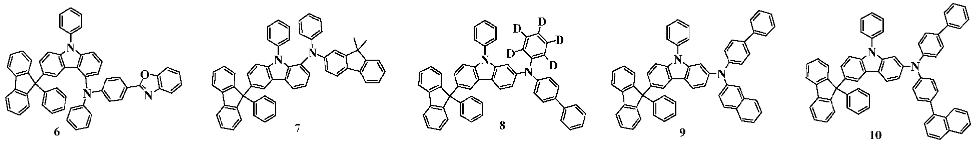

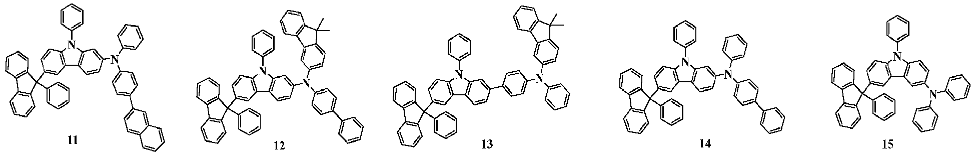

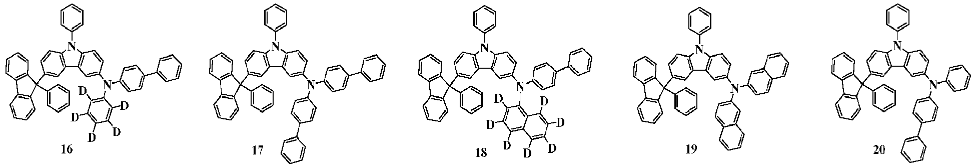

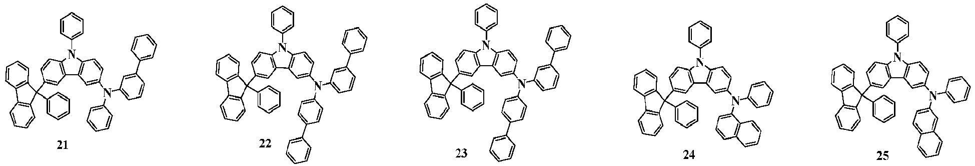

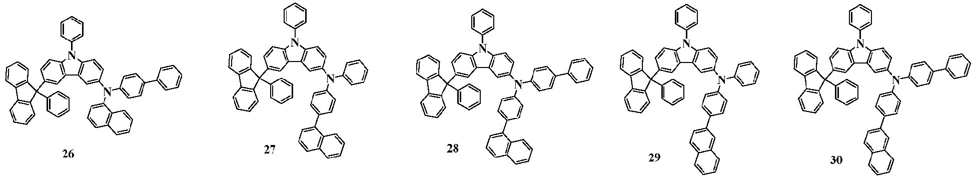

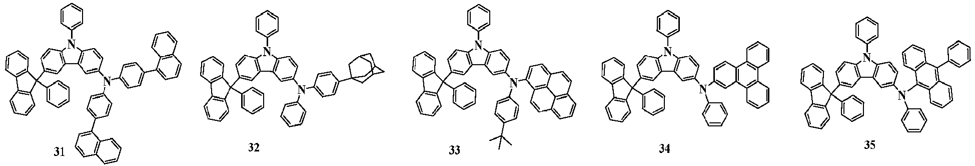

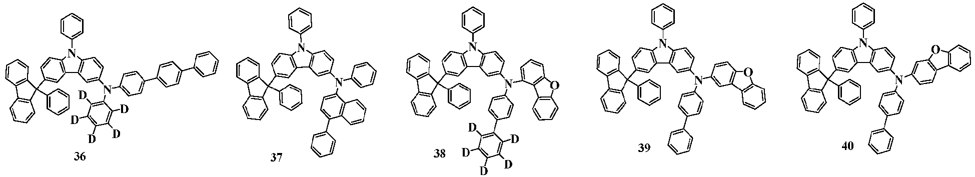

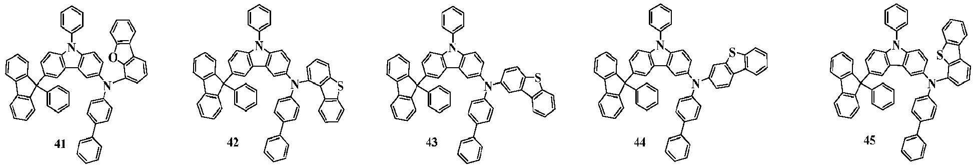

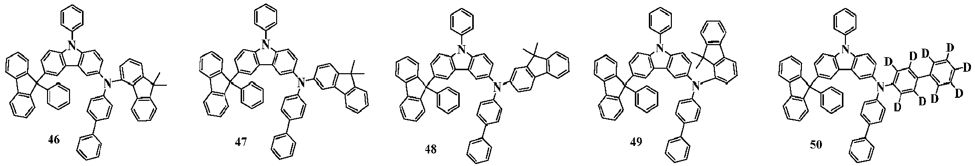

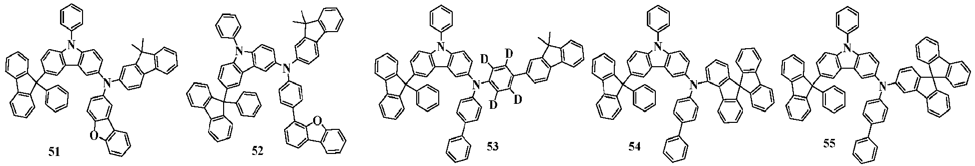

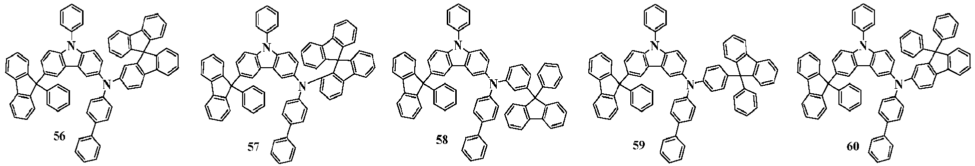

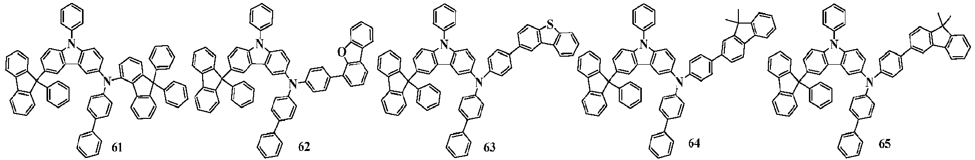

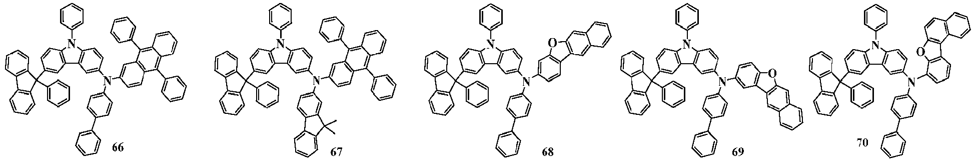

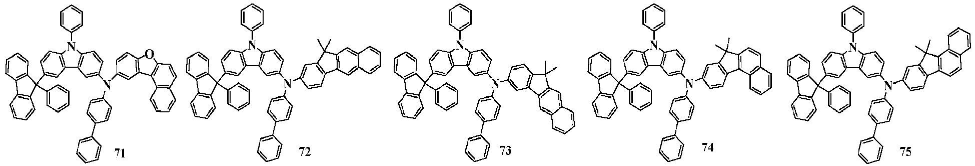

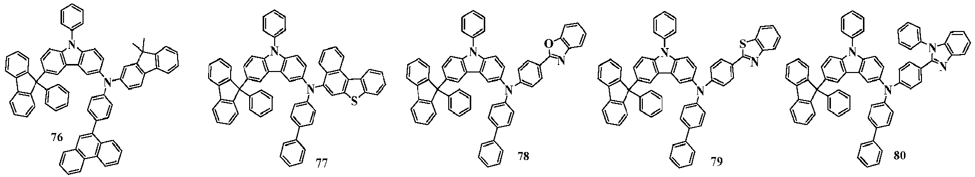

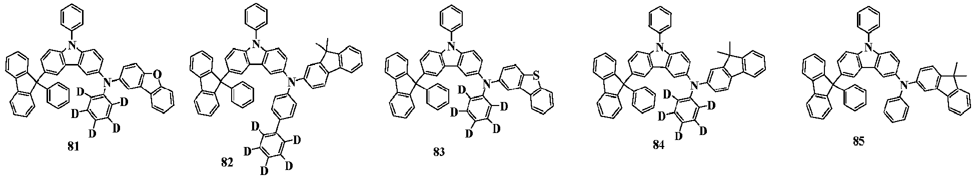

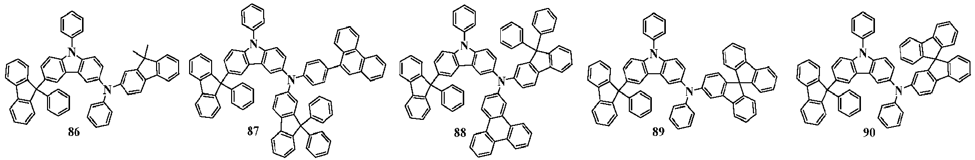

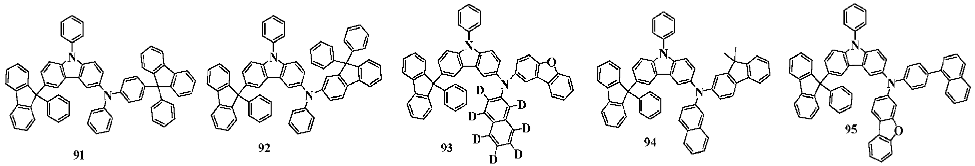

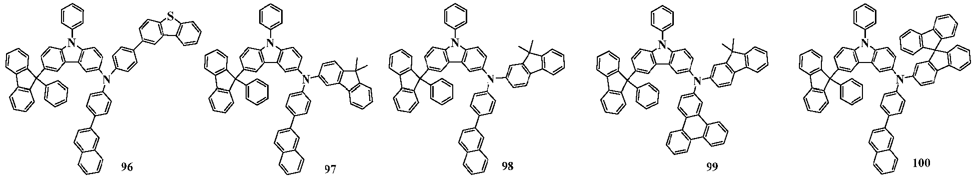

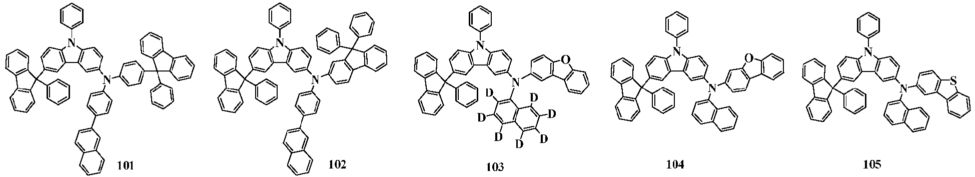

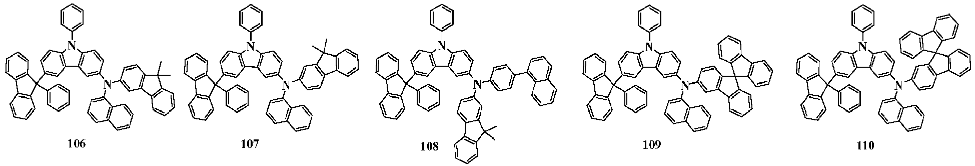

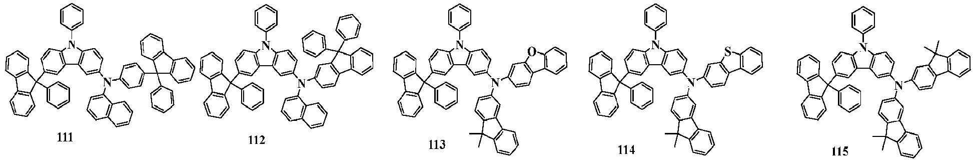

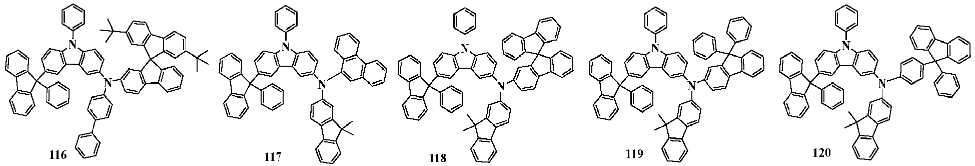

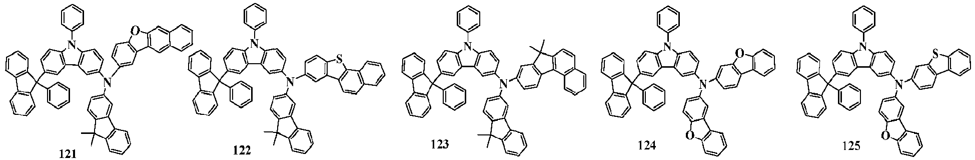

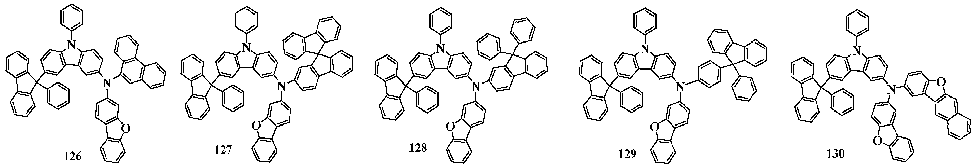

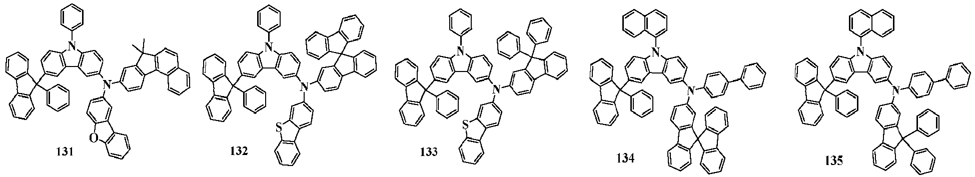

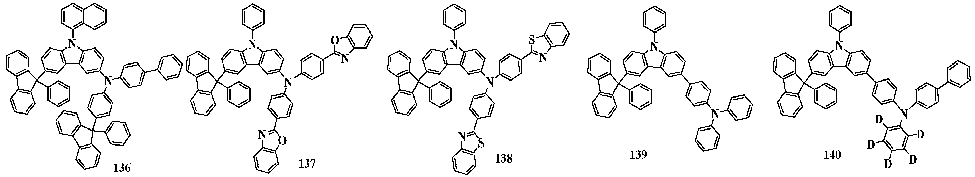

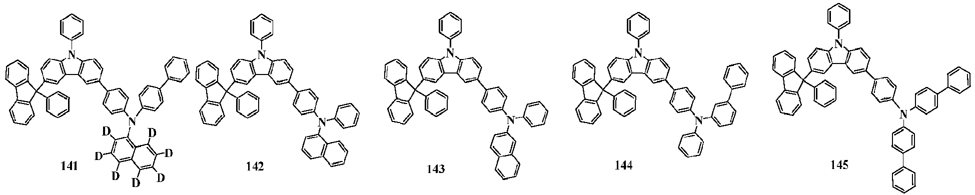

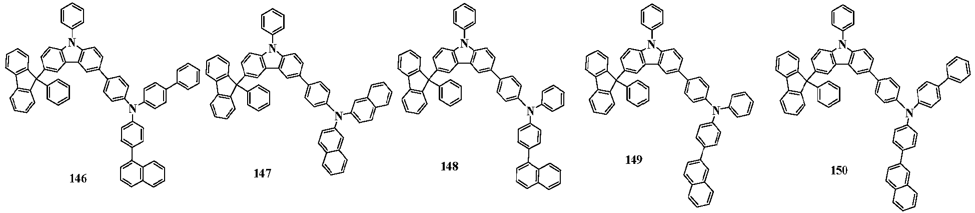

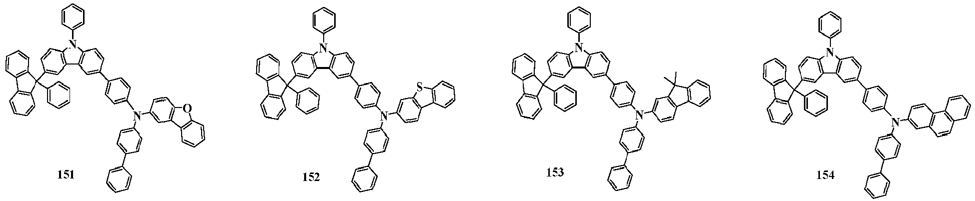

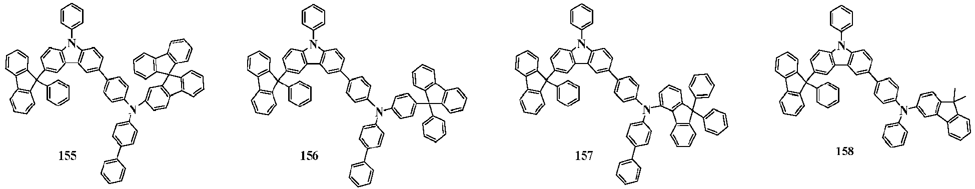

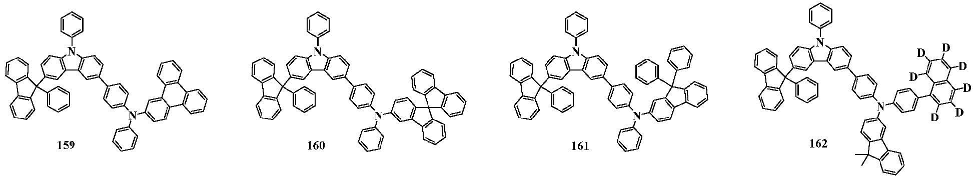

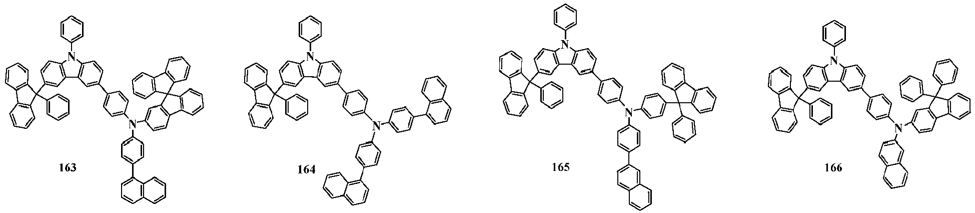

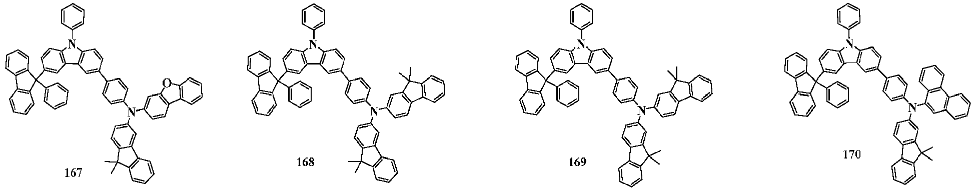

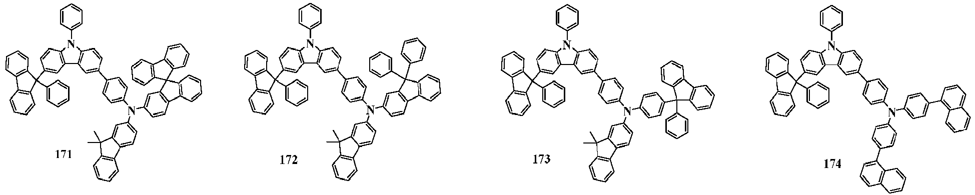

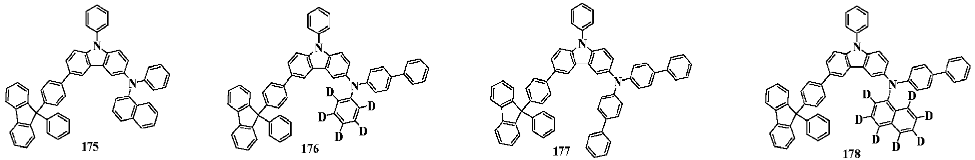

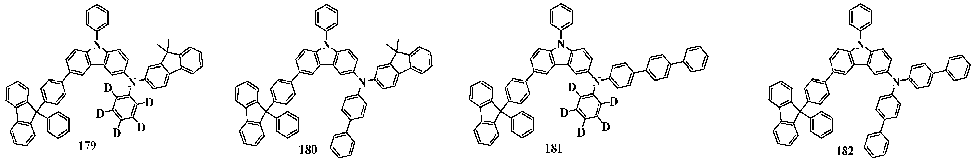

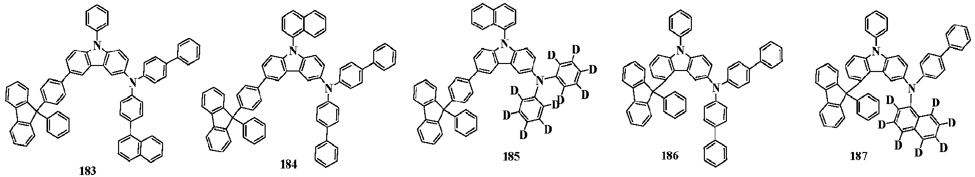

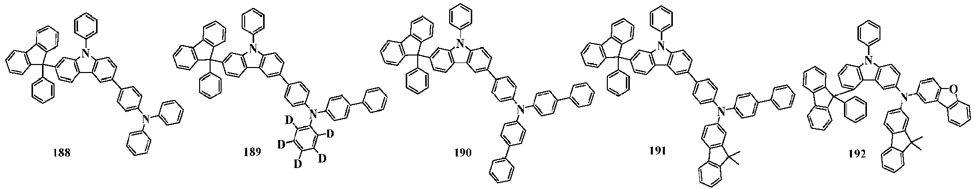

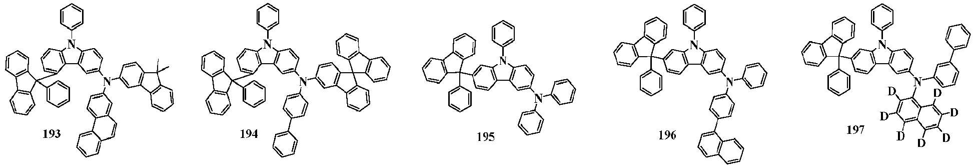

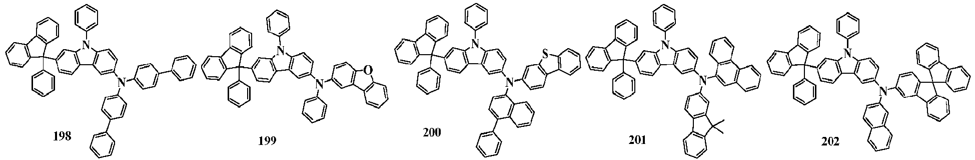

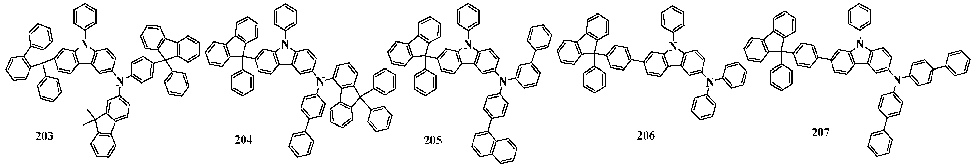

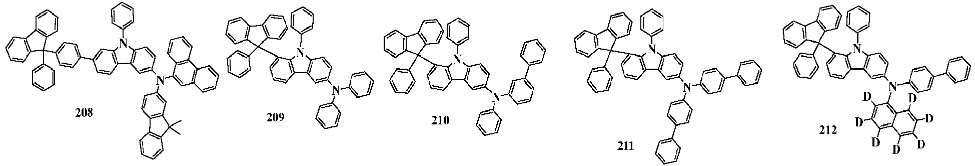

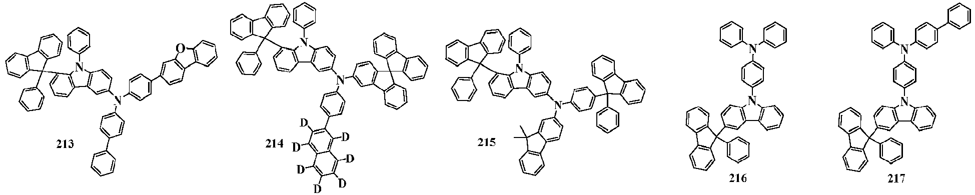

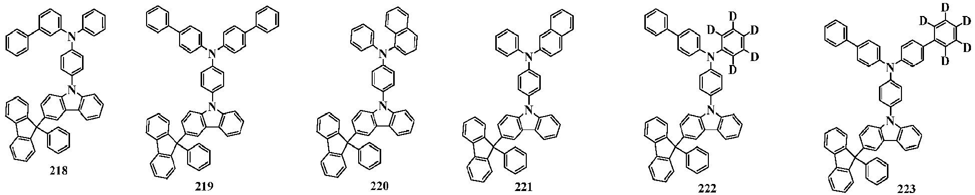

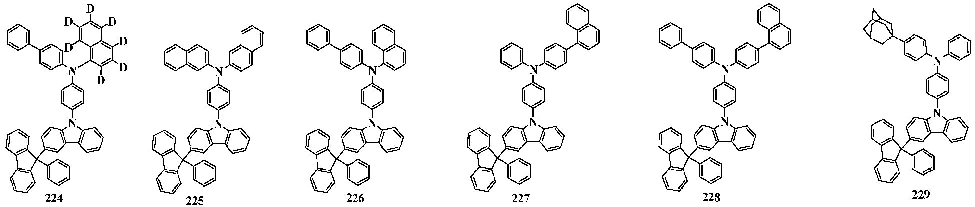

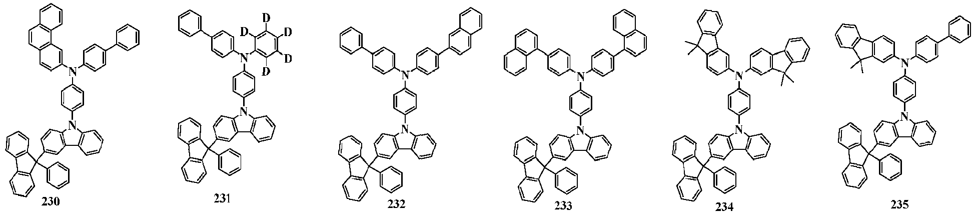

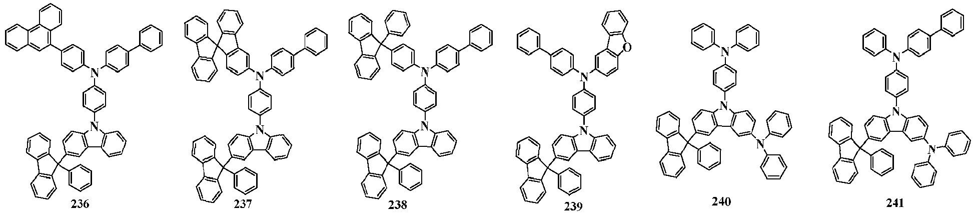

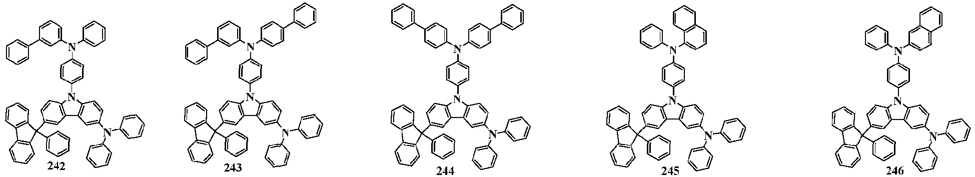

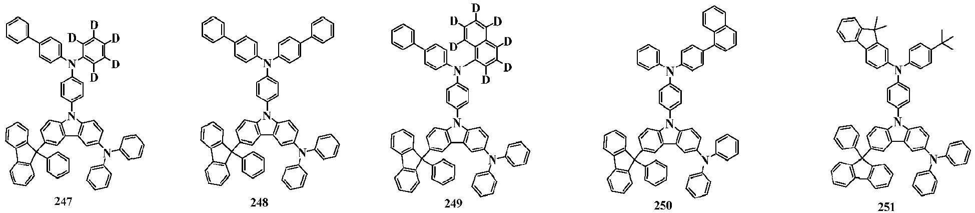

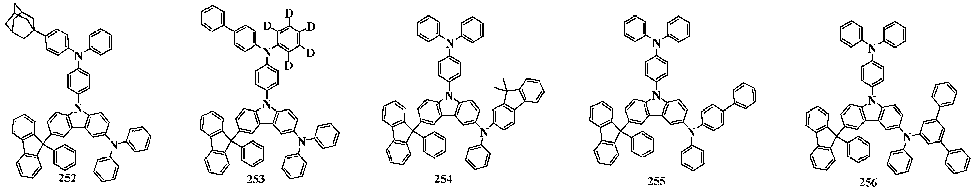

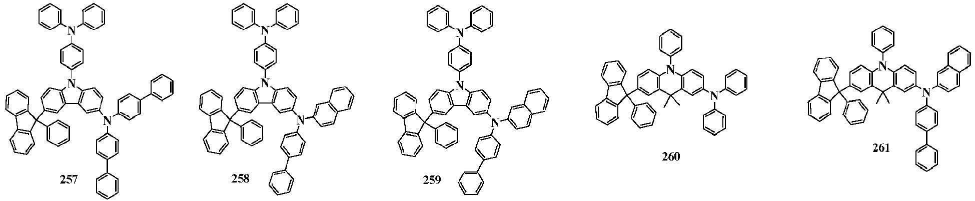

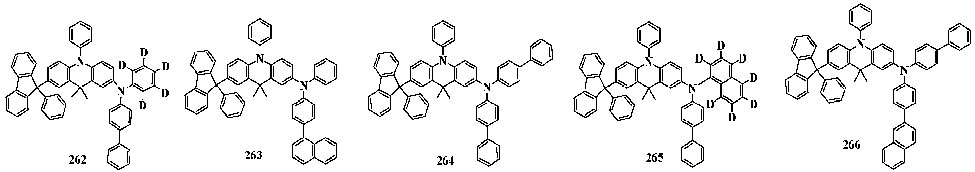

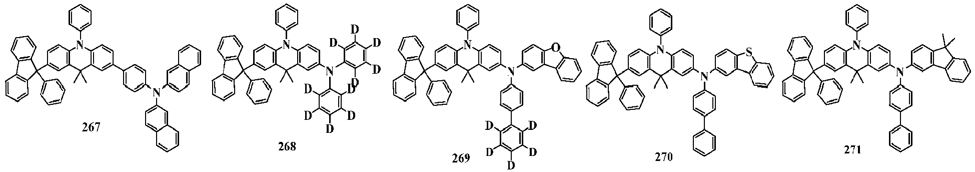

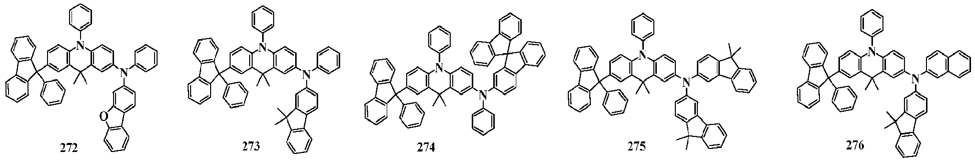

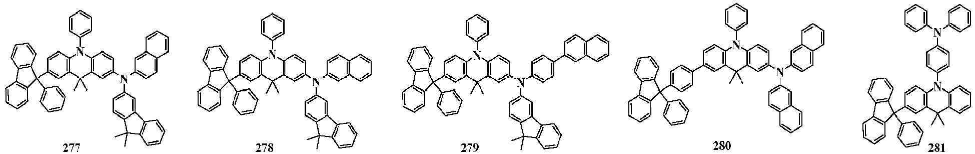

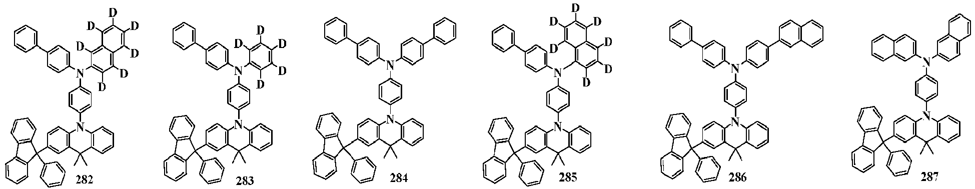

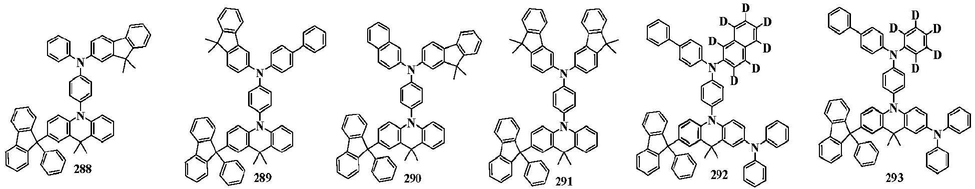

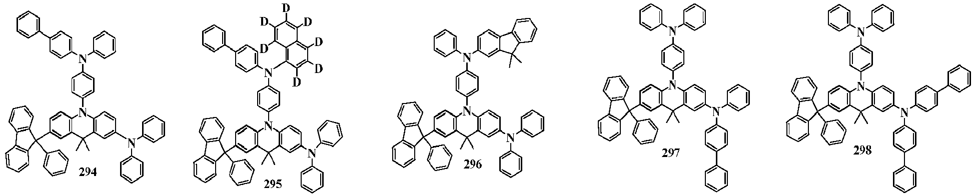

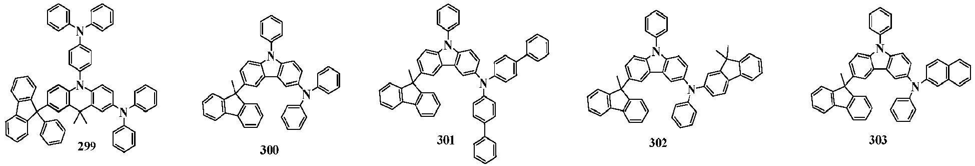

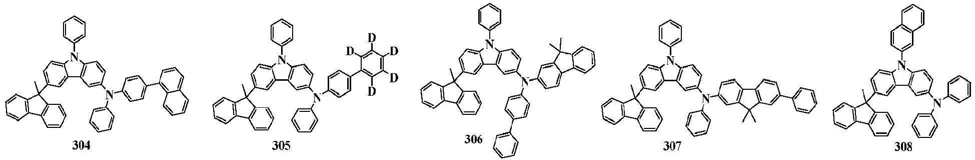

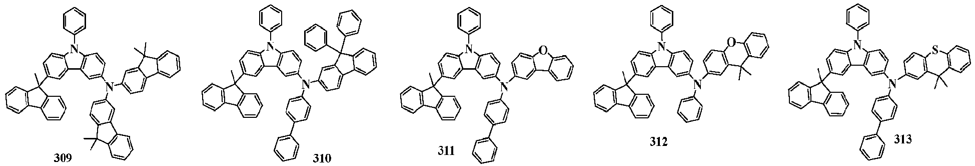

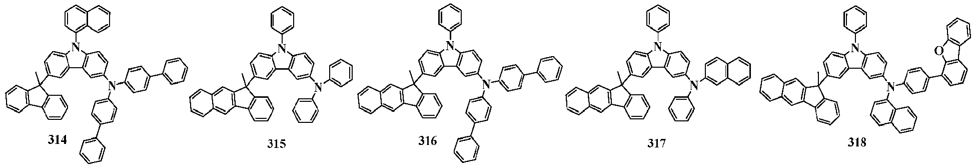

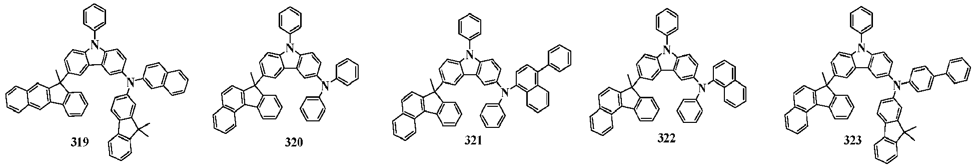

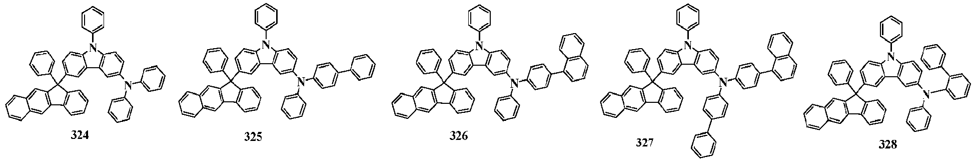

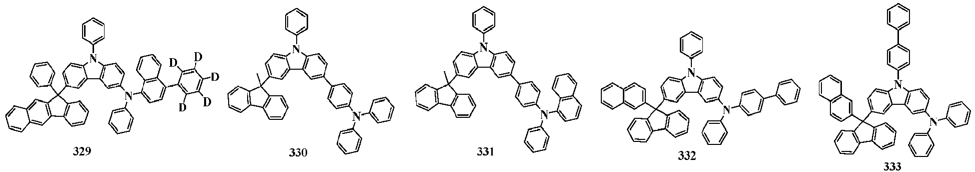

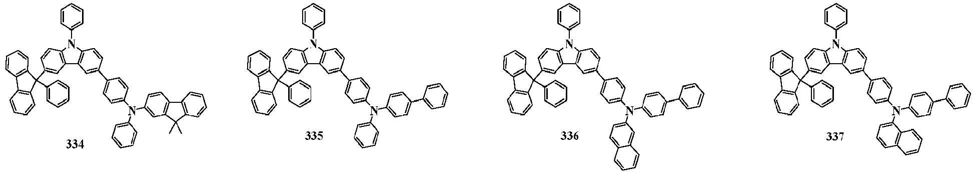

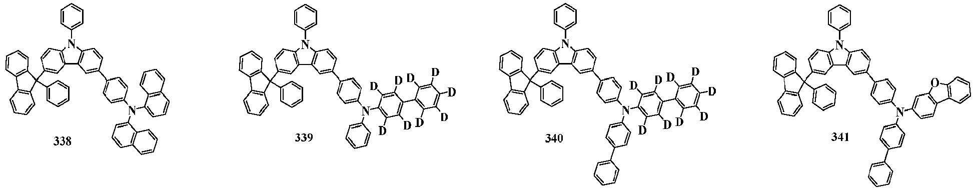

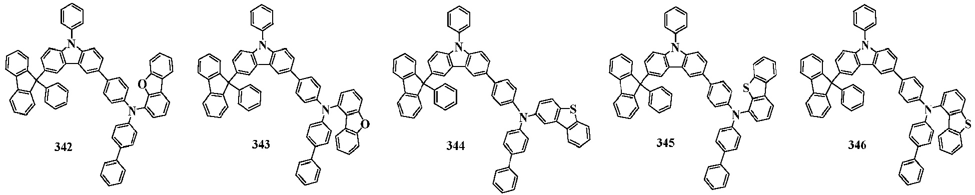

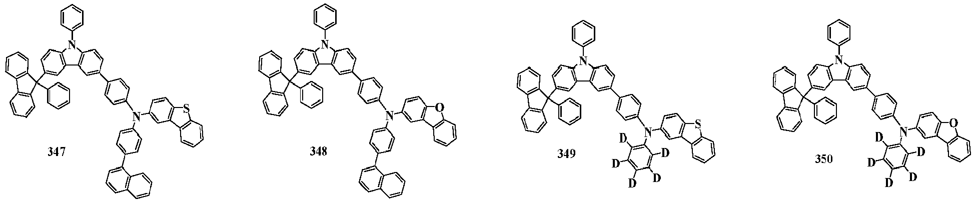

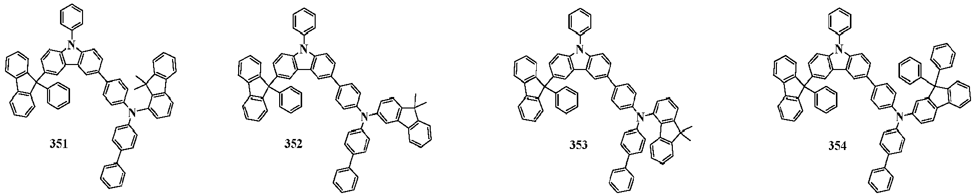

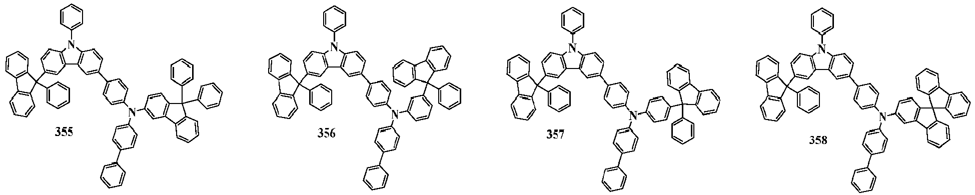

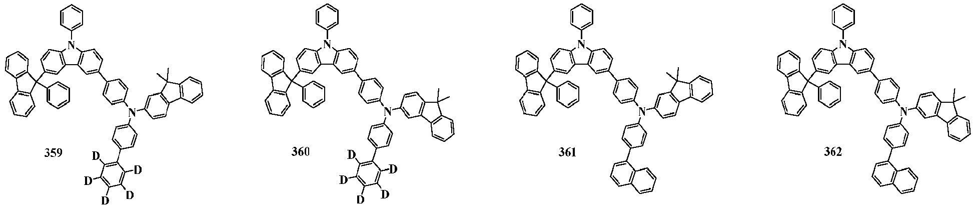

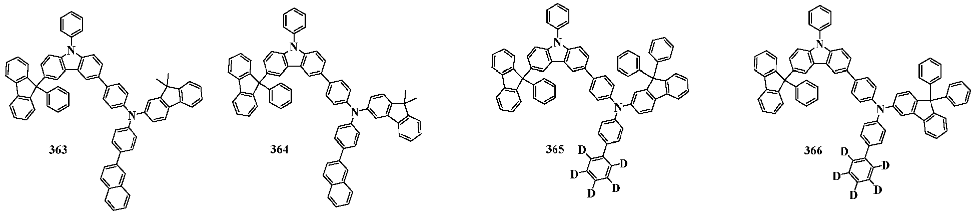

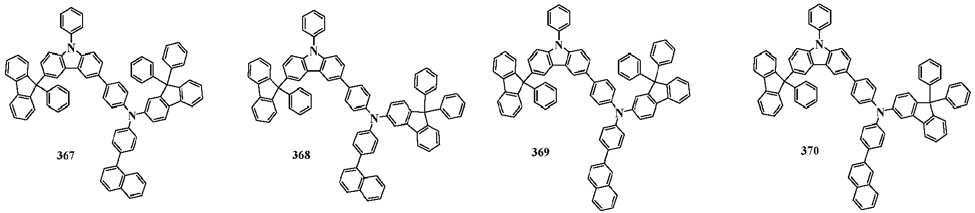

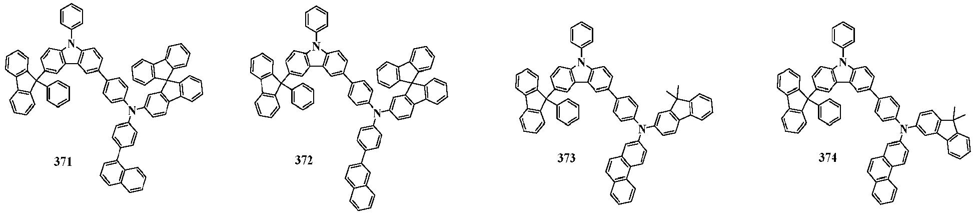

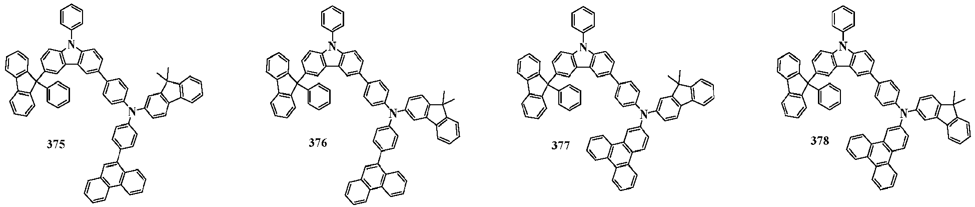

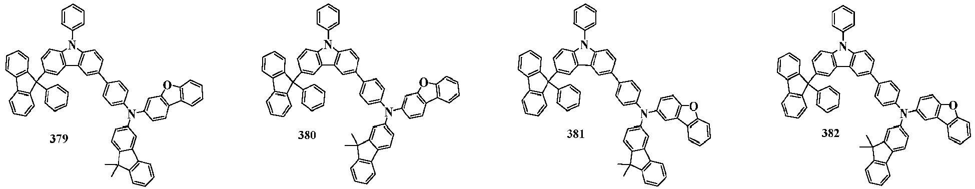

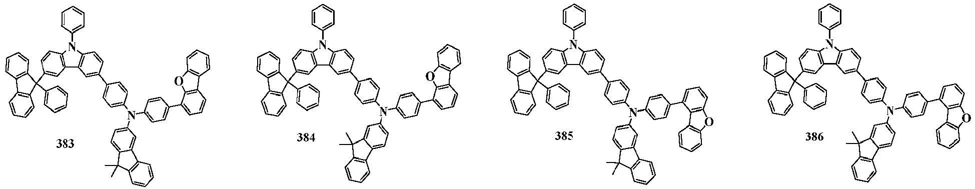

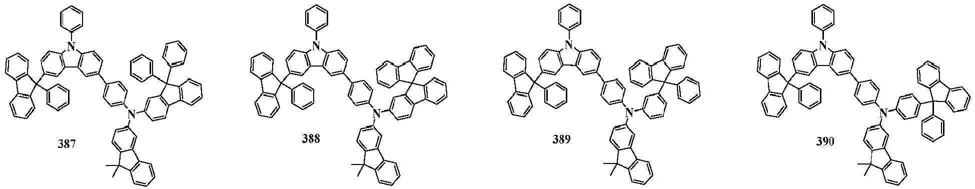

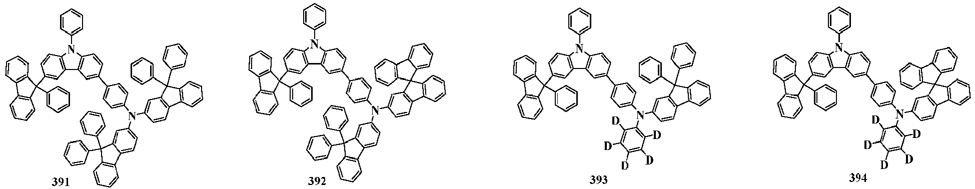

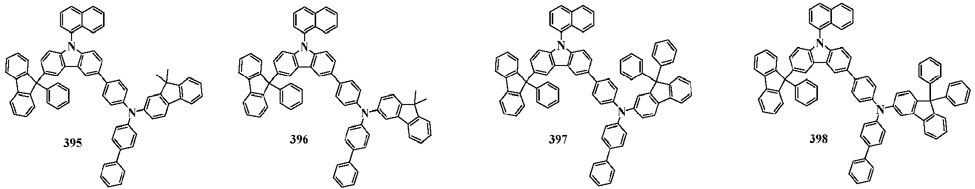

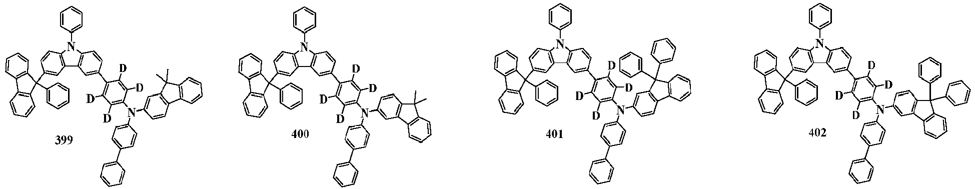

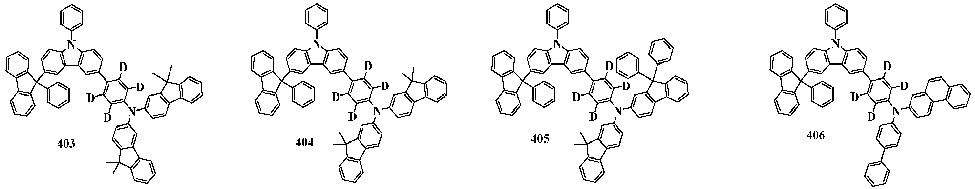

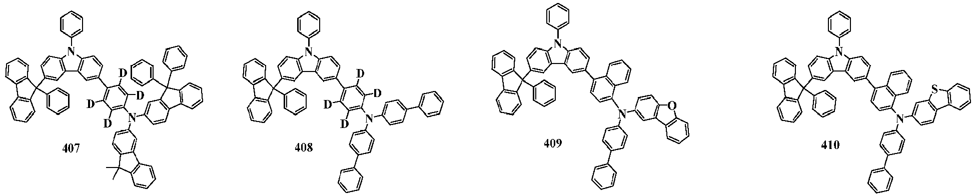

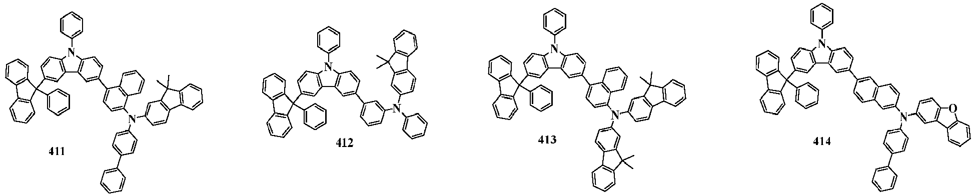

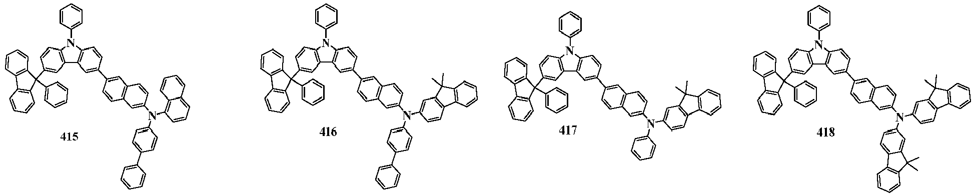

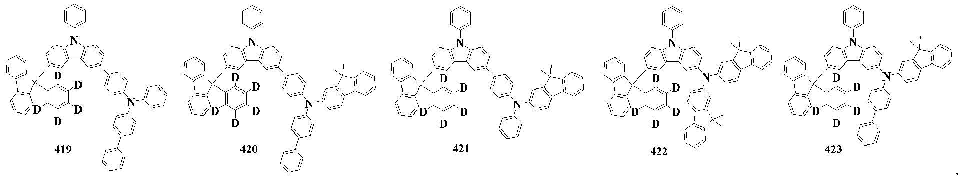

Some specific structures of the organic compound of the present disclosure are exemplified above. However, the present disclosure is not limited to these chemical structures. Any structures based on the structure represented by Formula I and having substituents which are groups defined above are included.

The present disclosure further provides a synthesis method of the compound of Formula I. A specific synthesis route is shown as follows:
1. When X is a single bond, the preparation method is shown by Route 1:

### [Route 1]

synthesis of Intermediate A and Intermediate A':

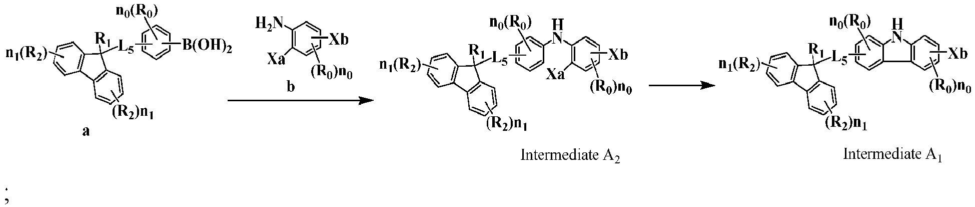

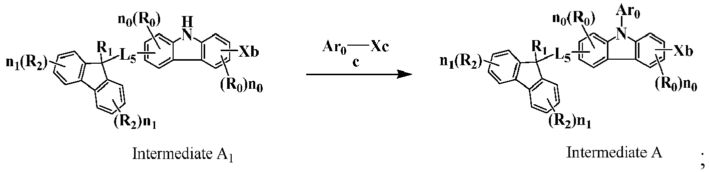

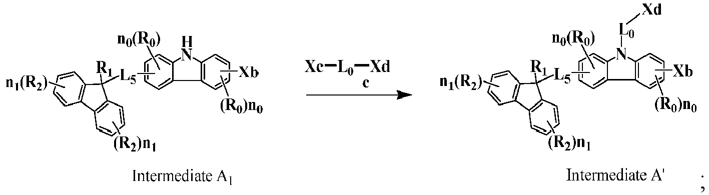

synthesis of Intermediate C and Intermediate C':

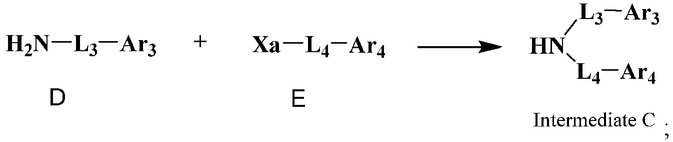

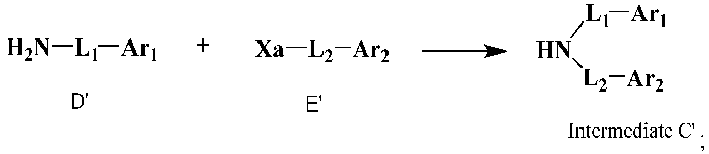

Intermediate A and Intermediate C, Intermediate A' and Intermediate C', Intermediate A' and Intermediate C, and Intermediate C' are subjected to a Buchwald-Hartwig arylamination reaction to synthesize the compound of Formula I in the present disclosure.

2. When X is C(R₃R₄), the preparation method is shown by Route 2:

### [Route 2]

(i) synthesis of Intermediate A, Intermediate A' and Intermediate A": 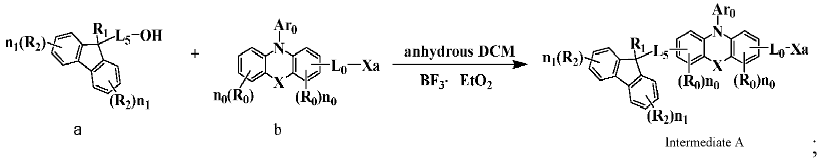 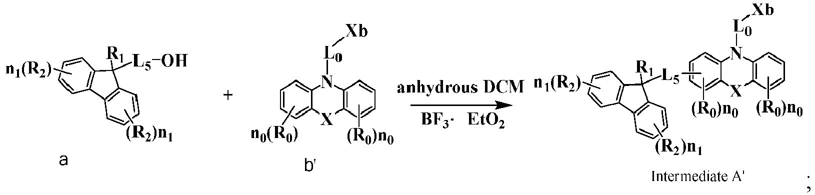 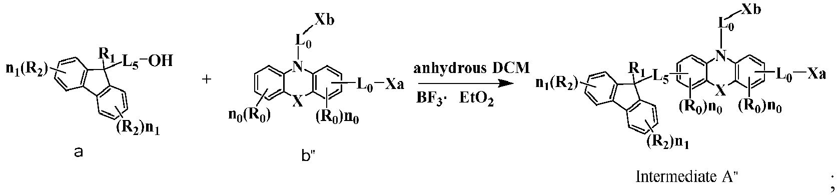
(ii) synthesis of Intermediate C and Intermediate C':

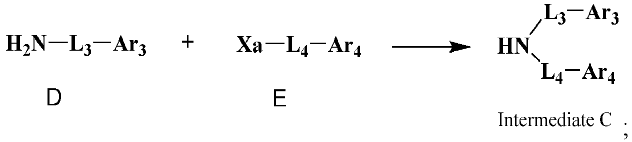

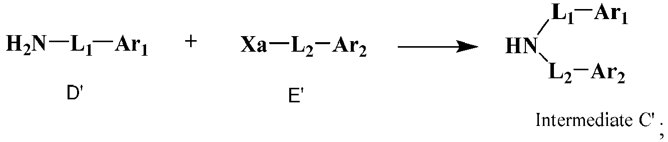

Intermediate A and Intermediate C, Intermediate A' and Intermediate C', Intermediate A" and Intermediate C, and Intermediate C' are subjected to a Buchwald-Hartwig arylamination reaction to synthesize the compound of Formula I in the present disclosure.

X, Ar₀, Ar₁ to Ar₄, L₀ to L₅, m₁, m₂, R₀, R₁ to R₄, n₀ and n₁ are defined the same as above, and Xa to Xd are independently selected from any one of H, I, Br or Cl.

The present disclosure further provides an organic electroluminescent device. The device includes an anode, a cathode and an organic layer, where the organic layer is disposed between the anode and the cathode or disposed on an outer side of one or more of the anode and the cathode, where the organic layer includes at least one of the triarylamine derivatives of the present disclosure.

Preferably, the organic electroluminescent device of the present disclosure may include one or more organic layers, where the organic layers may include a light-emitting layer, a hole injection layer, a hole transport layer, an electron transport layer, an electron injection layer, a hole blocking layer, an electron blocking layer, a capping layer and the like. Specifically, the organic layer disposed between the anode and the cathode may include the light-emitting layer, the hole injection layer, the hole transport layer, the electron transport layer, the electron injection layer, the hole blocking layer, the electron blocking layer and the like, and the organic layer disposed on the outer side of one or more of the anode and the cathode may include the capping layer and the like. The organic layer may be formed by a single-layer structure or may be formed by a multi-layer structure in which the above organic layers are stacked. Meanwhile, each of the organic layers may also include one layer or multiple layers. For example, the hole transport layer includes a first hole transport layer and a second hole transport layer. However, the structure of the organic electroluminescent device is not limited thereto and may include fewer or more organic layers.

Preferably, the hole transport layer of the present disclosure is selected from any one of a single-layer structure composed of a single compound, a single-layer structure composed of two or more compounds or a multi-layer structure composed of two or more compounds, and the hole transport layer contains at least one of the triarylamine derivatives of the present disclosure.

More preferably, the hole transport layer includes the first hole transport layer and the second hole transport layer, and the second hole transport layer contains at least one of the triarylamine derivatives of the present disclosure.

Still more preferably, the first hole transport layer is disposed on an upper side of the anode, the second hole transport layer is disposed on an upper side of the first hole transport layer, and the cathode is disposed on an upper side of the second hole transport layer.

Preferably, the capping layer of the present disclosure is selected from any one of a single-layer structure composed of a single compound, a single-layer structure composed of two or more compounds or a multi-layer structure composed of two or more compounds, and the capping layer contains at least one of the triarylamine derivatives of the present disclosure or contains a conventional capping layer material well-known to those skilled in the art.

Preferably, the organic electroluminescent device of the present disclosure has, but is not limited to, the following structure:
(1) anode/hole transport layer/light-emitting layer/electron transport layer/cathode
(2) anode/hole injection layer/hole transport layer/light-emitting layer/electron transport layer/electron injection layer/cathode
(3) anode/hole injection layer/hole transport layer/light-emitting layer/electron transport layer/electron injection layer/cathode/capping layer
(4) anode/hole injection layer/hole transport layer/light-emitting layer/hole blocking layer/electron transport layer/electron injection layer/cathode
(5) anode/hole injection layer/hole transport layer/electron blocking layer/light-emitting layer/electron transport layer/electron injection layer/cathode
(6) anode/hole injection layer/hole transport layer/electron blocking layer/light-emitting layer/electron transport layer/electron injection layer/cathode/capping layer
(7) anode/hole injection layer/hole transport layer/electron blocking layer/light-emitting layer/hole blocking layer/electron transport layer/electron injection layer/cathode
(8) anode/hole injection layer/hole transport layer/light-emitting layer/hole blocking layer/electron transport layer/electron injection layer/cathode/capping layer
(9) anode/hole injection layer/first hole transport layer/second hole transport layer/light-emitting layer/electron transport layer/electron injection layer/cathode
(10) anode/hole injection layer/first hole transport layer/second hole transport layer/light-emitting layer/electron transport layer/electron injection layer/cathode/capping layer

The anode of the present disclosure preferably uses a high work function material capable of promoting the injection of holes into the organic layer. Specific examples of anode materials may include, but are not limited to, a metal such as vanadium, chromium, copper, zinc and gold or an alloy thereof; a metal oxide such as zinc oxide, indium oxide, indium tin oxide (ITO) and indium zinc oxide (IZO); a combination of a metal and an oxide, such as ZnO:Al; and a conductive polymer such as poly(3-methylthiophene), poly[3,4-(ethylene-1,2-dioxy)thiophene] (PEDOT), polypyrrole and polyaniline.

The cathode of the present disclosure preferably uses a low work function material capable of promoting the injection of electrons into the organic layer. Specific examples of cathode materials may include, but are not limited to, a metal such as Ag, Mg, Cu, Al, Pt, Pd, Au, Ni, Nd, Ir, Cr, Li, Ca, LiF/Ca, LiF/Al, Mo and Ti or an alloy thereof; and a material with a multi-layer structure, such as LiF/Al or LiO₂/Al.

The hole injection layer of the present disclosure preferably uses a material having a good ability to accept holes. Specific examples include, but are not limited to, metalloporphyrin, oligothiophene, an organic material based on arylamine, an organic material based on hexacyanohexaazatriphenylene, an organic material based on quinacridone, an organic material based on perylene, anthraquinone, a conductive polymer based on polyaniline, a conductive polymer based on polythiophene and the like.

Generally, the hole transport layer of the present disclosure preferably uses a material having high hole mobility. In addition to the triarylamine derivative provided by the present disclosure, the material may also be selected from the following structures: a carbazole derivative, a triarylamine derivative, a biphenyldiamine derivative, a fluorene derivative, a phthalocyanine compound, a hexacyanohexaazatriphenylene compound, a quinacridone compound, an anthraquinone compound, polyaniline, polythiophene, polyethylene carbazole and the like, but the material is not limited thereto.

A material of the light-emitting layer of the present disclosure may include a host material (also referred to as a matrix material) and a doping material (also referred to as a guest material). The material of the light-emitting layer may include multiple host materials and multiple doping materials. The light-emitting layer may be a single light-emitting layer or may be a composite light-emitting layer stacked together in a horizontal or vertical direction. The type of the doping material may be a fluorescent material or a phosphorescent material. The amount of the doping material is preferably 0.1% to 70% by mass, more preferably 0.1% to 30% by mass, still more preferably 1% to 30% by mass, further more preferably 1% to 20% by mass, and most preferably 1% to 10% by mass.

A fluorescent doping material that can be used in the present disclosure may include, but is not limited to, a fused polycyclic aromatic derivative, a styrylamine derivative, a fused-ring amine derivative, a boron-containing compound, a pyrrole derivative, an indole derivative, a carbazole derivative and the like. A phosphorescent doping material that can be used in the present disclosure may include, but is not limited to, a heavy metal complex, a phosphorescent rare earth metal complex and the like. The heavy metal complex may include, for example, an iridium complex, a platinum complex, an osmium complex and the like. The rare earth metal complex may include, for example, a terbium complex, a europium complex and the like, but it is not limited thereto.

The host material that can be used in the present disclosure may include a fused aromatic ring derivative, a heterocycle-containing compound and the like. Specifically, the fused aromatic ring derivative includes an anthracene derivative, a pyrene derivative, a naphthalene derivative, a pentacene derivative, a phenanthrene derivative, a fluoranthene derivative and the like, and the heterocycle-containing compound includes a carbazole derivative, a dibenzofuran derivative, a dibenzothiophene derivative, a pyrimidine derivative and the like, but it is not limited thereto.

The electron transport layer of the present disclosure preferably uses a material having a relatively strong electron-withdrawing ability and relatively low HOMO and LUMO energy levels. Specific examples may include, but are not limited to, quinolines, imidazoles, phenanthroline derivatives, triazoles and other materials, such as 2,9-(dimethyl)-4,7-biphenyl-1,10-phenanthroline (BCP), 8-hydroxyquinolinolato-lithium (LiQ), 1,3,5-tris(1-phenyl-1H-benzimidazol-2-yl)benzene (TPBi), 3-(biphen-4-yl)-5-(4-tert-butylphenyl)-4-phenyl-4H-1,2,4-triazole (TAZ), 4,4'-bis(4,6-diphenyl-1,3,5-tiiazinyl)biphenyl (BTB), 1,3,5-tris[(3-pyridyl)-phenyl]benzene (TmPyPB), 2-(naphthalen-2-yl)-4,7-(diphenyl)-1,10-phenanthroline (HNBphen) and the like.

The electron injection layer of the present disclosure preferably uses a material having a relatively small potential difference from an adjacent organic transport material, host material, or the like. Specific examples may include, but are not limited to, a metal oxide such as Al₂O₃ and MoO₃, an alkaline metal salt such as LiF and CsF, and an alkaline earth metal salt such as MgF₂.

The organic electroluminescent device of the present disclosure may be manufactured by sequentially stacking the preceding structures. The manufacturing method may be well-known methods such as a wet film formation method and a dry film formation method. Specific examples of the wet film formation method may include various coating methods such as spin-coating, impregnation, casting and ink-jetting. Specific examples of the dry film formation method may include vacuum vapor deposition, sputtering, plasma, ion plating and the like, but it is not limited thereto.

An organic light-emitting device in the present disclosure may be widely applied to the fields of panel display, lighting sources, flexible OLEDs, electronic paper, organic solar cells, organic photoreceptors or organic thin-film transistors, signs, traffic lights and the like.

The manufacturing of the preceding organic electroluminescent device is specifically described in the following examples. However, the following examples are merely for illustrating the specification, and the scope of the specification is not limited to these examples.

### Preparation and characterization of compounds

Description of raw materials, reagents and characterization devices:
The sources of raw materials used in the following examples are not particularly limited, and the raw materials may be commercially available products or be prepared by preparation methods well-known to those skilled in the art.

Mass spectrometry is conducted using a G2-Si quadrupole tandem time-of-flight high-resolution mass spectrometer from Waters Co., Britain, with chloroform as a solvent.

An elemental analysis is conducted using Vario EL cube Elemental Analyzer from Elementar Co., Germany, with a sample mass of 5 to 10 mg.

### [Synthesis Example 1] Synthesis of Intermediate A-1

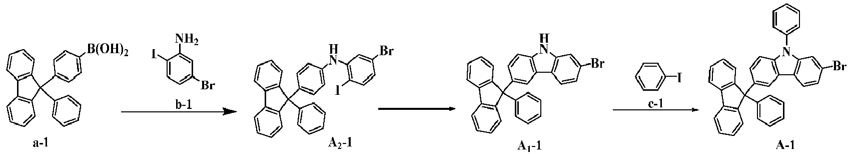

a-1 (30.79 g, 85 mmol), b-1 (23.83 g, 80 mmol), dried Cu(OAc)₂·H₂O (1.60 g, 8.0 mmol) and n-decanoic acid (2.75 g, 16 mmol) were added to a CaCh-protected reaction flask, and then DBU (14.81 g, 96 mmol) and anhydrous toluene (250 mL) were sequentially added to the reaction mixture and stirred at room temperature for 24 h. After the reaction was completed, the reaction mixture was diluted with water (150 mL) and extracted with ethyl acetate (2 × 150 mL). The combined ethyl acetate fractions were washed with brine (150 mL), dried over anhydrous Na₂SO₄, and filtered. The filtrate was concentrated under reduced pressure. The crude product was separated, purified and refined through column chromatography with hexane/ethyl acetate (99:1) as eluents to obtain Intermediate A₂-1 (36.86 g, with a yield of 75%). The solid purity detected through HPLC was greater than or equal to 99.5%.

Intermediate A₂-1 (36.74 g, 60 mmol), anhydrous toluene (300 mL), Pd(OAc)₂ (0.67 g, 3.0 mmol) and DBU (55.53 g, 360 mmol) were sequentially added to a reaction flask, and the reaction mixture was degassed with nitrogen and heated at 100 °C for 12 h. After the reaction was completed, the reaction mixture was diluted with water (300 mL) at room temperature. The reaction mixture was diluted with water (150 mL) and extracted with ethyl acetate (2 × 150 mL). The combined ethyl acetate fractions were washed with brine (150 mL), dried over anhydrous Na₂SO₄, and filtered. The filtrate was concentrated under reduced pressure. The crude product was separated, purified and refined through column chromatography with hexane/ethyl acetate (90:10) as eluents to obtain Intermediate A₁-1 (24.22 g, with a yield of 83%). The solid purity detected through HPLC was greater than or equal to 99.4%.

Intermediate A₁-1 (19.46 g, 40 mmol), toluene (200 mL), c-1 (8.16 g, 40 mmol), Pd₂(dba)₃ (0.36 g, 0.40 mmol), BINAP (0.74 g, 1.20 mmol) and sodium tert-butoxide (7.68 g, 80 mmol) were added to a reaction flask, stirred and dissolved, and refluxed under nitrogen protection for 7 h. After the reaction was completed, the mixture was cooled to room temperature and filtered. The filtrate was distilled under reduced pressure to remove an organic solvent. The obtained solid was recrystallized from methanol to obtain Intermediate **A-1** (18.0 g, with a yield of 80%). The solid purity detected through HPLC was greater than or equal to 99.6%. Mass spectrometry (m/z): 561.1129 (Calcd.: 561.1092).

### [Synthesis Example 2] Synthesis of Intermediate A-2

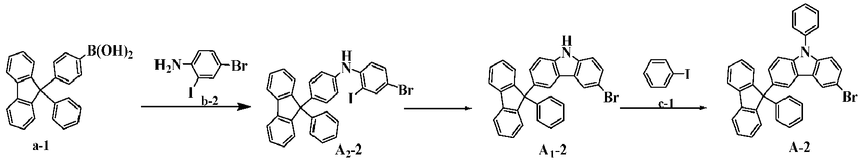

Intermediate A-2 (18.23 g) was synthesized by the same method as Synthesis Example 1 except that b-1 was replaced with an equimolar amount of b-2. The solid purity detected through HPLC was greater than or equal to 99.7%. Mass spectrometry (m/z): 561.1135 (Calcd.: 561.1092).

### [Synthesis Example 3] Synthesis of Intermediate A-3

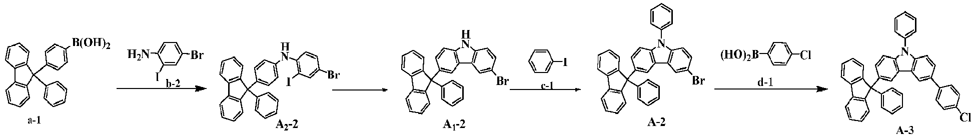

Intermediate A-2 (16.88 g, 30 mmol), tetrahydrofuran (200 mL) and d-1 (4.69 g, 30 mmol) were added to a reaction flask, then an aqueous solution of 2 M potassium carbonate (100 mL) was added, and Pd(PPh₃)₄ (0.46 g, 0.4 mmol) was added, stirred and dissolved, and refluxed under nitrogen protection for 5 h. After the reaction was completed, the reactant was cooled to room temperature, subjected to rotary evaporation under reduced pressure to remove tetrahydrofuran, and extracted with ethyl acetate and water. The organic layer was distilled under reduced pressure to remove an organic solvent. The obtained solid was recrystallized from ethyl acetate to finally obtain Intermediate A-3 (15.86 g, with a yield of 89%). The solid purity detected through HPLC was greater than or equal to 99.6%. Mass spectrometry (m/z): 593.1953 (Calcd.: 593.1910).

### [Synthesis Example 4] Synthesis of Intermediate A-4

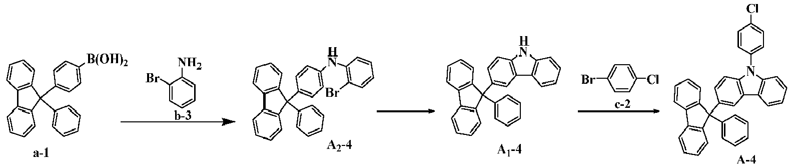

Intermediate A-4 (16.99 g) was synthesized by the same method as Synthesis Example 1 except that b-1 was replaced with an equimolar amount of b-3 and c-1 was replaced with an equimolar amount of c-2. The solid purity detected through HPLC was greater than or equal to 99.5%. Mass spectrometry (m/z): 517.1620 (Calcd.: 517.1597).

### [Synthesis Example 5] Synthesis of Intermediate A-5

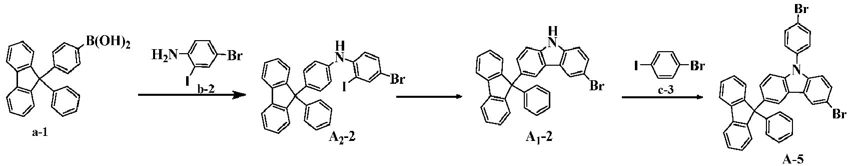

Intermediate A-5 (20.01 g) was synthesized by the same method as Synthesis Example 1 except that b-1 was replaced with an equimolar amount of b-2 and c-1 was replaced with an equimolar amount of c-3. The solid purity detected through HPLC was greater than or equal to 99.2%. Mass spectrometry (m/z): 595.0741 (Calcd.: 595.0702).

### [Synthesis Example 6] Synthesis of Intermediate A-6

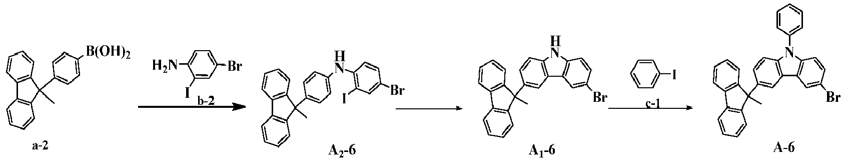

Intermediate A-6 (16.21 g) was synthesized by the same method as Synthesis Example 1 except that a-1 was replaced with an equimolar amount of a-2 and b-1 was replaced with an equimolar amount of b-2. The solid purity detected through HPLC was greater than or equal to 99.5%. Mass spectrometry (m/z): 499.0896 (Calcd.: 499.0936).

### [Synthesis Example 7] Synthesis of Intermediate A-7

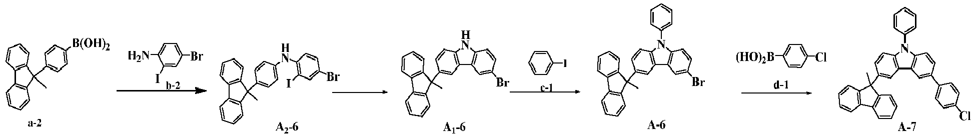

Intermediate A-7 (14.37 g) was synthesized by the same method as Synthesis Example 3 except that a-1 was replaced with an equimolar amount of a-2. The solid purity detected through HPLC was greater than or equal to 99.7%. Mass spectrometry (m/z): 531.1709 (Calcd.: 531.1754).

### [Synthesis Example 8] Synthesis of Compound 1

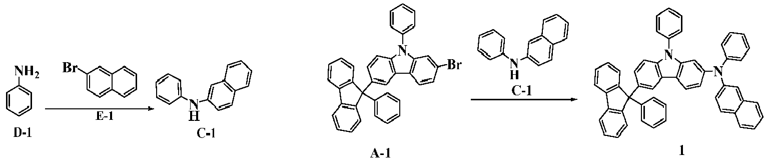

### Synthesis of Intermediate C-1

Under nitrogen protection, toluene as a solvent (200 mL), D-1 (3.72 g, 40 mmol), E-1 (8.28 g, 40 mmol), Pd₂(dba)₃ (0.36 g, 0.40 mmol), BINAP (0.74 g, 1.20 mmol) and sodium tert-butoxide (7.68 g, 80 mmol) were sequentially added to a reaction flask, stirred and dissolved, and refluxed under nitrogen protection for 7 h. After the reaction was completed, the mixture was cooled to room temperature and filtered through Celite to obtain a filtrate. The filtrate was distilled under reduced pressure to remove an organic solvent. The obtained solid was recrystallized from methanol to finally obtain Intermediate C-1 (7.19 g, with a yield of 82%). The solid purity detected through HPLC was greater than or equal to 99.6%.

### Synthesis of Compound 1

Under nitrogen protection, toluene as a solvent (250 mL), Intermediate C-1 (6.57 g, 30 mmol), Intermediate A-1 (16.87 g, 30 mmol), Pd₂(dba)₃ (0.27 g, 0.30 mmol), BINAP (0.56 g, 0.90 mmol) and sodium tert-butoxide (5.76 g, 60 mmol) were sequentially added to a reaction flask, stirred and dissolved, and refluxed under nitrogen protection for 8 h. After the reaction was completed, the mixture was cooled to room temperature and filtered through Celite to obtain a filtrate. The filtrate was concentrated. The obtained solid was recrystallized from ethyl acetate to finally obtain Compound 1 (16.40 g, with a yield of 78%). The solid purity detected through HPLC was greater than or equal to 99.6%.

Mass spectrometry (m/z): 700.2855 (Calcd.: 700.2878). Theoretical element content (%) of C₅₃H₃₆N₂: C, 90.83; H, 5.18; N, 4.00. Measured element content (%): C, 90.78; H, 5.12; N, 4.04.

### [Synthesis Example 9] Synthesis of Compound 3

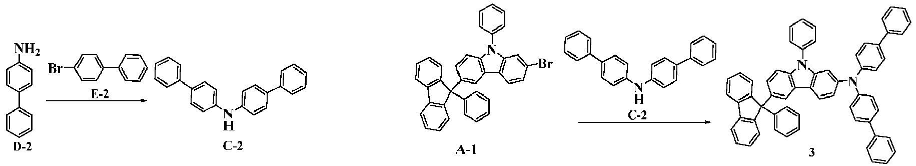

Compound 3 (18.79 g) was synthesized by the same method as Synthesis Example 8 except that D-1 was replaced with an equimolar amount of D-2 and E-1 was replaced with an equimolar amount of E-2. The solid purity detected through HPLC was greater than or equal to 99.4%. Mass spectrometry (m/z): 802.3379 (Calcd.: 802.3348). Theoretical element content (%) of C₆₁H₄₂N₂: C, 91.24; H, 5.27; N, 3.49. Measured element content (%): C, 91.17; H, 5.22; N, 3.54.

### [Synthesis Example 10] Synthesis of Compound 17

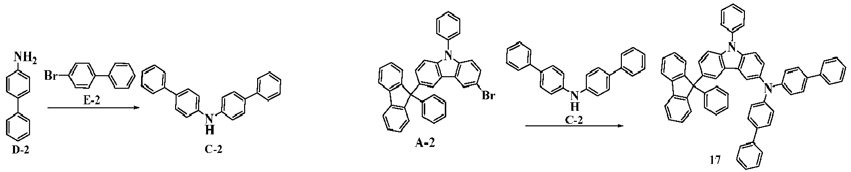

Compound 17 (18.54 g) was synthesized by the same method as Synthesis Example 8 except that D-1 was replaced with an equimolar amount of D-2, E-1 was replaced with an equimolar amount of E-2, and Intermediate A-1 was replaced with an equimolar amount of Intermediate A-2. The solid purity detected through HPLC was greater than or equal to 99.7%. Mass spectrometry (m/z): 802.3386 (Calcd.: 802.3348). Theoretical element content (%) of C₆₁H₄₂N₂: C, 91.24; H, 5.27; N, 3.49. Measured element content (%): C, 91.18; H, 5.24; N, 3.53.

### [Synthesis Example 11] Synthesis of Compound 28

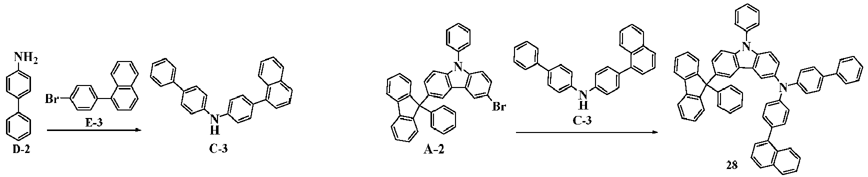

Compound 28 (19.96 g) was synthesized by the same method as Synthesis Example 8 except that D-1 was replaced with an equimolar amount of D-2, E-1 was replaced with an equimolar amount of E-3, and Intermediate A-1 was replaced with an equimolar amount of Intermediate A-2. The solid purity detected through HPLC was greater than or equal to 99.6%. Mass spectrometry (m/z): 852.3015 (Calcd.: 852.3504). Theoretical element content (%) of C₆₅H₄₄N₂: C, 91.52; H, 5.20; N, 3.28. Measured element content (%): C, 91.46; H, 5.24; N, 3.35.

### [Synthesis Example 12] Synthesis of Compound 39

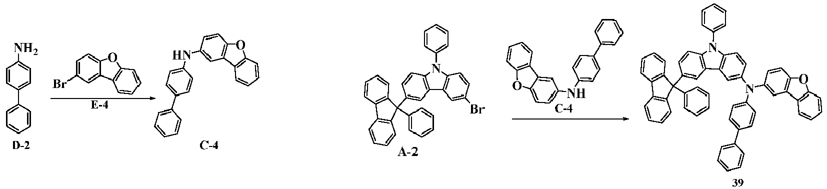

Compound 39 (18.36 g) was synthesized by the same method as Synthesis Example 8 except that D-1 was replaced with an equimolar amount of D-2, E-1 was replaced with an equimolar amount of E-4, and Intermediate A-1 was replaced with an equimolar amount of Intermediate A-2. The solid purity detected through HPLC was greater than or equal to 99.6%. Mass spectrometry (m/z): 816.3115 (Calcd.: 816.3141). Theoretical element content (%) of C₆₁H₄₀N₂O: C, 89.68; H, 4.94; N, 3.43. Measured element content (%): C, 89.74; H, 5.01; N, 3.37.

### [Synthesis Example 13] Synthesis of Compound 48

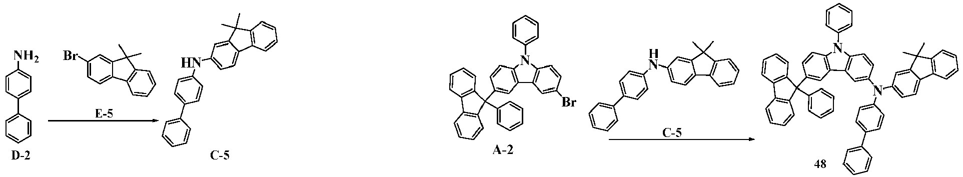

Compound 48 (18.95 g) was synthesized by the same method as Synthesis Example 8 except that D-1 was replaced with an equimolar amount of D-2, E-1 was replaced with an equimolar amount of E-5, and Intermediate A-1 was replaced with an equimolar amount of Intermediate A-2. The solid purity detected through HPLC was greater than or equal to 99.6%. Mass spectrometry (m/z): 842.3629 (Calcd.: 842.3661). Theoretical element content (%) of C₆₄H₄₆N₂: C, 91.18; H, 5.50; N, 3.32. Measured element content (%): C, 91.25; H, 5.54; N, 3.28.

### [Synthesis Example 14] Synthesis of Compound 59

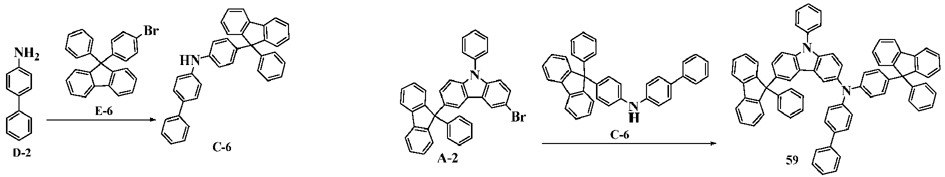

Compound 59 (21.74 g) was synthesized by the same method as Synthesis Example 8 except that D-1 was replaced with an equimolar amount of D-2, E-1 was replaced with an equimolar amount of E-6, and Intermediate A-1 was replaced with an equimolar amount of Intermediate A-2. The solid purity detected through HPLC was greater than or equal to 99.3%. Mass spectrometry (m/z): 966.3938 (Calcd.: 966.3974). Theoretical element content (%) of C₇₄H₅₀N₂: C, 91.89; H, 5.21; N, 2.90. Measured element content (%): C, 91.95; H, 5.17; N, 2.94.

### [Synthesis Example 15] Synthesis of Compound 72

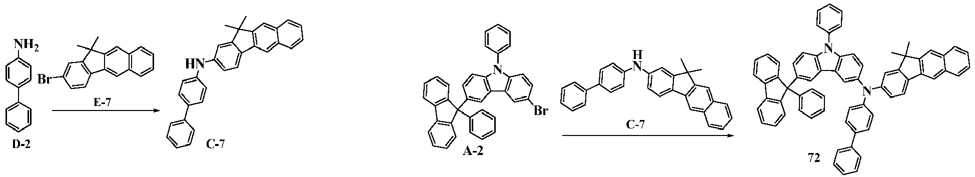

Compound 72 (20.07 g) was synthesized by the same method as Synthesis Example 8 except that D-1 was replaced with an equimolar amount of D-2, E-1 was replaced with an equimolar amount of E-7, and Intermediate A-1 was replaced with an equimolar amount of Intermediate A-2. The solid purity detected through HPLC was greater than or equal to 99.3%. Mass spectrometry (m/z): 892.3843 (Calcd.: 892.3817). Theoretical element content (%) of C₆₈H₄₈N₂: C, 91.45; H, 5.42; N, 3.14. Measured element content (%): C, 91.52; H, 5.47; N, 3.08.

### [Synthesis Example 16] Synthesis of Compound 78

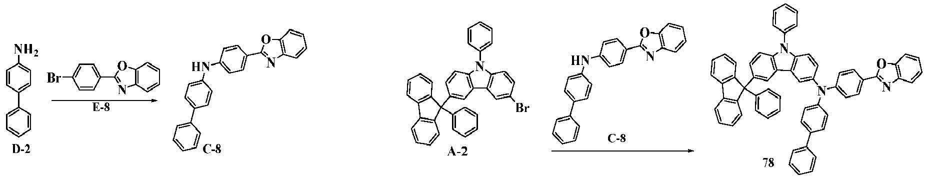

Compound 78 (18.97 g) was synthesized by the same method as Synthesis Example 8 except that D-1 was replaced with an equimolar amount of D-2, E-1 was replaced with an equimolar amount of E-8, and Intermediate A-1 was replaced with an equimolar amount of Intermediate A-2. The solid purity detected through HPLC was greater than or equal to 99.5%. Mass spectrometry (m/z): 843.3216 (Calcd.: 843.3250). Theoretical element content (%) of C₆₂H₄₁N₃O: C, 88.23; H, 4.90; N, 4.98. Measured element content (%): C, 88.18; H, 4.84; N, 5.03.

### [Synthesis Example 17] Synthesis of Compound 90

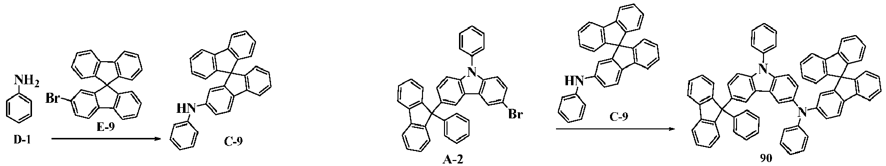

Compound 90 (19.98 g) was synthesized by the same method as Synthesis Example 8 except that E-1 was replaced with an equimolar amount of E-9 and Intermediate A-1 was replaced with an equimolar amount of Intermediate A-2. The solid purity detected through HPLC was greater than or equal to 99.7%. Mass spectrometry (m/z): 888.3527 (Calcd.: 888.3504). Theoretical element content (%) of C₆₈H₄₄N₂: C, 91.86; H, 4.99; N, 3.15. Measured element content (%): C, 91.93; H, 5.05; N, 3.12.

### [Synthesis Example 18] Synthesis of Compound 115

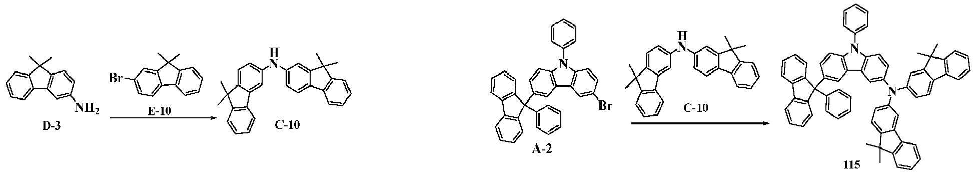

Compound 115 (19.85 g) was synthesized by the same method as Synthesis Example 8 except that D-1 was replaced with an equimolar amount of D-3, E-1 was replaced with an equimolar amount of E-10, and Intermediate A-1 was replaced with an equimolar amount of Intermediate A-2. The solid purity detected through HPLC was greater than or equal to 99.8%. Mass spectrometry (m/z): 882.3938 (Calcd.: 882.3974). Theoretical element content (%) of C₆₇H₅₀N₂: C, 91.12; H, 5.71; N, 3.17. Measured element content (%): C, 91.05; H, 5.65; N, 3.23.

### [Synthesis Example 19] Synthesis of Compound 141

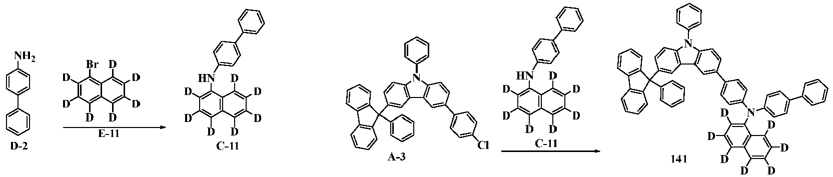

Compound 141 (19.31 g) was synthesized by the same method as Synthesis Example 8 except that D-1 was replaced with an equimolar amount of D-2, E-1 was replaced with an equimolar amount of E-11, and Intermediate A-1 was replaced with an equimolar amount of Intermediate A-3. The solid purity detected through HPLC was greater than or equal to 99.5%. Mass spectrometry (m/z): 858.4019 (Calcd.: 858.3991). Theoretical element content (%) of C₆₆H₃₈D₇N: C, 92.27; H, 6.10; N, 1.63. Measured element content (%): C, 92.34; H, 6.14; N, 1.58.

### [Synthesis Example 20] Synthesis of Compound 153

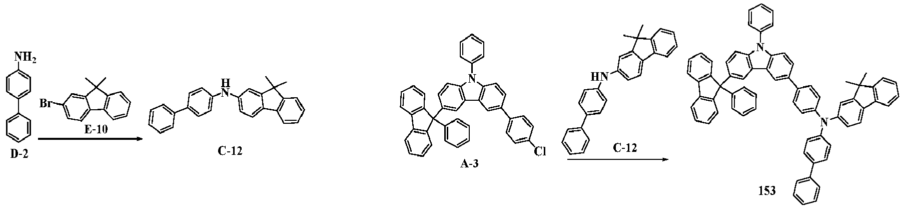

Compound 153 (20.66 g) was synthesized by the same method as Synthesis Example 8 except that D-1 was replaced with an equimolar amount of D-2, E-1 was replaced with an equimolar amount of E-10, and Intermediate A-1 was replaced with an equimolar amount of Intermediate A-3. The solid purity detected through HPLC was greater than or equal to 99.6%. Mass spectrometry (m/z): 918.3946 (Calcd.: 918.3974). Theoretical element content (%) of C₇₀H₅₀N₂: C, 91.47; H, 5.48; N, 3.05. Measured element content (%): C, 91.54; H, 5.53; N, 2.99.

### [Synthesis Example 21] Synthesis of Compound 161

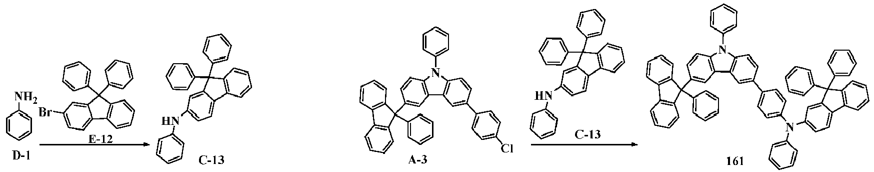

Compound 161 (22.61 g) was synthesized by the same method as Synthesis Example 8 except that E-1 was replaced with an equimolar amount of E-12 and Intermediate A-1 was replaced with an equimolar amount of Intermediate A-3. The solid purity detected through HPLC was greater than or equal to 99.4%. Mass spectrometry (m/z): 966.4011 (Calcd.: 966.3974). Theoretical element content (%) of C₇₄H₅₀N₂: C, 91.89; H, 5.21; N, 2.90. Measured element content (%): C, 91.95; H, 5.25; N, 2.85.

### [Synthesis Example 22] Synthesis of Compound 170

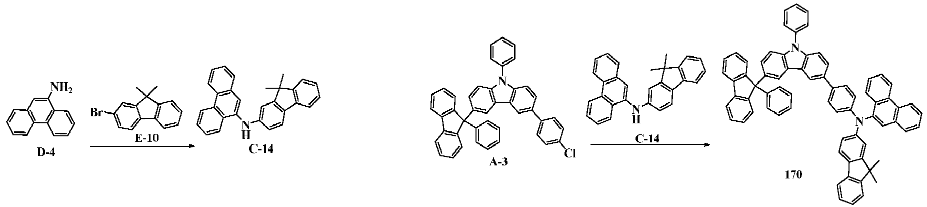

Compound 170 (21.76 g) was synthesized by the same method as Synthesis Example 8 except that D-1 was replaced with an equimolar amount of D-4, E-1 was replaced with an equimolar amount of E-10, and Intermediate A-1 was replaced with an equimolar amount of Intermediate A-3. The solid purity detected through HPLC was greater than or equal to 99.6%. Mass spectrometry (m/z): 942.4001 (Calcd.: 942.3974). Theoretical element content (%) of C₇₂H₅₀N₂: C, 91.69; H, 5.34; N, 2.97. Measured element content (%): C, 91.75; H, 5.40; N, 2.94.

### [Synthesis Example 23] Synthesis of Compound 219

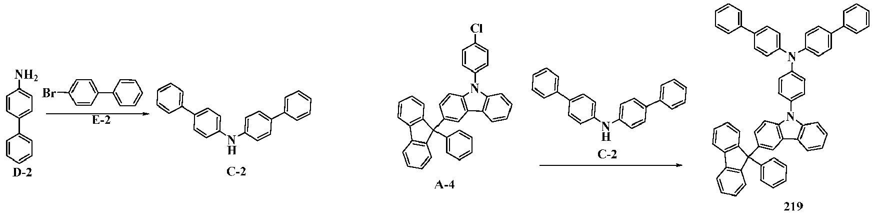

Compound 219 (12.20 g, with a yield of 76%) was synthesized by the same method as Synthesis Example 8 except that D-1 was replaced with an equimolar amount of D-2, E-1 was replaced with an equimolar amount of E-2, and Intermediate A-1 was replaced with an equimolar amount of Intermediate A-4. The solid purity detected through HPLC was greater than or equal to 99.7%. Mass spectrometry (m/z): 802.3309 (Calcd.: 802.3348). Theoretical element content (%) of C₆₁H₄₂N₂: C, 91.24; H, 5.27; N, 3.49. Measured element content (%): C, 91.31; H, 5.34; N, 3.45.

### [Synthesis Example 24] Synthesis of Compound 240

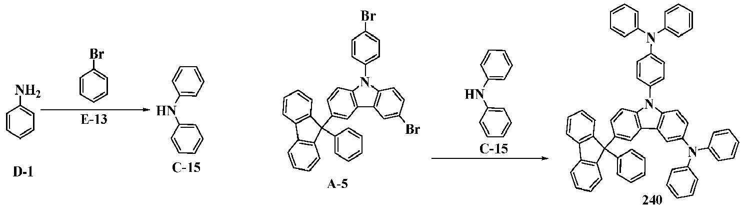

Under nitrogen protection, toluene as a solvent (250 mL), Intermediate C-15 (8.46 g, 50 mmol), Intermediate A-5 (16.04 g, 25 mmol), Pd₂(dba)₃ (0.45 g, 0.50 mmol), BINAP (0.93 g, 1.5 mmol) and sodium tert-butoxide (9.61 g, 100 mmol) were sequentially added to a reaction flask, stirred and dissolved, and refluxed under nitrogen protection for 8 h. After the reaction was completed, the mixture was cooled to room temperature and filtered through Celite to obtain a filtrate. The filtrate was concentrated. The obtained solid was recrystallized from ethyl acetate to obtain Compound 240 (14.72 g, with a yield of 72%). The solid purity detected through HPLC was greater than or equal to 99.7%. Mass spectrometry (m/z): 817.3429 (Calcd.: 817.3457). Theoretical element content (%) of C₆₁H₄₃N₃: C, 89.56; H, 5.30; N, 5.14. Measured element content (%): C, 89.63; H, 5.35; N, 5.09.

### [Synthesis Example 25] Preparation of Compound 301

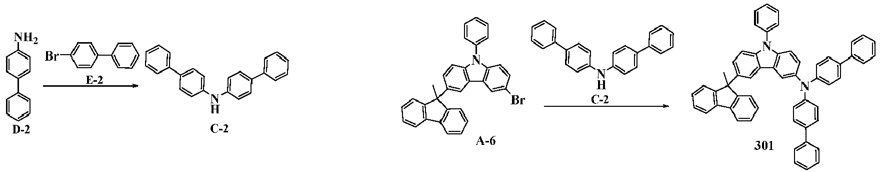

Compound 301 (17.78 g) was synthesized by the same method as Synthesis Example 8 except that E-1 was replaced with an equimolar amount of E-2, D-1 was replaced with an equimolar amount of D-2, and Intermediate A-1 was replaced with an equimolar amount of Intermediate A-6. The solid purity detected through HPLC was greater than or equal to 99.8%. Mass spectrometry (m/z): 740.3215 (Calcd.: 740.3191). Theoretical element content (%) of C₅₆H₄₀N₂: C, 90.78; H, 5.44; N, 3.78. Measured element content (%): C, 90.80; H, 5.45; N, 3.75.

### [Synthesis Example 26] Synthesis of Compound 312

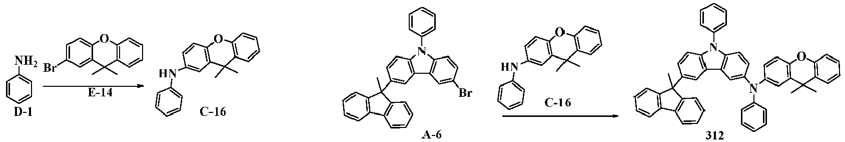

Compound 312 (16.85 g) was synthesized by the same method as Synthesis Example 8 except that E-1 was replaced with an equimolar amount of E-14 and Intermediate A-1 was replaced with an equimolar amount of Intermediate A-6. The solid purity detected through HPLC was greater than or equal to 99.6%. Mass spectrometry (m/z): 720.3178 (Calcd.: 720.3141). Theoretical element content (%) of C₅₃H₄₀N₂O: C, 88.30; H, 5.59; N, 3.89. Measured element content (%): C, 88.38; H, 5.65; N, 3.83.

### [Synthesis Example 27] Synthesis of Compound 330

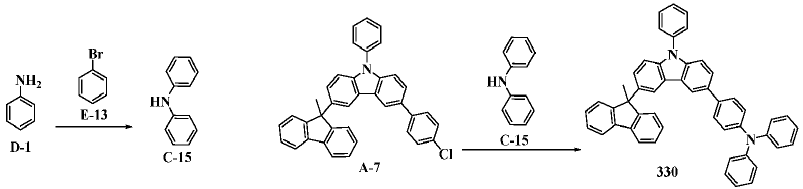

Compound 330 (15.34 g) was synthesized by the same method as Synthesis Example 8 except that E-1 was replaced with an equimolar amount of E-13 and Intermediate A-1 was replaced with an equimolar amount of Intermediate A-7. The solid purity detected through HPLC was greater than or equal to 99.8%. Mass spectrometry (m/z): 664.2911 (Calcd.: 664.2878). Theoretical element content (%) of C₅₀H₃₆N₂: C, 90.33; H, 5.46; N, 4.21. Measured element content (%): C, 90.40; H, 5.52; N, 4.17.

### [Synthesis Examples 28 to 43]

The following Compound 14, Compound 16, Compound 64, Compound 103, Compound 137, Compound 139, Compound 146, Compound 155, Compound 198, Compound 235, Compound 308, Compound 340, Compound 354, Compound 361, Compound 374, Compound 386, Compound 393, Compound 399, Compound 408 and Compound 415 were synthesized by the same method as Synthesis Example 8. The structural characterization of the final products obtained is shown in Table 1.

**Table 1**

| Example | Compound | Test Result |
|---|---|---|
| Example 28 | Compound 14 | The solid purity detected through HPLC was greater than or equal to 99.4%. Mass spectrometry (m/z): 726.2991 (Calcd.: 726.3035). |
| | | Theoretical element content (%) of C₅₅H₃₈N₂: C, 90.88; H, 5.27; N, 3.85. Measured element content (%): C, 90.85; H, 5.25; N, 3.88. |
| Example 29 | Compound 16 | The solid purity detected through HPLC was greater than or equal to 99.7%. Mass spectrometry (m/z): 731.3383 (Calcd.: 731.3349). |
| | | Theoretical element content (%) of C₅₅H₃₃D₅N₂: C, 90.25; H, 5.92; N, 3.83. Measured element content (%): C, 90.26; H, 5.89; N, 3.81. |
| Example 30 | Compound 64 | The solid purity detected through HPLC was greater than or equal to 99.6%. Mass spectrometry (m/z): 918.4005 (Calcd.: 918.3974). |
| | | Theoretical element content (%) of C₇₀H₅₀N₂: C, 91.47; H, 5.48; N, 3.05. Measured element content (%): C, 91.44; H, 5.50; N, 3.08. |
| Example 31 | Compound 103 | The solid purity detected through HPLC was greater than or equal to 99.8%. Mass spectrometry (m/z): 797.3463 (Calcd.: 797.3424). |
| | | Theoretical element content (%) of C₅₉H₃₁D₇N₂O: C, 90.33; H, 5.46; N, 4.21. Measured element content (%): C, 90.40; H, 5.52; N, 4.17. |
| Example 32 | Compound 137 | The solid purity detected through HPLC was greater than or equal to 99.5%. Mass spectrometry (m/z): 884.3104 (Calcd.: 884.3151). |
| | | Theoretical element content (%) of C₆₃H₄₀N₄O₂: C, 85.50; H, 4.56; N, 6.33. Measured element content (%): C, 85.46; H, 4.57; N, 6.30. |
| Example 33 | Compound 139 | The solid purity detected through HPLC was greater than or equal to 99.3%. Mass spectrometry (m/z): 726.2994 (Calcd.: 726.3035). |
| | | Theoretical element content (%) of C₅₅H₃₈N₂: C, 90.88; H, 5.27; N, 3.85. Measured element content (%): C, 90.91; H, 5.28; N, 3.83. |
| Example 34 | Compound 146 | The solid purity detected through HPLC was greater than or equal to 99.6%. Mass spectrometry (m/z): 928.3785 (Calcd.: 928.3817). |
| | | Theoretical element content (%) of C₇₁H₄₈N₂: C, 91.78; H, 5.21; N, 3.01. Measured element content (%): C, 91.77; H, 5.24; N, 3.03. |
| Example 35 | Compound 155 | The solid purity detected through HPLC was greater than or equal to 99.1%. Mass spectrometry (m/z): 1040.4176 (Calcd.: 1040.4130). |
| | | Theoretical element content (%) of C₈₀H₅₂N₂: C, 92.28; H, 5.03; N, 2.69. Measured element content (%): C, 92.30; H, 5.01; N, 2.72. |
| Example 36 | Compound 198 | The solid purity detected through HPLC was greater than or equal to 99.9%. Mass spectrometry (m/z): 802.3385 (Calcd.: 802.3348). |
| | | Theoretical element content (%) of C₆₁H₄₂N₂: C, 91.24; H, 5.27; N, 3.49. Measured element content (%): C, 91.22; H, 5.24; N, 3.52. |
| Example 37 | Compound 235 | The solid purity detected through HPLC was greater than or equal to 99.4%. Mass spectrometry (m/z): 842.3607 (Calcd.: 842.3661). |
| | | Theoretical element content (%) of C₆₄H₄₆N₂: C, 91.18; H, 5.50; N, 3.32. Measured element content (%): C, 91.16; H, 5.52; N, 3.35. |
| Example 38 | Compound 308 | The solid purity detected through HPLC was greater than or equal to 99.5%. Mass spectrometry (m/z): 638.2749 (Calcd.: 638.2722). |
| | | Theoretical element content (%) of C₄₈H₃₄N₂: C, 90.25; H, 5.36; N, 4.39. Measured element content (%): C, 90.22; H, 5.37; N, 4.41. |
| Example 39 | Compound 340 | The solid purity detected through HPLC was greater than or equal to 99.7%. Mass spectrometry (m/z): 887.4272 (Calcd.: 887.4226). |
| | | Theoretical element content (%) of C₆₇H₃₇D₉N₂: C, 90.61; H, 6.24; N, 3.15. Measured element content (%): C, 90.64; H, 6.22; N, 3.16. |
| Example 40 | Compound 354 | The solid purity detected through HPLC was greater than or equal to 99.2%. Mass spectrometry (m/z): 1042.4315 (Calcd.: 1042.4287). |
| | | Theoretical element content (%) of C₈₀H₅₄N₂: C, 92.10; H, 5.22; N, 2.69. Measured element content (%): C, 92.08; H, 5.25; N, 2.71. |
| Example 41 | Compound 361 | The solid purity detected through HPLC was greater than or equal to 99.8%. Mass spectrometry (m/z): 968.4095 (Calcd.: 968.4130). |
| | | Theoretical element content (%) of C₇₄H₅₂N₂: C, 91.70; H, 5.41; N, 2.89. Measured element content (%): C, 91.71; H, 5.39; N, 2.91. |
| Example 42 | Compound 374 | The solid purity detected through HPLC was greater than or equal to 99.4%. Mass spectrometry (m/z): 942.3928 (Calcd.: 942.3974). |
| | | Theoretical element content (%) of C₇₂H₅₀N₂: C, 91.69; H, 5.34; N, 2.97. Measured element content (%): C, 91.71; H, 5.31; N, 2.95. |
| Example 43 | Compound 386 | The solid purity detected through HPLC was greater than or equal to 99.7%. Mass spectrometry (m/z): 1008.4062 (Calcd.: 1008.4080). |
| | | Theoretical element content (%) of C₇₆H5₂N₂O: C, 90.45; H, 5.19; N, 2.78. Measured element content (%): C, 90.43; H, 5.20; N, 2.76. |
| Example 44 | Compound 393 | The solid purity detected through HPLC was greater than or equal to 99.8%. Mass spectrometry (m/z): 971.4247 (Calcd.: 971.4288). |
| | | Theoretical element content (%) of C₇₄H₄₅D₅N₂: C, 91.42; H, 5.70; N, 2.88. Measured element content (%): C, 91.44; H, 5.68; N, 2.87. |
| Example 45 | Compound 399 | The solid purity detected through HPLC was greater than or equal to 99.6%. Mass spectrometry (m/z): 922.4266 (Calcd.: 922.4225). |
| | | Theoretical element content (%) of C₇₀H₄₆D₄N₂: C, 91.07; H, 5.90; N, 3.03. Measured element content (%): C, 91.05; H, 5.87; N, 3.01. |
| Example 46 | Compound 408 | The solid purity detected through HPLC was greater than or equal to 99.3%. Mass spectrometry (m/z): 882.3882 (Calcd.: 882.3912). |
| | | Theoretical element content (%) of C₆₇H₄₂D₄N₂: C, 91.12; H, 5.71; N, 3.17. Measured element content (%): C, 91.10; H, 5.69; N, 3.20. |
| Example 47 | Compound 415 | The solid purity detected through HPLC was greater than or equal to 99.8%. Mass spectrometry (m/z): 902.3628 (Calcd.: 902.3661). |
| | | Theoretical element content (%) of C₆₉H₄₆N₂: C, 91.76; H, 5.13; N, 3.10. Measured element content (%): C, 91.73; H, 5.11; N, 3.12. |

### [Example 48] Test of glass transition temperature

Test samples: Compound 1, Compound 3, Compound 14, Compound 16, Compound 17, Compound 28, Compound 39, Compound 48, Compound 59, Compound 64, Compound 72, Compound 78, Compound 90, Compound 103, Compound 115, Compound 137 , Compound 139, Compound 141, Compound 146, Compound 153, Compound 155, Compound 161, Compound 198, Compound 170, Compound 219, Compound 235, Compound 240, Compound 301, Compound 308, Compound 312, Compound 330, Compound 340, Compound 354, Compound 361, Compound 374, Compound 386, Compound 393, Compound 399, Compound 408, Compound 415 and Comparative Compounds 1 and 2 were tested individually, and the mass of each sample was 5 mg.

Test instrument: DSC 25 Differential Scanning Calorimeter (TA Co., USA).

Test conditions: a test atmosphere of nitrogen with a flowrate of 50 mL/min; a temperature ramping rate of 10 °C/min, and a temperature range of 50-350 °C. The test results of the glass transition temperature (Tg) are shown in Table 2.

**Table 2 Test results of the glass transition temperature**

| Compound | Tg (°C) | Compound | Tg (°C) |
|---|---|---|---|
| Compound 1 | 158.9 | Compound 161 | 165.0 |
| Compound 3 | 159.1 | Compound 198 | 159.0 |
| Compound 14 | 158.5 | Compound 170 | 168.9 |
| Compound 16 | 158.6 | Compound 219 | 159.5 |
| Compound 17 | 159.4 | Compound 235 | 163.8 |
| Compound 28 | 161.0 | Compound 240 | 164.2 |
| Compound 39 | 163.0 | Compound 301 | 156.7 |
| Compound 48 | 165.7 | Compound 308 | 156.5 |
| Compound 59 | 168.0 | Compound 312 | 157.0 |
| Compound 64 | 166.0 | Compound 330 | 156.2 |
| Compound 72 | 168.3 | Compound 340 | 160.0 |
| Compound 78 | 162.7 | Compound 354 | 167.2 |
| Compound 90 | 169.7 | Compound 361 | 167.0 |
| Compound 103 | 163.4 | Compound 374 | 168.5 |
| Compound 115 | 169.4 | Compound 386 | 171.0 |
| Compound 137 | 163.5 | Compound 393 | 165.1 |
| Compound 139 | 158.3 | Compound 399 | 166.2 |
| Compound 141 | 161.3 | Compound 408 | 159.8 |
| Compound 146 | 161.5 | Compound 415 | 162.0 |
| Compound 153 | 165.9 | Comparative Compound 1 | 150.2 |
| Compound 155 | 170.0 | | |

As can be known from the results in Table 1, the triarylamine derivative provided by the present disclosure has a relatively high glass transition temperature and good thermal stability and is not easy to crystallize in a thin-film state. The application of the triarylamine derivative to the organic electroluminescent device can improve the stability of the device and extend the service life of the device.

### [Example 49] Test of film formability of a material

Compound 17, Compound 115, Compound 146, Compound 153 and Compound 354 of the present disclosure and Comparative Compound 1 were vapor-deposited on ITO glass substrates respectively to prepare samples each having a thickness of 10 nm. The morphology of thin films formed by Compound 17, Compound 115, Compound 146, Compound 153 and Compound 354 of the present disclosure and Comparative Compound 1 were characterized by an atomic force microscope. The morphology of each sample thin film is shown in FIG. 1, and the obtained surface roughness values are shown in Table 3.

**Table 3 Surface roughness (root mean square (RMS)) of compounds**

| Sample | Surface Roughness (RMS) |
|---|---|
| Compound 17 | 0.491 |
| Compound 115 | 0.535 |
| Compound 146 | 0.344 |
| Compound 153 | 0.476 |
| Compound 354 | 0.328 |
| Comparative Compound 1 | 0.872 |

As can be known from the results in Table 3, the compounds of the present disclosure have stericity and are not easy to crystallize and can all form continuous and uniform thin films. Compared with Comparative Compound 1, the compounds of the present disclosure have significantly lower roughness (RMS) than Comparative Compound 1, which indicates that the compound of the present disclosure can form a more uniform, more stable and flatter thin film through evaporation and has better film formability.

### [Device Example 1]

Firstly, an ITO glass substrate was washed twice in distilled water, washed with ultrasonic waves for 30 min, repeatedly washed twice in distilled water, and washed with ultrasonic waves for 10 min. After washed in distilled water and washed with ultrasonic waves in isopropanol, acetone and methanol solvents in sequence, the ITO glass substrate was dried on a hot plate heated to 120 °C. The dried substrate was transferred to a plasma cleaner. After washed for 5 min, the substrate was transferred to an evaporator.

Then, HT-1 (60 nm) and HATCN (5 nm) were deposited on the cleaned ITO substrate as a hole injection layer. Compound 1 of the present disclosure was deposited on the hole injection layer as a hole transport layer with a thickness of 80 nm. On the hole transport layer, H-1 was deposited through vacuum evaporation as a host material and D-1 was deposited as a doping material, where H-1 and D-1 were doped at a ratio of 96:4 to form a light-emitting layer with a thickness of 25 nm. BAlq₃ was deposited on the light-emitting layer as a hole blocking layer with a thickness of 10 nm. ET-1 and Liq (with a doping ratio of 1:1) were deposited on the hole blocking layer as an electron transport layer with a thickness of 30 nm. Lithium fluoride was deposited on the electron transport layer as an electron injection layer with a thickness of 1 nm. Then, Al was deposited on the electron injection layer as a cathode with a thickness of 150 nm, thereby preparing an organic electroluminescent device.

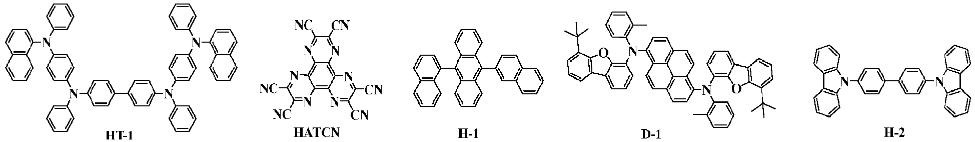

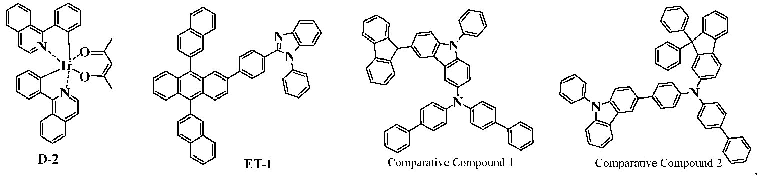

### [Device Examples 2 to 40]

Organic electroluminescent devices were prepared by the same method as Device Example 1 except that Compound 1 in Device Example 1 was replaced with Compound 3, Compound 14, Compound 16, Compound 17, Compound 28, Compound 39, Compound 48, Compound 59, Compound 64, Compound 72, Compound 78, Compound 90, Compound 103, Compound 115, Compound 137, Compound 139, Compound 141, Compound 146, Compound 153, Compound 155, Compound 161, Compound 198, Compound 170, Compound 219, Compound 235, Compound 240, Compound 301, Compound 308, Compound 312, Compound 330, Compound 340, Compound 354, Compound 361, Compound 374, Compound 386, Compound 393, Compound 399, Compound 408 and Compound 415 of the present disclosure respectively as the hole transport layer.

### [Comparative Device Example 1]

An organic electroluminescent device was prepared by the same method as Device Example 1 except that Compound 1 in Device Example 1 was replaced with Comparative Compound 1 as the hole transport layer.

The driving voltage and the luminescence efficiency of each organic electroluminescent device were tested using a combined IVL test system composed of test software, a computer, a K2400 digital source meter manufactured by Keithley, USA and a PR788 spectral scanning photometer manufactured by Photo Research, USA. The lifetime was tested using an M6000 OLED lifetime test system from McScience. The test was conducted in an atmospheric environment and at room temperature. The test results of the luminescence characteristics of the organic electroluminescent devices obtained in Device Examples 1 to 40 of the present disclosure and Comparative Examples 1 and 2 are shown in Table 4.

**Table 4 Luminescence characteristics of the organic electroluminescent devices**

| Example | Hole Transport Layer | Driving Voltage (@10 mA/cm²) | Luminescence Efficiency (@10 mA/cm²) | Lifetime (T95) |
|---|---|---|---|---|
| Device Example 1 | Compound 1 | 4.2 | 7.80 | 161 |
| Device Example 2 | Compound 3 | 4.2 | 7.52 | 155 |
| Device Example 3 | Compound 14 | 4.2 | 7.61 | 157 |
| Device Example 4 | Compound 16 | 4.2 | 7.58 | 156 |
| Device Example 5 | Compound 17 | 4.2 | 7.67 | 158 |
| Device Example 6 | Compound 28 | 3.9 | 9.32 | 186 |
| Device Example 7 | Compound 39 | 4.0 | 8.53 | 173 |
| Device Example 8 | Compound 48 | 3.9 | 9.02 | 181 |
| Device Example 9 | Compound 59 | 4.1 | 8.20 | 169 |
| Device Example 10 | Compound 64 | 4.0 | 8.98 | 180 |
| Device Example 11 | Compound 72 | 4.2 | 7.76 | 160 |
| Device Example 12 | Compound 78 | 4.3 | 7.39 | 151 |
| Device Example 13 | Compound 90 | 4.1 | 7.91 | 162 |
| Device Example 14 | Compound 103 | 4.1 | 8.18 | 167 |
| Device Example 15 | Compound 115 | 4.0 | 8.84 | 178 |
| Device Example 16 | Compound 137 | 4.3 | 7.33 | 151 |
| Device Example 17 | Compound 139 | 4.2 | 7.68 | 159 |
| Device Example 18 | Compound 141 | 4.0 | 8.67 | 177 |
| Device Example 19 | Compound 146 | 3.9 | 9.35 | 187 |
| Device Example 20 | Compound 153 | 4.0 | 8.35 | 171 |
| Device Example 21 | Compound 155 | 4.1 | 7.94 | 163 |
| Device Example 22 | Compound 161 | 4.1 | 8.03 | 164 |
| Device Example 23 | Compound 198 | 4.2 | 7.49 | 154 |
| Device Example 24 | Compound 170 | 3.9 | 9.18 | 185 |
| Device Example 25 | Compound 219 | 4.3 | 7.21 | 149 |
| Device Example 26 | Compound 235 | 4.3 | 7.15 | 148 |
| Device Example 27 | Compound 240 | 4.3 | 7.06 | 146 |
| Device Example 28 | Compound 301 | 4.2 | 6.71 | 140 |
| Device Example 29 | Compound 308 | 4.2 | 6.80 | 142 |
| Device Example 30 | Compound 312 | 4.3 | 6.98 | 143 |
| Device Example 31 | Compound 330 | 4.3 | 6.76 | 138 |
| Device Example 32 | Compound 340 | 4.0 | 8.46 | 172 |
| Device Example 33 | Compound 354 | 4.1 | 8.14 | 166 |
| Device Example 34 | Compound 361 | 4.0 | 8.60 | 175 |
| Device Example 35 | Compound 374 | 3.9 | 9.23 | 185 |
| Device Example 36 | Compound 386 | 3.9 | 9.12 | 183 |
| Device Example 37 | Compound 393 | 4.1 | 8.05 | 168 |
| Device Example 38 | Compound 399 | 4.1 | 8.32 | 170 |
| Device Example 39 | Compound 408 | 4.0 | 8.48 | 173 |
| Device Example 40 | Compound 415 | 3.9 | 9.16 | 184 |
| Comparative Device Example 1 | Comparative Compound 1 | 4.4 | 6.54 | 130 |

As can be known from the test results in Table 4, with respect to Comparative Example 1, the use of the triarylamine derivative provided by the present disclosure as the hole transport layer of the organic electroluminescent device can reduce the driving voltage of the organic electroluminescent device, significantly improve the luminescence efficiency, and extend the service life of the device. In particular, when R₁ in the present disclosure is an aryl group, the triarylamine derivative of the present disclosure has a particularly significant effect of improving the performance of the organic electroluminescent device.

### [Device Example 41]

Firstly, an ITO glass substrate was washed twice in distilled water, washed with ultrasonic waves for 30 min, repeatedly washed twice in distilled water, and washed with ultrasonic waves for 10 min. After washed in distilled water and washed with ultrasonic waves in isopropanol, acetone and methanol solvents in sequence, the ITO glass substrate was dried on a hot plate heated to 120 °C. The dried substrate was transferred to a plasma cleaner. After washed for 5 min, the substrate was transferred to an evaporator.

Then, HT-1 (60 nm) and HATCN (5 nm) were deposited on the cleaned ITO substrate as a hole injection layer. NPB was deposited on the hole injection layer as a first hole transport layer with a thickness of 60 nm. Compound 1 of the present disclosure was deposited on the first hole transport layer as a second hole transport layer with a thickness of 20 nm. On the second hole transport layer, H-1 was deposited through vacuum evaporation as a host material and D-1 was deposited as a doping material, where H-1 and D-1 were doped at a ratio of 96:4 to form a light-emitting layer with a thickness of 25 nm. BAlq₃ was deposited on the light-emitting layer as a hole blocking layer with a thickness of 10 nm. ET-1 and Liq (with a doping ratio of 1:1) were deposited on the hole blocking layer as an electron transport layer with a thickness of 30 nm. Lithium fluoride was deposited on the electron transport layer as an electron injection layer with a thickness of 1 nm. Then, Al was deposited on the electron injection layer as a cathode with a thickness of 150 nm, thereby preparing an organic electroluminescent device.

### [Device Examples 42 to 50]

Organic electroluminescent devices were prepared by the same method as Device Example 41 except that Compound 1 in Device Example 41 was replaced with Compound 3, Compound 17, Compound 28, Compound 39, Compound 48, Compound 90, Compound 115, Compound 153 and Compound 161 of the present disclosure respectively as the second hole transport layer.

### [Device Examples 51 to 55]

Organic electroluminescent devices were prepared by the same method as Device Example 41 except that NPB in Device Examples 41 to 50 was replaced with Compound 14, Compound 59, Compound 146, Compound 361 and Compound 399 of the present disclosure as the first hole transport layer. The test results are shown in Table 5.

**Table 5 Luminescence characteristics of the organic electroluminescent devices**

| Example | First Hole Transport Layer | Second Hole Transport Layer | Driving Voltage (@ 1 0mA/cm²) | Luminescence Efficiency (@ 1 0mA/cm²) | Lifetime (T95) |
|---|---|---|---|---|---|
| Device Example 41 | NPB | Compound 1 | 4.1 | 8.99 | 196 |
| Device Example 42 | NPB | Compound 3 | 3.9 | 8.73 | 192 |
| Device Example 43 | NPB | Compound 17 | 4.0 | 8.86 | 194 |
| Device Example 44 | NPB | Compound 28 | 3.7 | 9.35 | 210 |
| Device Example 45 | NPB | Compound 39 | 3.9 | 9.2 | 208 |
| Device Example 46 | NPB | Compound 48 | 3.8 | 9.26 | 206 |
| Device Example 47 | NPB | Compound 90 | 4.0 | 9.03 | 198 |
| Device Example 48 | NPB | Compound 115 | 3.9 | 9.23 | 205 |
| Device Example 49 | NPB | Compound 153 | 3.9 | 9.14 | 202 |
| Device Example 50 | NPB | Compound 161 | 4.0 | 9.05 | 199 |
| Device Example 51 | Compound 14 | Compound 1 | 3.9 | 9.56 | 233 |
| Device Example 52 | Compound 59 | Compound 28 | 3.9 | 9.62 | 235 |
| Device Example 53 | Compound 146 | Compound 48 | 3.7 | 9.64 | 240 |
| Device Example 54 | Compound 361 | Compound 115 | 3.8 | 9.58 | 229 |
| Device Example 55 | Compound 399 | Compound 153 | 3.8 | 9.60 | 234 |

As can be known from the test results in Table 5, when the triarylamine derivative provided by the present disclosure is used as the second hole transport layer of the organic electroluminescent device and matched with another hole transport layer, the performance of the organic electroluminescent device can be improved; when the triarylamine derivatives provided by the present disclosure are used in combination as the first hole transport layer and the second hole transport layer, the driving voltage of the organic electroluminescent device can be further reduced, the luminescence efficiency can be significantly improved, and the service life of the device can be extended.

### [Device Example 56] Preparation of organic electroluminescent device 56

On an ITO substrate formed with a reflective layer including Ag, HT-1 (60 nm) and HATCN (5 nm) were deposited as a hole injection layer. NPB was deposited on the hole injection layer as a hole transport layer with a thickness of 120 nm. On the hole transport layer, H-2 was deposited through vacuum evaporation as a host material and D-2 was deposited as a doping material, where H-2 and D-2 were doped at a ratio of 95:5 to form a light-emitting layer with a thickness of 30 nm. BAlq₃ was deposited on the light-emitting layer as a hole blocking layer with a thickness of 10 nm. ET-1 and Liq (with a doping ratio of 1:1) were deposited on the hole blocking layer as an electron transport layer with a thickness of 30 nm. Lithium fluoride was deposited on the electron transport layer as an electron injection layer with a thickness of 1 nm. Then, Mg/Ag were deposited on the electron injection layer as a cathode with a thickness of 15 nm. Compound 1 of the present disclosure was deposited on the cathode as a capping layer with a thickness of 60 nm, thereby preparing an organic electroluminescent device.

### [Device Examples 57 to 60]

Organic electroluminescent devices were prepared by the same method as Device Example 56 except that Compound 1 in Device Example 56 was replaced with Compound 39, Compound 78, Compound 115 and Compound 153 of the present disclosure as the capping layer.

### [Comparative Example 2]

An organic electroluminescent device was prepared by the same method as Device Example 56 except that Compound 1 in Device Example 56 was replaced with Comparative Compound 2 as the capping layer. The test results are shown in Table 6.

**Table 6 Luminescence characteristics of the organic electroluminescent devices**

| Example | Capping Layer | Luminescence Efficiency (@10mA/cm²) | Lifetime (T97) |
|---|---|---|---|
| Device Example 56 | Compound 1 | 20.8 | 197 |
| Device Example 57 | Compound 39 | 21.9 | 209 |
| Device Example 58 | Compound 78 | 23.7 | 215 |
| Device Example 59 | Compound 115 | 20.6 | 196 |
| Device Example 60 | Compound 153 | 20.1 | 185 |
| Comparative Device Example 2 | Comparative Compound 2 | 13.5 | 160 |

As can be known from the test results in Table 6, the use of the triarylamine derivative provided by the present disclosure as the capping layer of the organic electroluminescent device can not only effectively improve light extraction efficiency and thus improve the luminescence efficiency of the organic electroluminescent device but also prevent the internal structure of the device from being eroded by moisture and oxygen in the air and extend the service life of the device.

It is to be noted that though the present disclosure has been specifically described through particular embodiments, those skilled in the art can make various modifications in form or detail without departing from the principle of the present disclosure and such modifications also fall within the scope of the present disclosure.

## Claims

1. A triarylamine derivative, wherein the triarylamine derivative has a structure represented by Formula I: 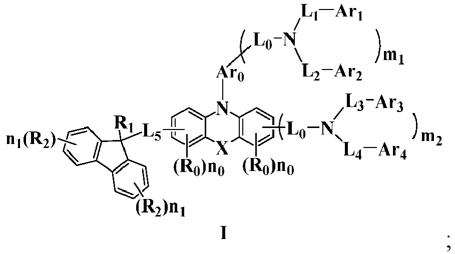 wherein in Formula I, X is selected from a single bond or C(R₃R₄); wherein R₃ and R₄ are independently selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl, or R₃ and R₄ are joined to form a substituted or unsubstituted ring;
Ar₁ and Ar₂ are the same as or different from each other and independently selected from any one of substituted or unsubstituted C6 to C30 aryl or substituted or unsubstituted C2 to C30 heteroaryl;
Ar₃ and Ar₄ are the same as or different from each other and independently selected from any one of substituted or unsubstituted C6 to C30 aryl or the following groups: 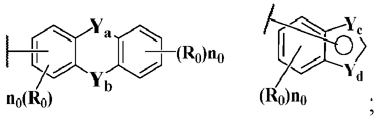
wherein, Ya is selected from O or S, Yb is selected from a single bond or C(RₓR_{y}),
Yc is selected from any one of O, S or N(R_{z}), and Yd is selected from O, S or N(R_{z});
Rₓ and R_{y} are independently selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl, or R₃ and R₄ are joined to form a substituted or unsubstituted ring;
R_{z} is selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
L₀ to L₅ are independently selected from any one of a single bond, substituted or unsubstituted C6 to C30 arylene or substituted or unsubstituted C2 to C30 heteroarylene;
m₁ is selected from 0 or 1, m₂ is selected from 0 or 1, and m₁ and m₂ are not simultaneously selected from 0;
when m₁ is 0, Ar₀ is selected from any one of substituted or unsubstituted C6 to C30 aryl; when m₁ is 1, Ar₀ is selected from a single bond;
R₁ is selected from any one of substituted or unsubstituted C1 to C12 alkyl, substituted or unsubstituted C3 to C12 cycloalkyl or substituted or unsubstituted C6 to C30 aryl;
R₀ and R₂ are independently selected from any one of hydrogen, deuterium, cyano, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
n₀ is independently selected from an integer from 0 to 4, and n₁ is independently selected from an integer from 0 to 4; when n₀ is greater than 1, two or more R₀ are the same as or different from each other;
when n₁ is greater than 1, two or more R₂ are the same as or different from each other, or two adjacent R₂ are joined to form a benzene ring or a naphthalene ring;
a substituent in the preceding "substituted or unsubstituted" group is selected from one or more of: deuterium, cyano, a halogen atom, amino, nitro, substituted or unsubstituted C1 to C12 alkyl, substituted or unsubstituted C3 to C12 cycloalkyl, substituted or unsubstituted C1 to C12 alkoxy, substituted or unsubstituted C6 to C30 aryl and substituted or unsubstituted C2 to C30 heteroaryl; in the presence of a plurality of substituents, the plurality of substituents are the same as or different from each other.

2. The triarylamine derivative of claim 1, wherein the compound of Formula I is selected from any one of structures represented by Formulas I-A to I-C:

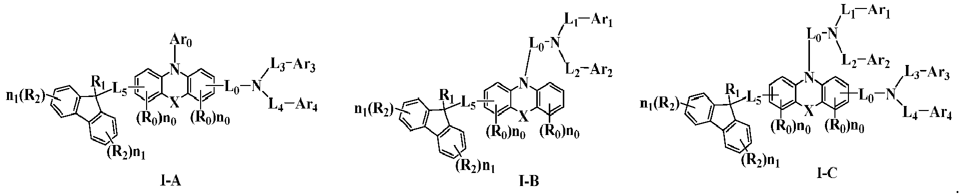

3. The triarylamine derivative of claim 1, wherein the compound of Formula I is selected from any one of structures represented by Formulas I-1 to 1-6:

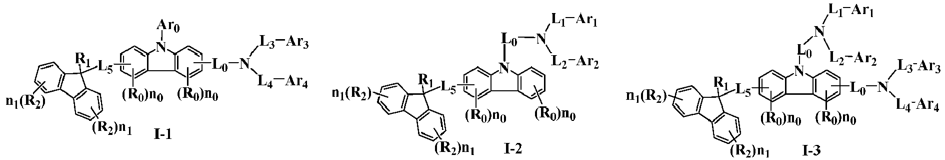

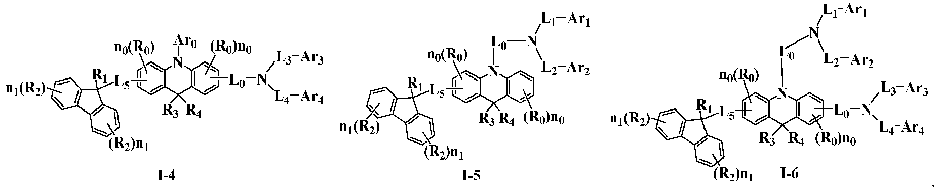

4. The triarylamine derivative of claim 1, wherein Ar₁ to Ar₄ are the same as or different from each other and independently selected from any one of the following groups: 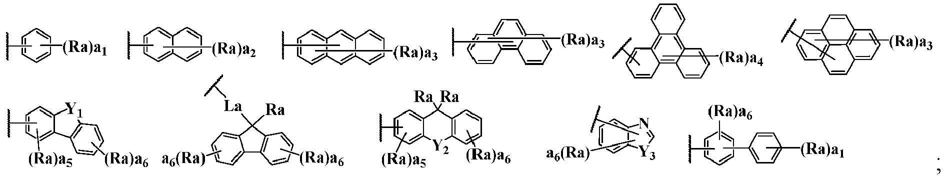 wherein Ra is selected from any one of hydrogen, deuterium, substituted or unsubstituted C1 to C12 alkyl, substituted or unsubstituted C3 to C12 cycloalkyl, substituted or unsubstituted C6 to C30 aryl or substituted or unsubstituted C2 to C30 heteroaryl;
a₁ is independently selected from an integer from 0 to 5, a₂ is selected from an integer from 0 to 7, a₃ is independently selected from an integer from 0 to 9, a₄ is selected from an integer from 0 to 11, as is independently selected from an integer from 0 to 3, and a₆ is independently selected from an integer from 0 to 4;
when a₁, a₂, a₃, a₄, as or a₆ is greater than 1, two or more Ra are the same as or different from each other, or two adjacent Ra are joined to form a substituted or unsubstituted ring;
Y₁ is selected from any one of O, S or C(RbRc), Y₂ is selected from any one of O or S, and Y₃ is selected from any one of O, S or N(Re);
Rb and Rc are independently selected from any one of hydrogen, C1 to C12 alkyl, C3 to C12 cycloalkyl, C6 to C30 aryl or C2 to C30 heteroaryl, or Rb and Rc are joined to form a substituted or unsubstituted ring; Re is selected from any one of hydrogen, C1 to C12 alkyl, C3 to C12 cycloalkyl, C6 to C30 aryl or C2 to C30 heteroaryl; and
La is selected from any one of a single bond or substituted or unsubstituted C6 to C18 arylene.

5. The triarylamine derivative of claim 1, wherein Ar₁ to Ar₄ are the same as or different from each other and independently selected from any one of the following groups:

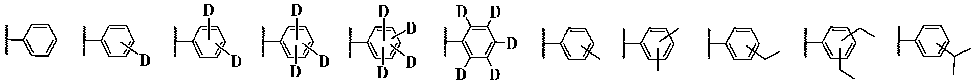

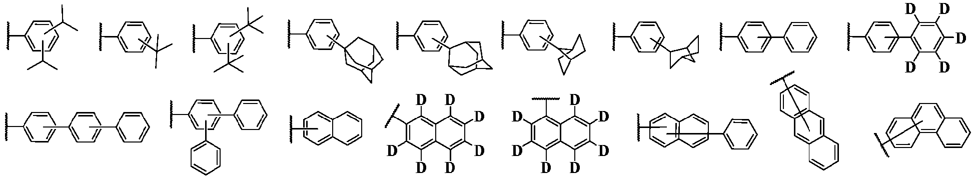

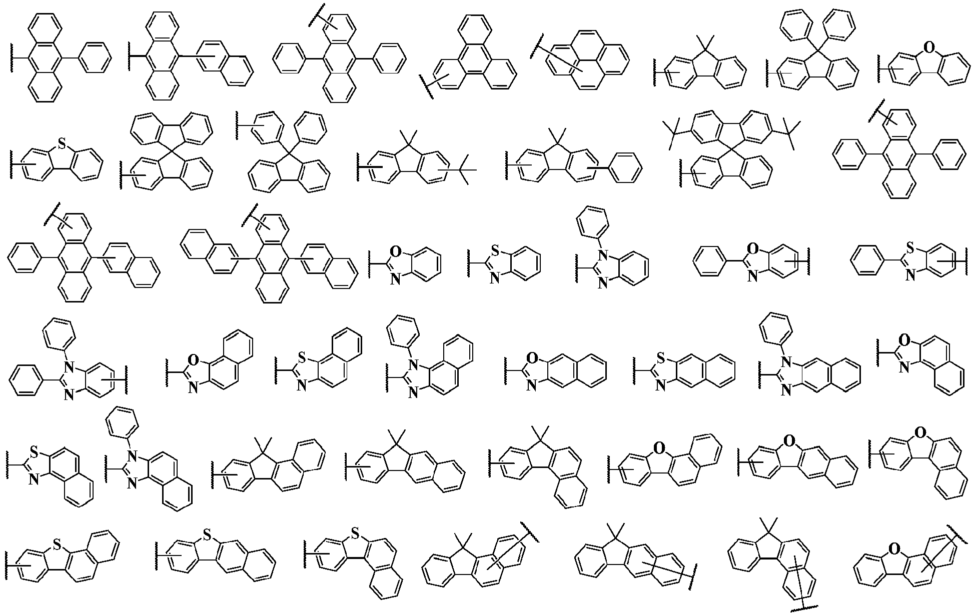

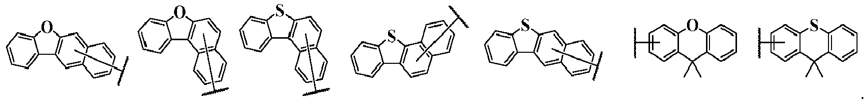

6. The triarylamine derivative of claim 1, wherein Lo to L₅ are independently selected from any one of a single bond or the following groups:

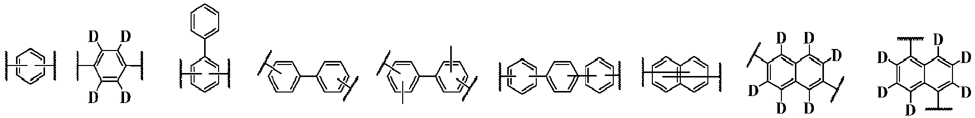

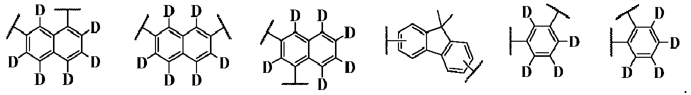

7. The triarylamine derivative of claim 1, wherein the compound of Formula I is selected from any one of the following structures:

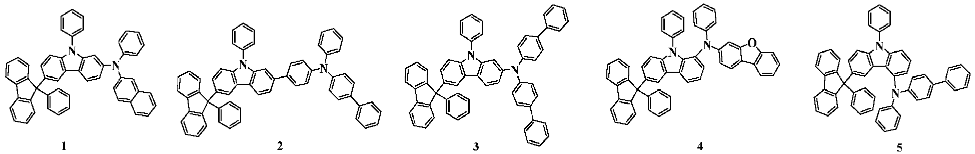

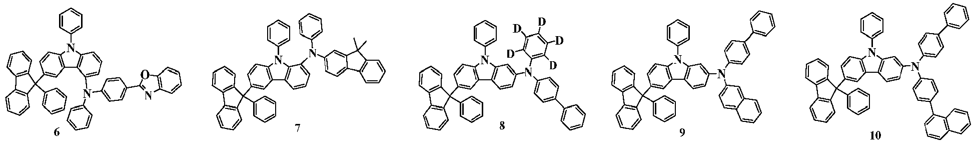

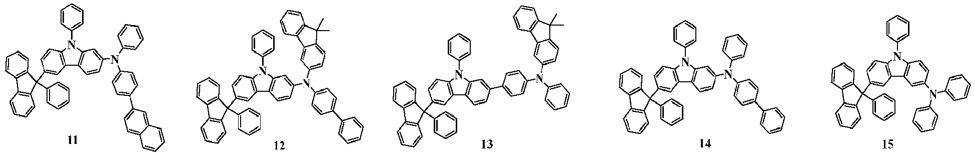

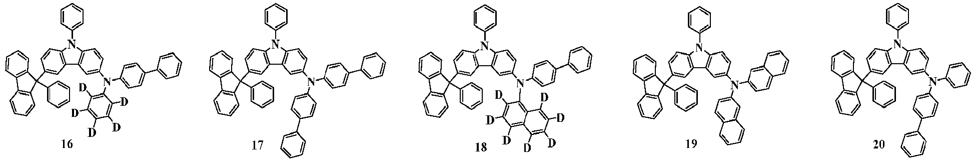

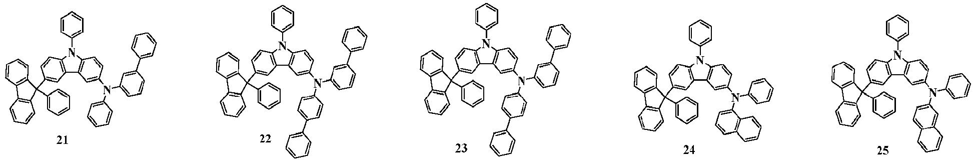

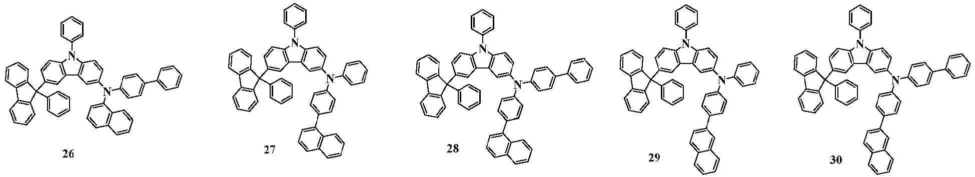

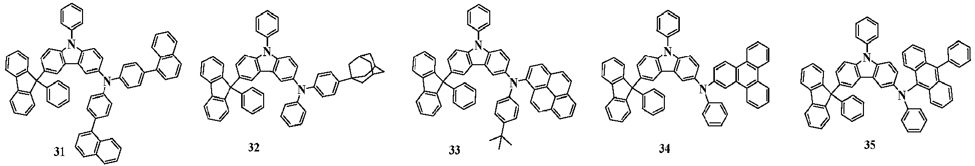

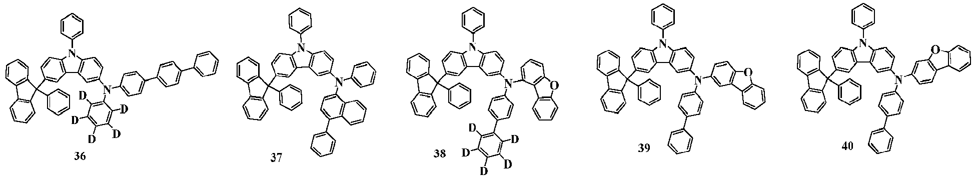

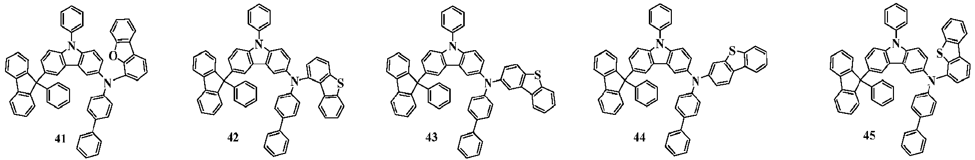

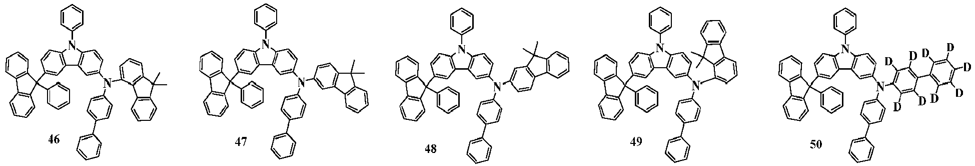

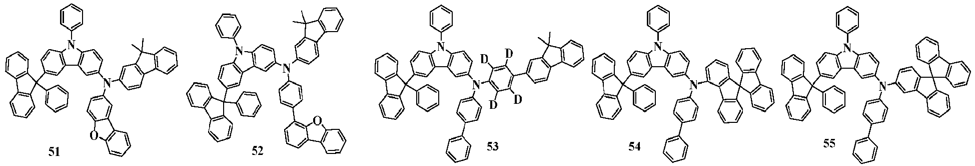

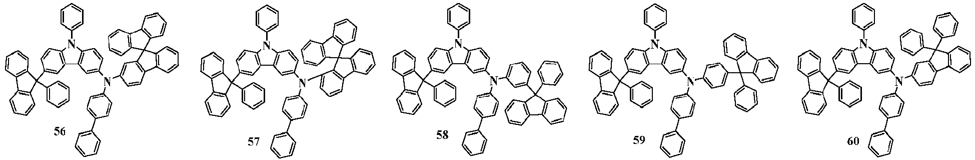

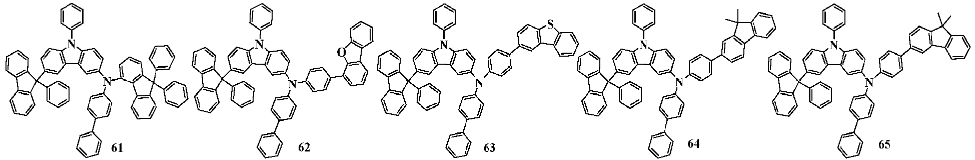

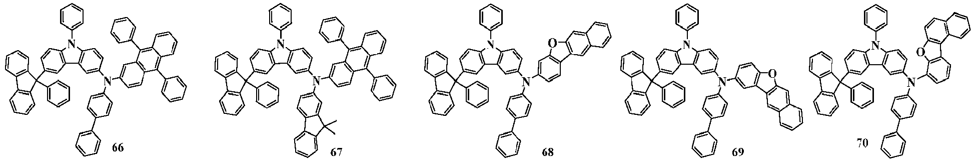

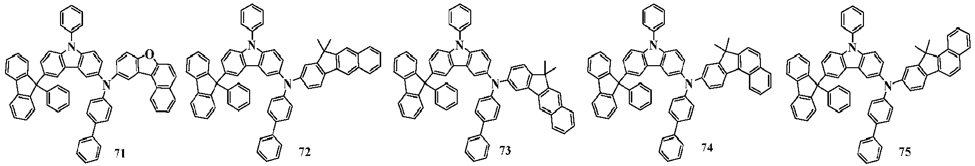

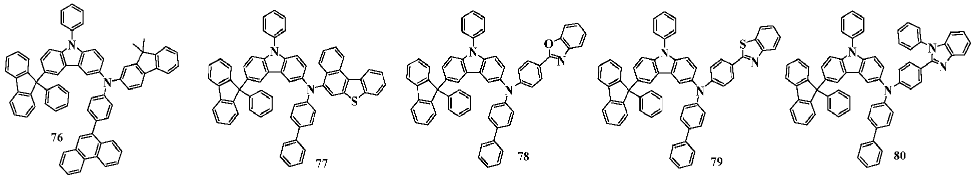

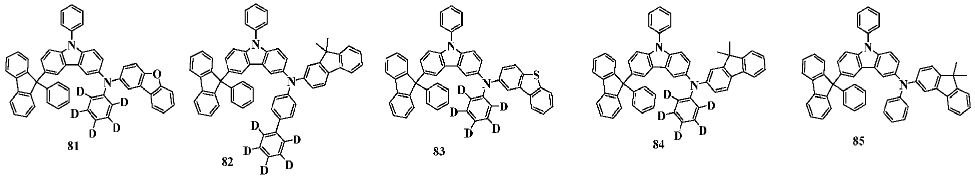

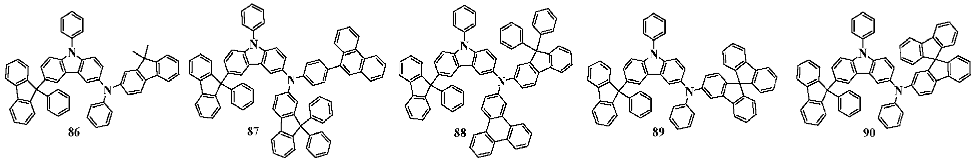

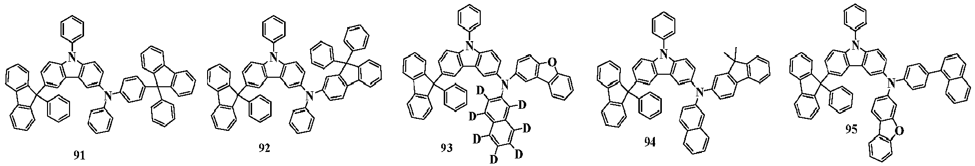

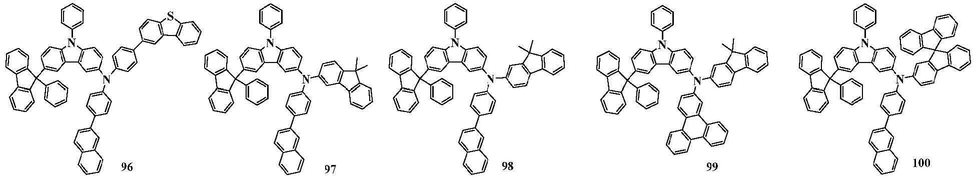

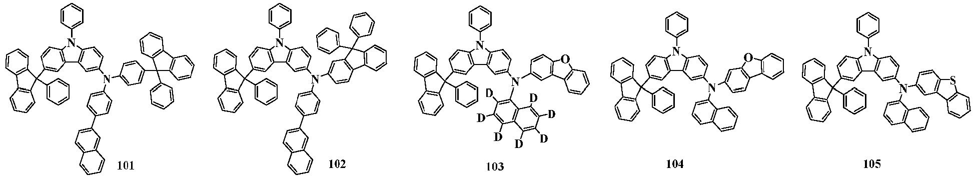

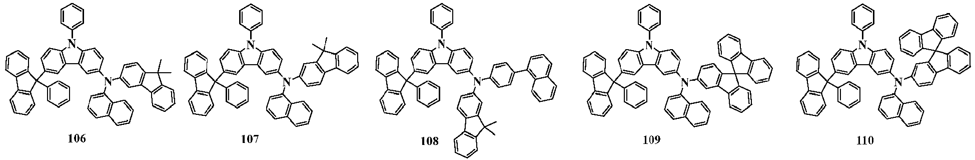

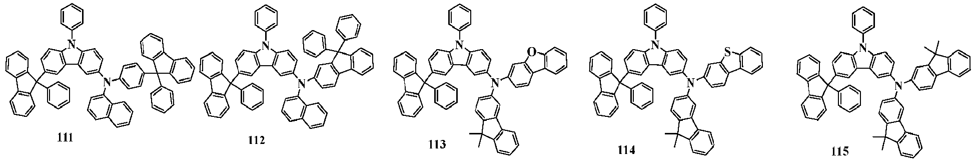

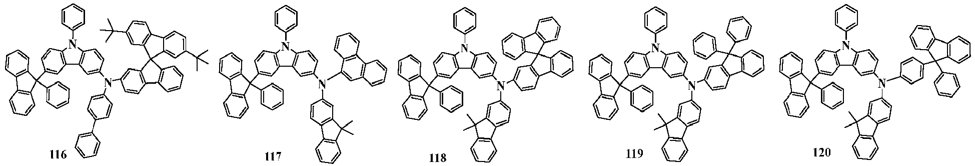

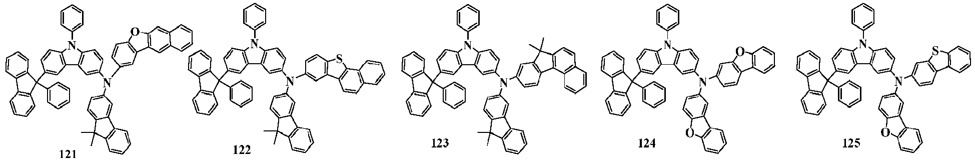

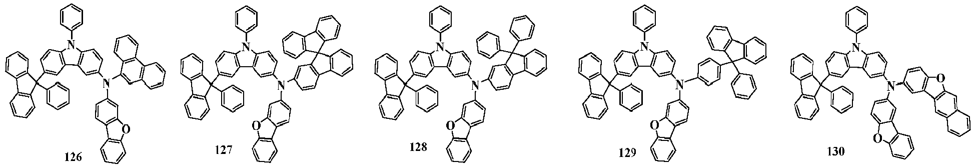

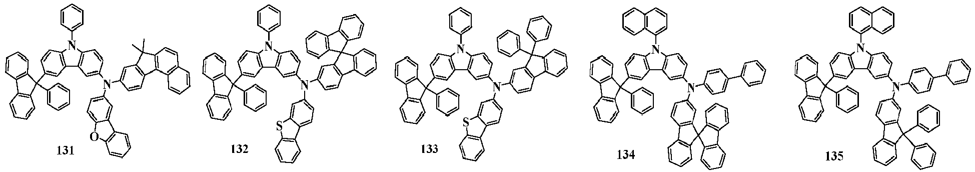

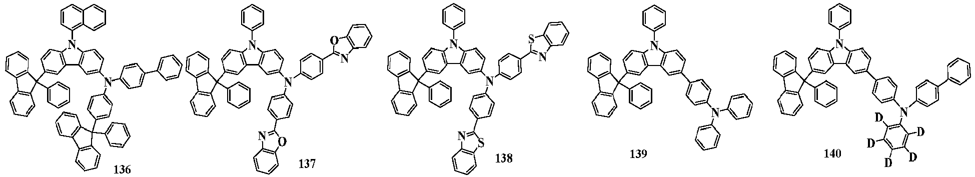

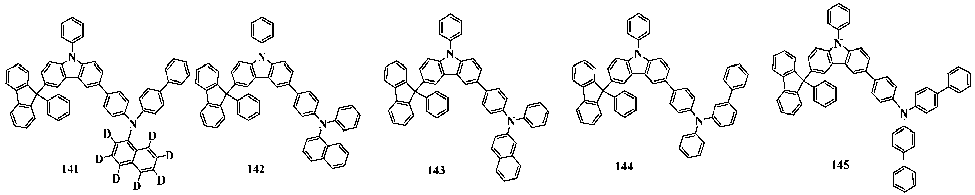

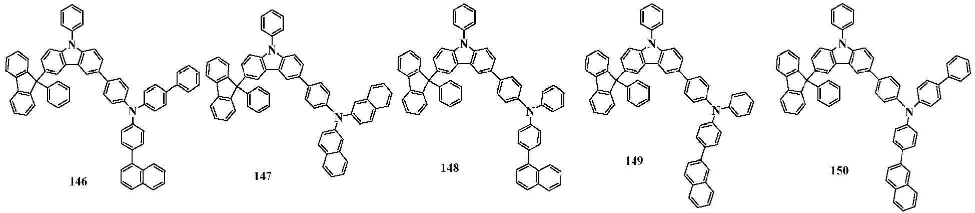

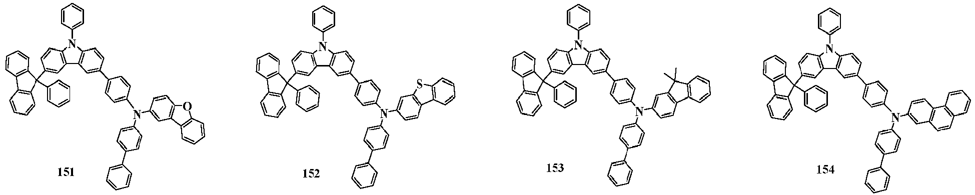

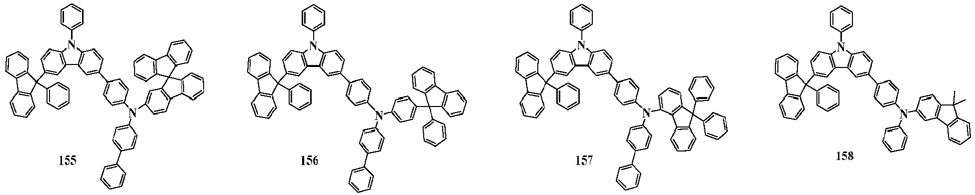

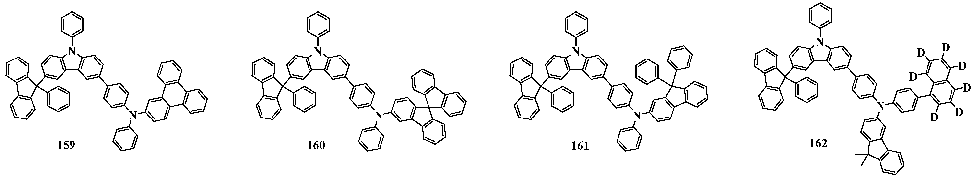

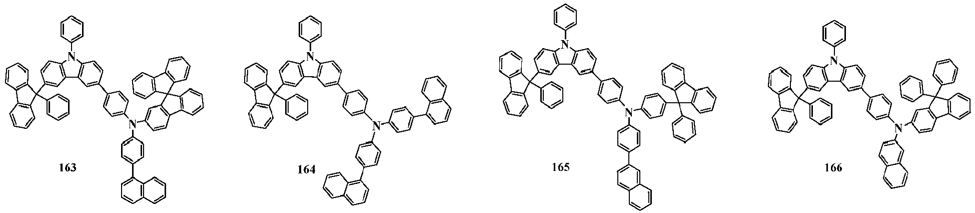

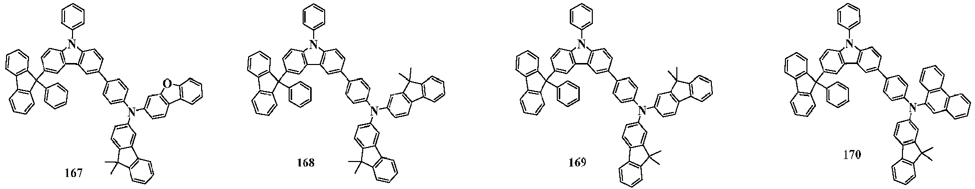

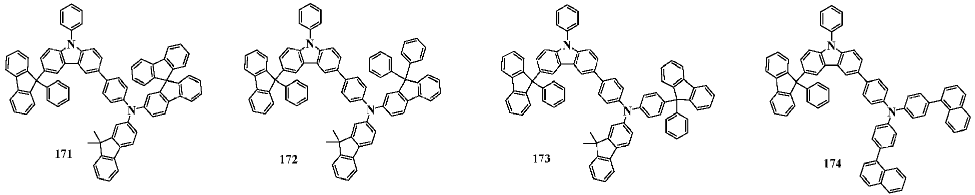

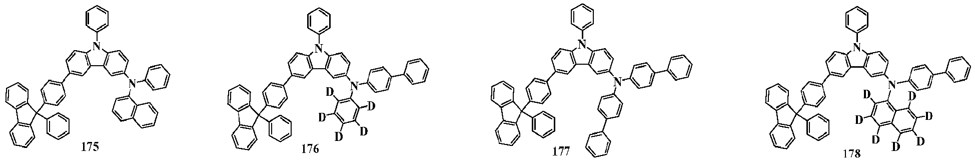

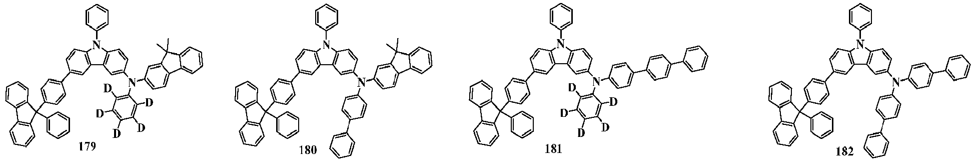

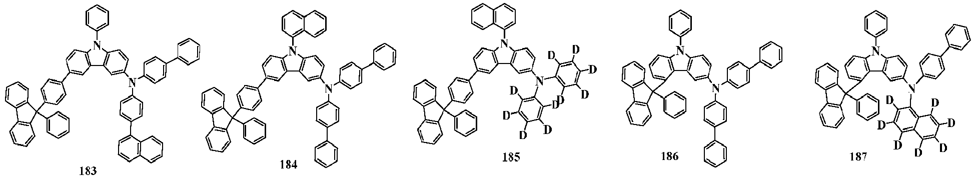

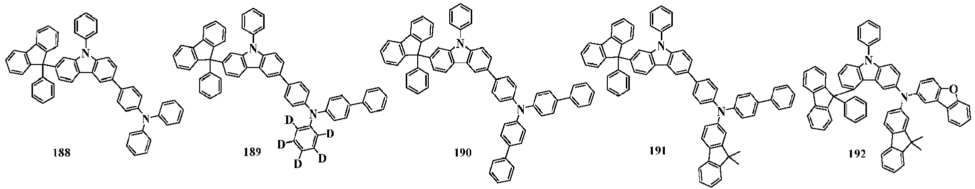

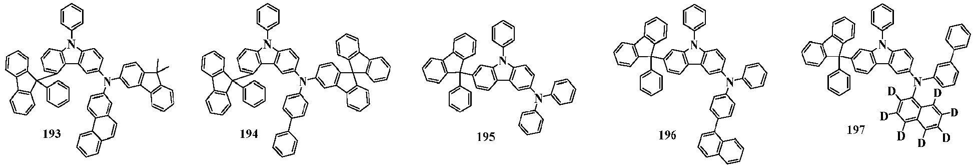

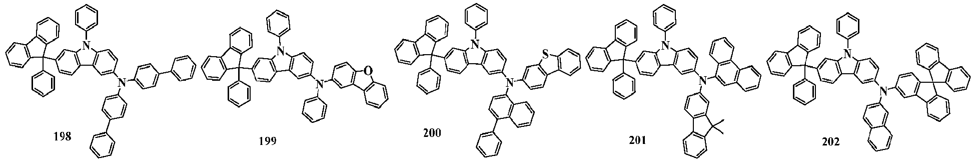

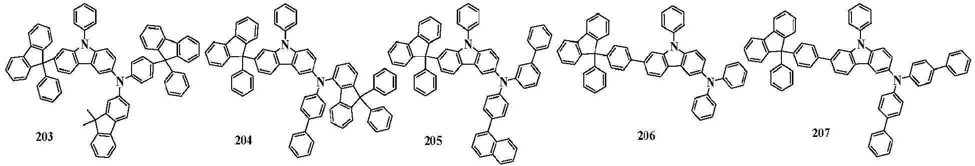

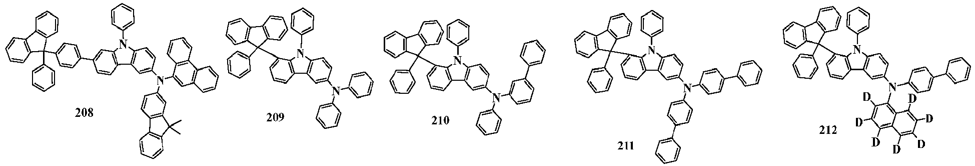

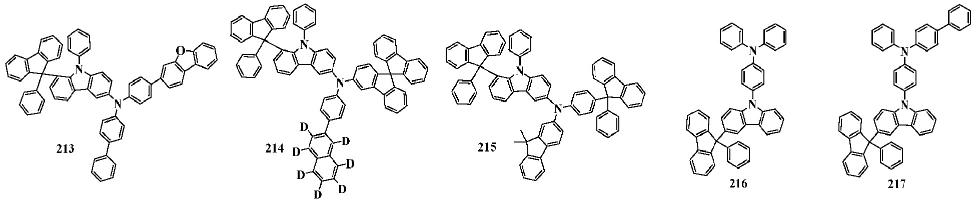

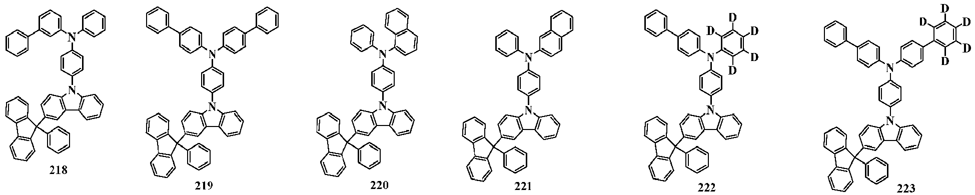

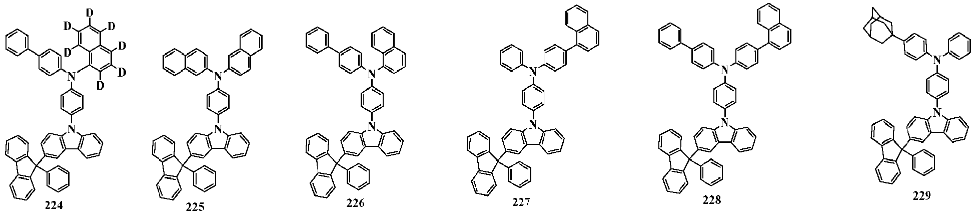

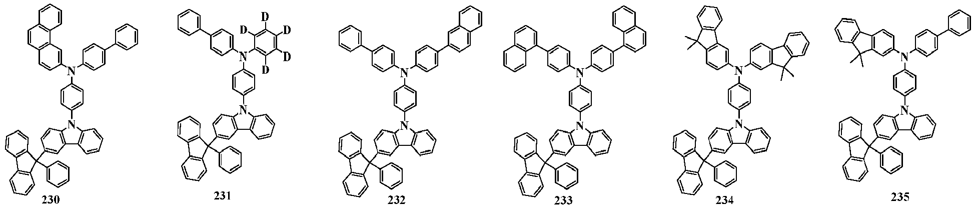

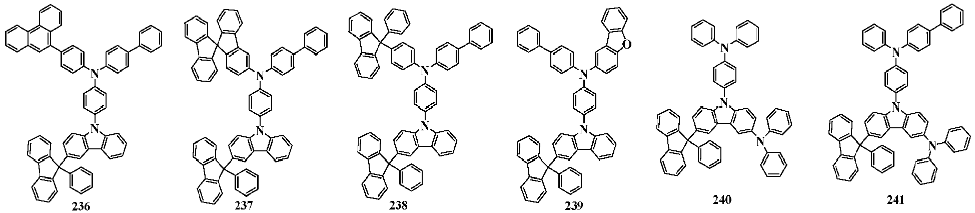

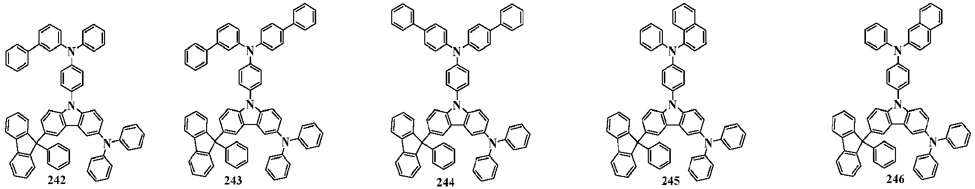

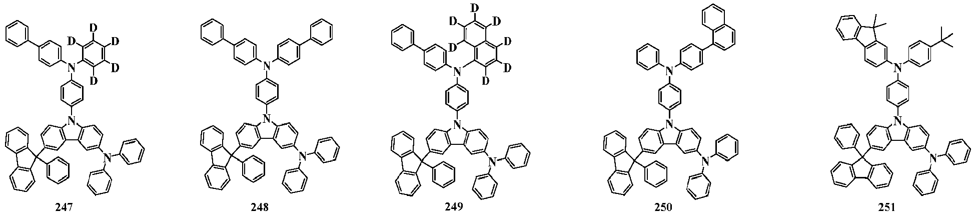

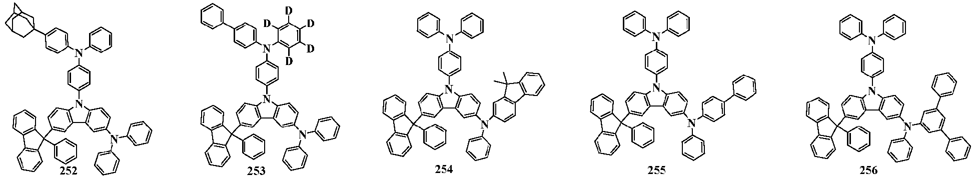

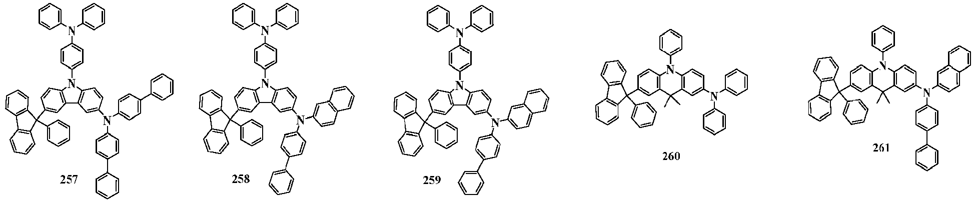

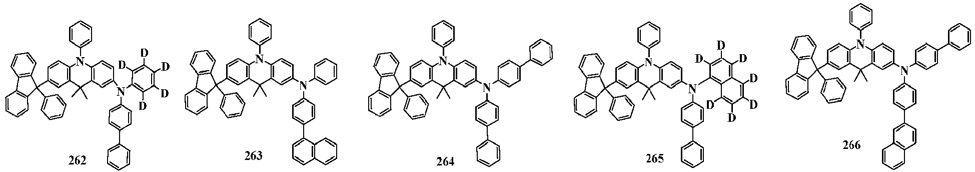

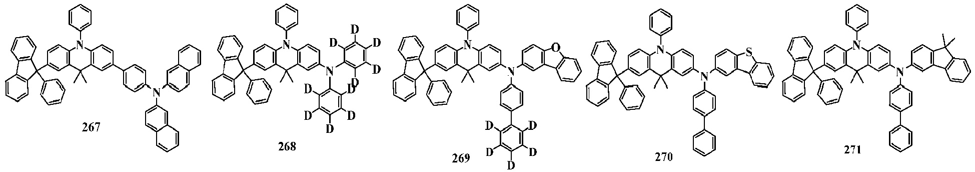

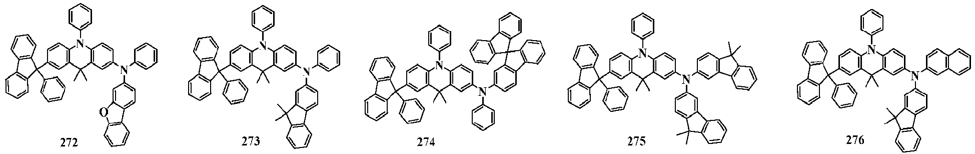

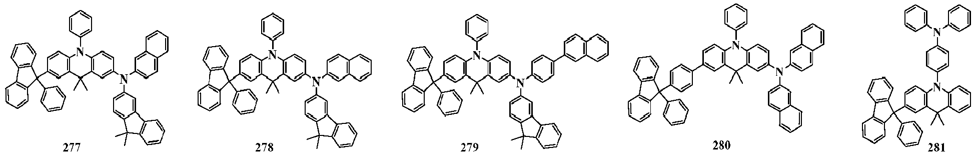

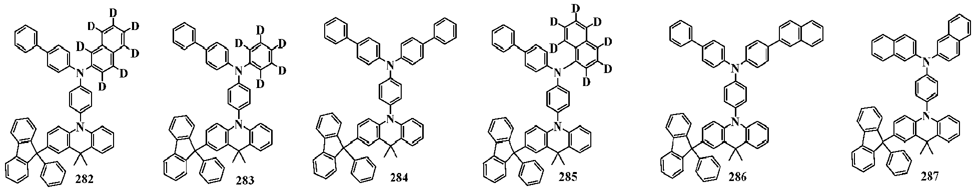

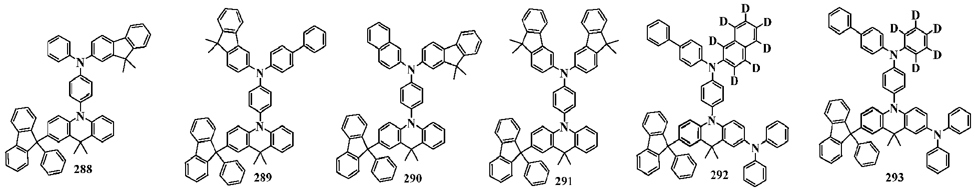

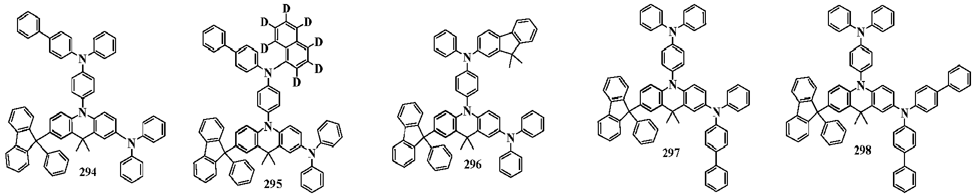

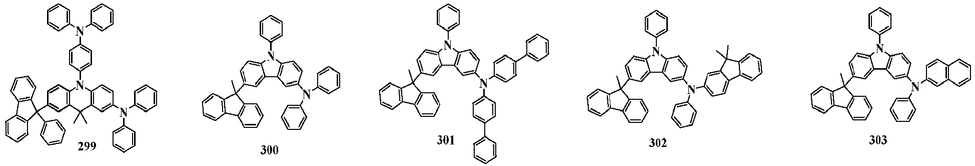

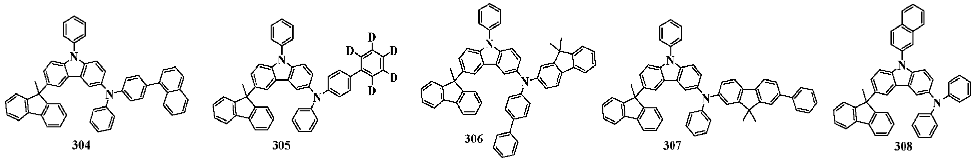

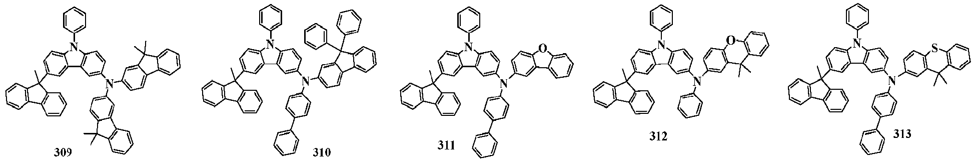

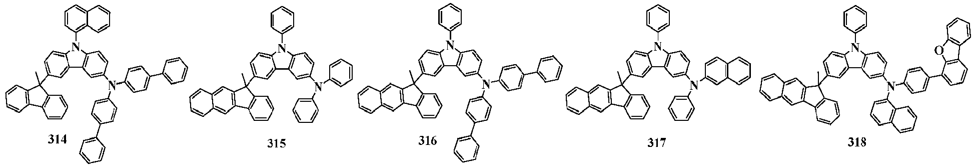

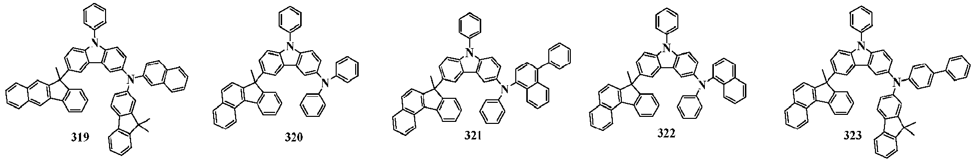

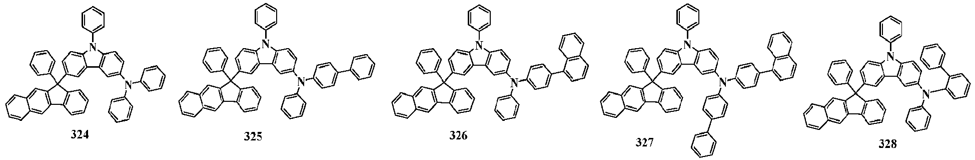

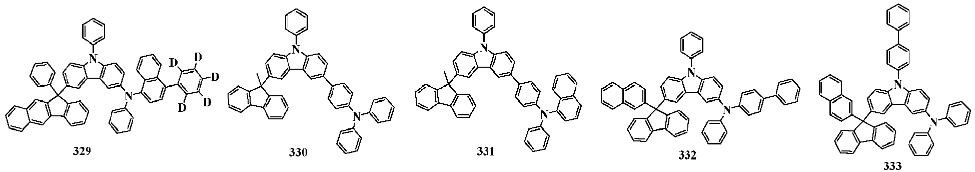

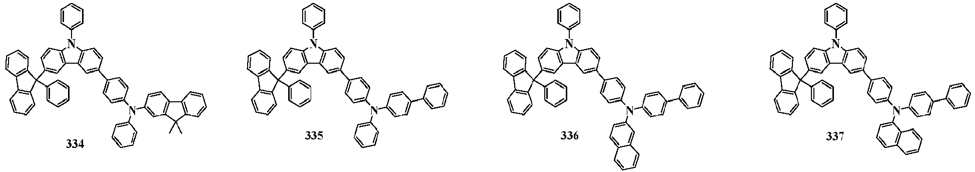

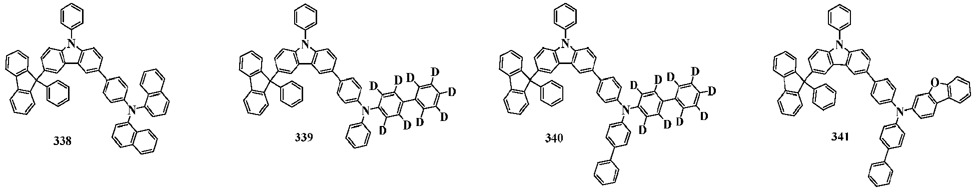

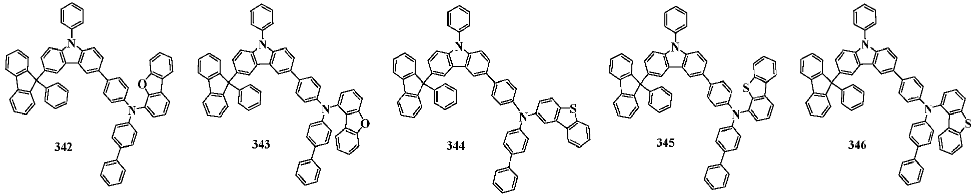

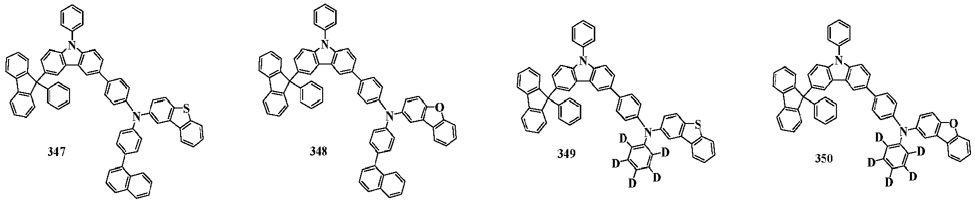

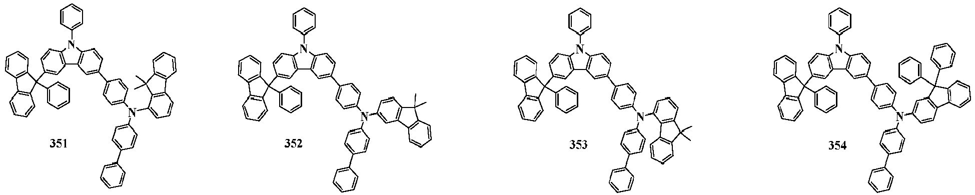

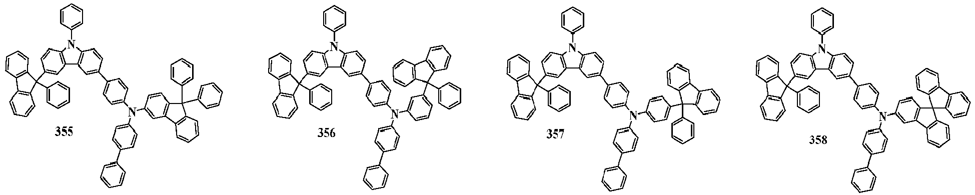

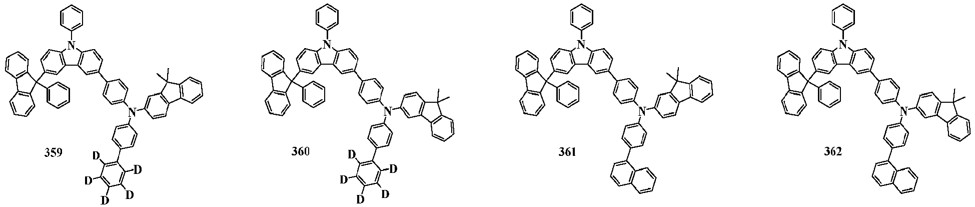

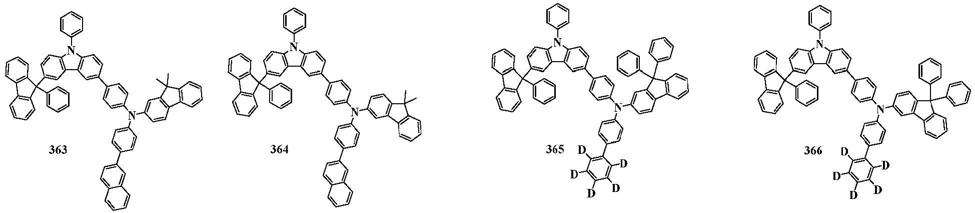

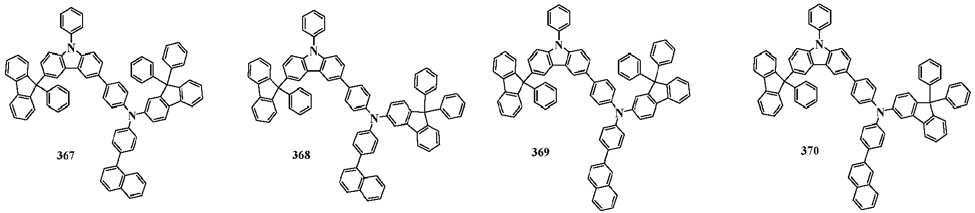

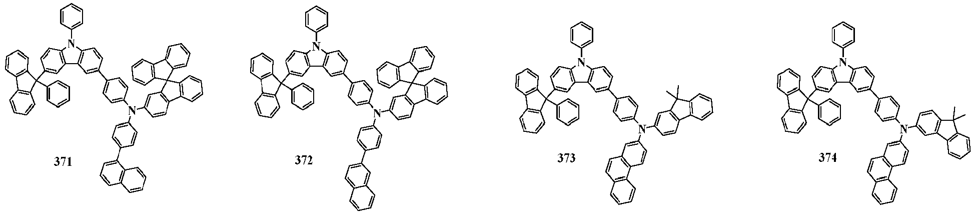

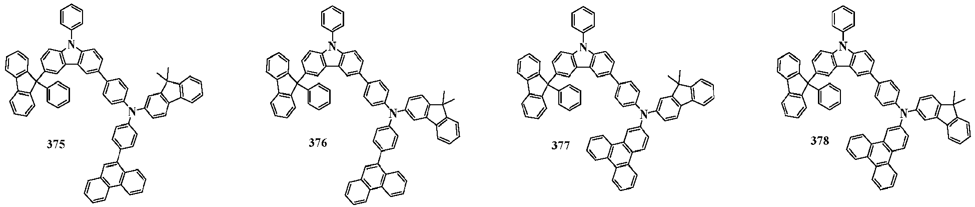

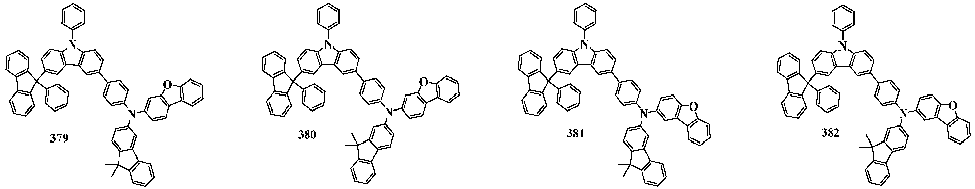

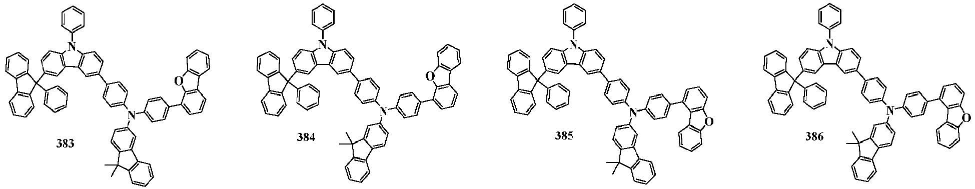

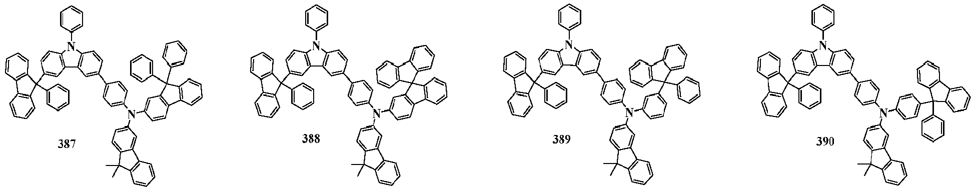

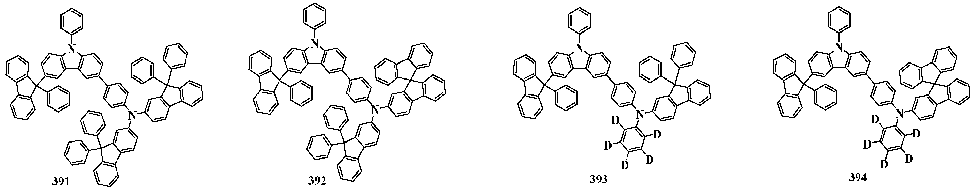

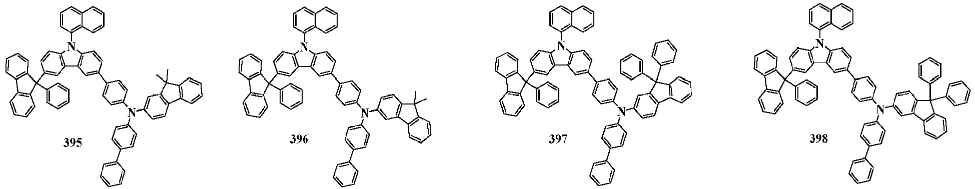

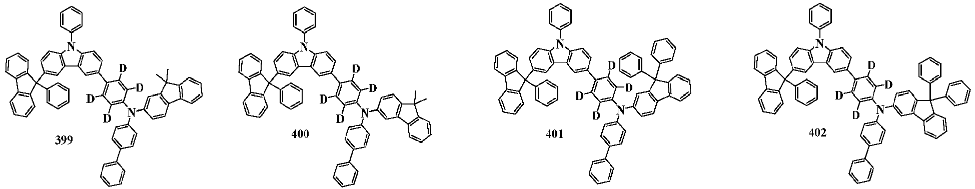

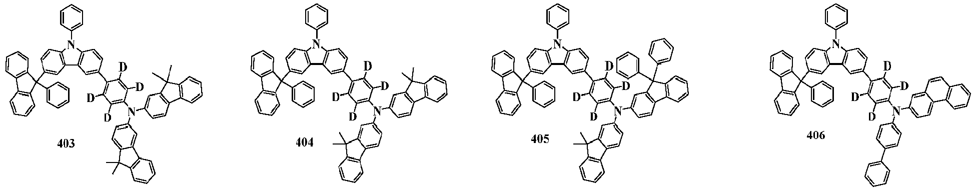

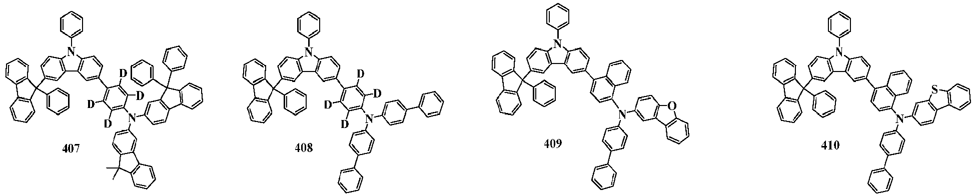

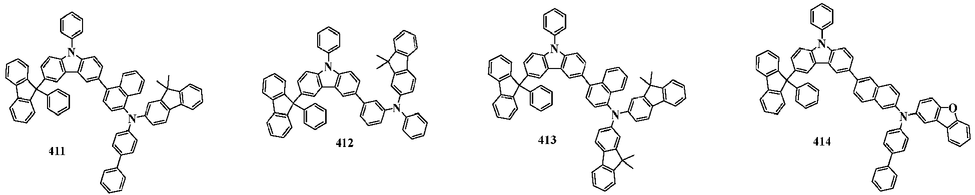

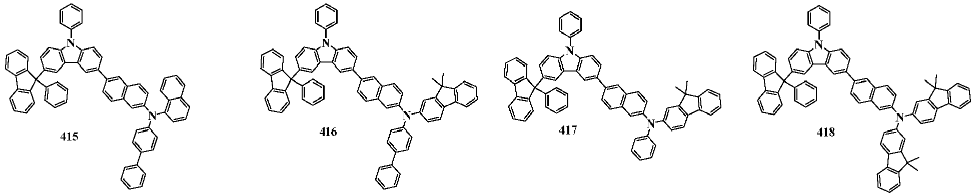

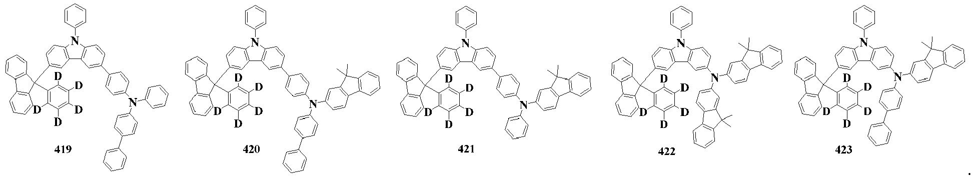

8. An organic electroluminescent device, comprising an anode, a cathode and an organic layer, wherein the organic layer is disposed between the anode and the cathode or disposed on an outer side of one or more of the anode and the cathode, wherein the organic layer comprises at least one of the triarylamine derivatives of any one of claims 1 to 7.

9. The organic electroluminescent device of claim 8, wherein the organic layer comprises a hole transport layer which comprises at least one of the triarylamine derivatives of any one of claims 1 to 7.

10. The organic electroluminescent device of claim 8, wherein the organic layer comprises a capping layer which comprises at least one of the triarylamine derivatives of any one of claims 1 to 7.
